# EUROPEAN PATENT APPLICATION

(11) **EP 3 255 478 A1**
(43) Date of publication of application: **13.12.2017**
(21) Application number: 17175273.6
(22) Date of filing: 09.06.2017
(51) Int. Cl.: G02C 7/04, A61B 3/10, A61F 2/16, G04G 11/00

(54) **ELECTRONIC OPHTHALMIC LENS WITH MEDICAL MONITORING**

(30) Priority: 10.06.2016 US 201615179184
(71) Applicant: Johnson & Johnson Vision Care Inc., Jacksonville, FL 32256 (US)
(72) Inventor: PUGH, Randall B., Jacksonville, FL 32256 (US); TONER, Adam, Jacksonville, FL 32256 (US)
(74) Representative: Carpmaels & Ransford LLP

(57) **Abstract**

An ophthalmic lens having an electronic system is described herein for monitoring the medical condition of the wearer using at least one sensor and at least one problem template. In a further embodiment, the problem template includes a pattern and/or a threshold. In at least one embodiment, the lens works in conjunction with a second lens and/or an external device to monitor for a medical condition or to perform a test protocol of the wearer. Examples of the at least one sensor include an eyelid position sensor system, an eye movement sensor system, a biosensor, a bioimpedance sensor, a temperature sensor, and a pulse oximeter.

## Description

### BACKGROUND OF THE INVENTION

### 1. Field of the Invention

The present invention relates to a powered or electronic ophthalmic lens, and more particularly, to a powered or electronic ophthalmic lens having a sensor and associated hardware and software for monitoring one or more medical conditions (or states) of the lens wearer.

### 2. Discussion of the Related Art

As electronic devices continue to be miniaturized, it is becoming increasingly more likely to create wearable or embeddable microelectronic devices for a variety of uses. Such uses may include monitoring aspects of body chemistry, administering controlled dosages of medications or therapeutic agents via various mechanisms, including automatically, in response to measurements, or in response to external control signals, and augmenting the performance of organs or tissues. Examples of such devices include glucose infusion pumps, pacemakers, defibrillators, ventricular assist devices and neurostimulators. A new, particularly useful field of application is in ophthalmic wearable lenses and contact lenses. For example, a wearable lens may incorporate a lens assembly having an electronically adjustable focus to augment or enhance performance of the eye. In another example, either with or without adjustable focus, a wearable contact lens may incorporate electronic sensors to detect concentrations of particular chemicals in the precorneal (tear) film. The use of embedded electronics in a lens assembly introduces a potential requirement for communication with the electronics, for a method of powering and/or re-energizing the electronics, for interconnecting the electronics, for internal and external sensing and/or monitoring, and for control of the electronics and the overall function of the lens.

The human eye has the ability to discern millions of colors, adjust easily to shifting light conditions, and transmit signals or information to the brain at a rate exceeding that of a highspeed internet connection. Lenses, such as contact lenses and intraocular lenses, currently are utilized to correct vision defects such as myopia (nearsightedness), hyperopia (farsightedness), presbyopia and astigmatism. However, properly designed lenses incorporating additional components may be utilized to enhance vision as well as to correct vision defects.

Contact lenses may be utilized to correct myopia, hyperopia, astigmatism as well as other visual acuity defects. Contact lenses may also be utilized to enhance the natural appearance of the wearer's eyes. Contact lenses or "contacts" are simply lenses placed on the anterior surface of the eye. Contact lenses are considered medical devices and may be worn to correct vision and/or for cosmetic or other therapeutic reasons. Contact lenses have been utilized commercially to improve vision since the 1950s. Early contact lenses were made or fabricated from hard materials, were relatively expensive and fragile. In addition, these early contact lenses were fabricated from materials that did not allow sufficient oxygen transmission through the contact lens to the conjunctiva and cornea which potentially could cause a number of adverse clinical effects. Although these contact lenses are still utilized, they are not suitable for all patients due to their poor initial comfort. Later developments in the field gave rise to soft contact lenses, based upon hydrogels, which are extremely popular and widely utilized today. Specifically, silicone hydrogel contact lenses that are available today combine the benefit of silicone, which has extremely high oxygen permeability, with the proven comfort and clinical performance of hydrogels. Essentially, these silicone hydrogel based contact lenses have higher oxygen permeability and are generally more comfortable to wear than the contact lenses made of the earlier hard materials.

Conventional contact lenses are polymeric structures with specific shapes to correct various vision problems as briefly set forth above. To achieve enhanced functionality, various circuits and components have to be integrated into these polymeric structures. For example, control circuits, microprocessors, communication devices, power supplies, sensors, actuators, light-emitting diodes, and miniature antennas may be integrated into contact lenses via custom-built optoelectronic components to not only correct vision, but to enhance vision as well as provide additional functionality as is explained herein. Electronic and/or powered ophthalmic lenses may be designed to provide enhanced vision via zoom-in and zoom-out capabilities, or just simply modifying the refractive capabilities of the lenses. Electronic and/or powered contact lenses may be designed to enhance color and resolution, to display textual information, to translate speech into captions in real time, to offer visual cues from a navigation system, and to provide image processing and internet access. The lenses may be designed to allow the wearer to see in low-light conditions. The properly designed electronics and/or arrangement of electronics on lenses may allow for projecting an image onto the retina, for example, without a variable-focus optic lens, and provide novelty image displays. Alternately, or in addition to any of these functions or similar functions, the contact lenses may incorporate components for the noninvasive monitoring of the wearer's biomarkers and health indicators. For example, sensors built into the lenses may allow a diabetic patient to keep tabs on blood sugar levels by analyzing components of the tear film without the need for drawing blood. In addition, an appropriately configured lens may incorporate sensors for monitoring cholesterol, sodium, and potassium levels, as well as other biological markers. This, coupled with a wireless data transmitter, could allow a physician to have almost immediate access to a patient's blood chemistry without the need for the patient to waste time getting to a laboratory and having blood drawn. In addition, sensors built into the lenses may be utilized to detect light incident on the eye to compensate for ambient light conditions or for use in determining blink patterns.

The proper combination of devices could yield potentially unlimited functionality; however, there are a number of difficulties associated with the incorporation of extra components on a piece of optical-grade polymer. In general, it is difficult to manufacture such components directly on the lens for a number of reasons, as well as mounting and interconnecting planar devices on a non-planar surface. It is also difficult to manufacture to scale. The components to be placed on or in the lens need to be miniaturized and integrated onto just 1.5 square centimeters of a transparent polymer while protecting the components from the liquid environment on the eye. It is also difficult to make a contact lens comfortable and safe for the wearer with the added thickness of additional components.

Given the area and volume constraints of an ophthalmic device such as a contact lens, and the environment in which it is to be utilized, the physical realization of the device must overcome a number of problems, including mounting and interconnecting a number of electronic components on a non-planar surface, the bulk of which comprises optic plastic. Accordingly, there exists a need for providing a mechanically and electrically robust electronic contact lens.

As these are powered lenses, energy or more particularly current consumption, to run the electronics is a concern given battery technology on the scale for an ophthalmic lens. In addition to normal current consumption, powered devices or systems of this nature generally require standby current reserves, precise voltage control and switching capabilities to ensure operation over a potentially wide range of operating parameters, and burst consumption, for example, up to eighteen (18) hours on a single charge, after potentially remaining idle for years. Accordingly, there exists a need for a system that is optimized for low-cost, long-term reliable service, safety and size while providing the required power.

In addition, because of the complexity of the functionality associated with a powered lens and the high level of interaction between all of the components comprising a powered lens, there is a need to coordinate and control the overall operation of the electronics and optics comprising a powered ophthalmic lens. Accordingly, there is a need for a system to control the operation of all of the other components that is safe, low-cost, and reliable, has a low rate of power consumption and is scalable for incorporation into an ophthalmic lens.

Powered or electronic ophthalmic lenses may have to account for certain unique physiological functions from the individual utilizing the powered or electronic ophthalmic lens. More specifically, powered lenses may have to account for blinking, including the number of blinks in a given time period, the duration of a blink, the time between blinks and any number of possible blink patterns, for example, if the individual is dosing off. Blink detection may also be utilized to provide certain functionality, for example, blinking may be utilized as a means to control one or more aspects of a powered ophthalmic lens. Additionally, external factors, such as changes in light intensity levels, and the amount of visible light that a person's eyelid blocks out, have to be accounted for when determining blinks. For example, if a room has an illumination level between fifty-four (54) and one hundred sixty-one (161) lux, a photosensor should be sensitive enough to detect light intensity changes that occur when a person blinks.

Ambient light sensors or photosensors are utilized in many systems and products, for example, on televisions to adjust brightness according to the room light, on lights to switch on at dusk, and on phones to adjust the screen brightness. However, these currently utilized sensor systems are not small enough and/or do not have low enough power consumption for incorporation into contact lenses.

It is also important to note that different types of blink detectors may be implemented with computer vision systems directed at one's eye(s), for example, a camera digitized to a computer. Software running on the computer can recognize visual patterns such as the eye open and closed. These systems may be utilized in ophthalmic clinical settings for diagnostic purposes and studies. Unlike the above described detectors and systems, these systems are intended for off-eye use and to look at rather than look away from the eye. Although these systems are not small enough to be incorporated into contact lenses, the software utilized may be similar to the software that would work in conjunction with powered contact lenses. Either system may incorporate software implementations of artificial neural networks that learn from input and adjust their output accordingly. Alternately, non-biology based software implementations incorporating statistics, other adaptive algorithms, and/or signal processing may be utilized to create smart systems.

There are a variety of jobs that require the worker to be aware and awake, for example, a truck driver, a security guard and military personnel on duty. It would be counterproductive and lead to potential issues if the worker were to fall asleep while performing his or her duties. Many of these jobs are such that the worker is required to have mobility while performing the duties and as such a fixed base monitoring system is not practical for providing monitoring of these workers. Furthermore, there are many jobs requiring regulated amounts of sleep in off-hours, which are manually logged by the worker instead of having automatic logging of the worker's sleep to provide better records.

Accordingly, there exists a need for a means and method for detecting certain physiological functions, such as a length of eye closure or a blink. The sensor being utilized needs to be sized and configured for use in a contact lens. In addition there exists a need to detect the position of a user's eyelids. An eyelid position sensor could be used to detect that a user is falling asleep, for example to log a data event of the wearer falling asleep. There are existing systems for detecting lid position; however, they are limited to devices like camera imagers, image recognition, and infrared emitter/detector pairs which rely on reflection off the eye and eyelid. Existing systems to detect lid position also rely on the use of spectacles or clinical environments and are not easily contained within a contact lens.

### SUMMARY OF THE INVENTION

In at least one embodiment, a powered ophthalmic lens includes a contact lens; and an eyelid position sensor system at least partially encapsulated in the contact lens, said eyelid position sensor system configured to detect vertical eyelid position and a signal conditioner configured to sample each individual sensor in said sensor system to detect eyelid position and provide an output lid signal; an eye movement sensor system at least partially encapsulated in the contact lens, said eye movement sensor system including at least one movement sensor to track and determine eye position and a signal conditioner cooperatively associated with said movement sensor and configured to track and determine eye position in spatial coordinates based on information from the output of said movement sensor and provide an output movement signal; a system controller in electrical communication with said eyelid position sensor system and said eye movement sensor system, said system controller having an associated memory containing a plurality of problem templates and at least two sets of registers for storing data received from said eyelid position sensor system and said eye movement sensor system, said system controller configured to compare the received output lid signal data and the output movement signal data to said plurality of problem templates and produce a control signal when at least one problem template is satisfied, and at least one alert mechanism in electrical communication with said system controller, said alert mechanism configured to receive the output control signal and capable of at least one of providing an alert and storing data. In a further embodiment, the at least one of the plurality of problem templates is based on historical data for an intended wearer of said lens.

In a further embodiment to any of the above embodiments, the powered ophthalmic lens further includes a user input in electrical communication with said system controller; and a storage memory in electrical communication with said system controller, and wherein said system controller includes a buffer memory for storing a plurality of signals from said eyelid position sensor system and said eye movement sensor system such that upon receipt of a signal from said user input, the system controller copies the data in the buffer memory into said storage memory. In a further embodiment, the user input includes a receiver capable of receiving wireless input originating with an individual to store the data present in said buffer memory.

In a further embodiment to any of the above embodiments, the powered ophthalmic lens further includes a receiver in electrical communication with said system controller, said receiver configured to receive a data request from an external device; and a transmitter in electrical communication with said system controller and said storage memory, and wherein said system controller in response to a received data request, transmits the contents of said storage memory through said transmitter to the external device. In a further embodiment to any of the above embodiments, the system controller determines an oscillating signal from said eye movement sensor system, said system controller copies the data in the buffer memory into a storage memory. In a further embodiment to any of the above embodiments, the eye movement sensor system includes at least one of at least one photodetector positioned to capture an image of the eye; at least one iris-facing camera configured to detect changes in images, patterns, or contrast to track eye movement; at least one accelerometer to track movement of at least one of the eye or the contact lens; and at least one neuromuscular sensor configured to detect neuromuscular activity associated with eye movement. In a further embodiment to any of the above embodiments, the eye movement sensor system further comprises a signal processor configured to receive signals from said movement sensor, perform digital signal processing, and output one or more to the system controller.

In a further embodiment to any of the above embodiments, the system controller detects a change in pupil size not in response to a change in environmental light condition as detected by said eyelid position sensor system and where the pupil size is based on at least one signal from said eye movement sensor system, said system controller sends the control signal to said alert mechanism. In a further embodiment to any of the above embodiments, the system controller detects a stable accelerometer reading in a direction indicative that a wearer is in a prone position after a rapid acceleration in that direction where the readings are from said eye movement sensor system; said system controller sends the control signal to said alert mechanism. In a further embodiment to any of the above embodiments, the spatial coordinates are in three dimensions. In a further embodiment to any of the above embodiments, the movement sensor includes at least one accelerometer; and said system controller compares each signal from said at least one accelerometer against a threshold, when any signal exceeds the threshold, said system controller sends the control signal to said alert mechanism. In a further embodiment to any of the above embodiments, the powered ophthalmic lens further includes an iris-facing light source is in electrical communication with said system controller; and at least one iris-facing photosensor arranged to receive reflected light back from the eye where said light originates from said light source, said at least photosensor is in electrical communication with said system controller; a transmitter in electrical communication with said system controller, and wherein said system controller is configured to send an oximeter signal to said light source and receive a signal from said at least one photosensor, which received signal is transmitted to an external device for processing by said system controller through said transmitter. In a further embodiment to any of the above embodiments, the system controller is configured to use more than one system sensor to confirm any determination by said system controller of a need for the output control signal to be sent to said alert mechanism.

In a further embodiment to any of the above embodiments, there is a lens pair including the above-described powered ophthalmic lens, wherein the eye movement sensor system further includes a communication system for communication with at least a second contact lens, said second contact lens having an eye movement sensor system incorporated into the contact lens, the eye movement sensor system including at least one sensor to track and determine eye position and a signal conditioner cooperatively associated with the sensor and configured to track and determine eye position in spatial coordinates based on information from the sensor output and provide an output movement signal; a system controller in electrical communication with said eye movement sensor system, and a communication system for communicating the output of the eye movement sensor system to said first contact lens. Further to the previous embodiment, the system controller in said first contact lens detects divergence of lines of vision of the wearer's eyes; said system controller sends the control signal to said alert mechanism. Further to either of the previous embodiments, each lens further includes a rear-facing pupil diameter sensor in electrical communication with said system controller, said rear-facing pupil diameter sensor for measuring pupil diameter; said system controller of said second lens is configured to transmit said pupil diameter measurement via said communication systems to said system controller of said first lens such that said first lens system controller is configured to determine whether the measured pupil dilations of the wearer's eye are substantially similar, when the pupil dilations are different, the first system controller configured to send the output control signal to said alert mechanism.

In at least one embodiment, the powered ophthalmic lens includes a contact lens; and a first sensor in said contact lens; at least one second sensor in said contact lens; a system controller in electrical communication with said first sensor and said at least one second sensor, said system controller having an associated memory containing a plurality of problem templates and at least two sets of registers for storing data received from said sensors, said system controller configured to compare the received sensor data to said plurality of problem templates and produce a control signal when a match occurs, and at least one alert mechanism in electrical communication with said system controller, said alert mechanism configured to receive the output control signal and capable of at least one of providing an alert and storing data. In a further embodiment, the first sensor and/or said at least one second sensor is selected from a group consisting of an eyelid position sensor system, an eye movement sensor system, a biosensor, a bioimpedance sensor, a temperature sensor, and pulse oximeter. In a still further embodiment, at least one of the above-described sensors is used as the first sensor and/or at least one second sensor in the previous two embodiments.

In at least one embodiment, a powered ophthalmic lens includes a contact lens; an iris-facing light source in said contact lens; at least one iris-facing photosensor arranged to receive reflected light back from the eye where said light originates from said light source; and a system controller in electrical communication with said iris-facing light source and said at least one iris-facing photosensor, said system controller configured to process at least one signal from said iris-facing photosensor and correlate the processed signal with at least one signal sent to said iris-facing light source. In a further embodiment, the powered ophthalmic lens further includes a transmitter in electrical communication with said system controller, and wherein said system controller is configured to send the correlated signals via said transmitter to an external device for processing. In a further embodiment to the other embodiments of this paragraph, the iris-facing light source and said at least one iris-facing photosensor are spaced from each other such that said iris-facing light source and said at least one iris-facing photosensor are proximate to opposing edges of said contact lens. In a further embodiment to the other embodiments of this paragraph, the iris-facing light source includes a first light emitter transmitting a light having a wavelength of about 660 nm and a second light emitter transmitting a light having a wavelength of between about 890 nm and about 950 nm.

In at least one embodiment, a system for conducting a test protocol on a wearer of at least one contact lens includes a device having a processor configured to run a test protocol, a camera connected to said processor, a display connected to said processor and configured to display images generated by said processor, communications module; and at least one powered ophthalmic contact lens having an eye movement sensor system including a sensor to determine and track eye position, said eye movement sensor system configured to output a spatial location of the eye, a system controller cooperatively associated with the sensor, the system controller configured to determine movement of the eye based on the spatial location output from said eye movement sensor system, said system controller is further configured to output a control signal based on the determination, and communications circuit configured to facilitate communication with said communications module of said device during performance of the test protocol; and wherein said processor performs the test protocol in conjunction with said system controller. In a further embodiment, the control signal produced by said system controller includes gaze direction information; said test protocol correlates movement of said device by a subject while the display is providing directions to the subject with the received gaze direction transmitted by said system controller through said communications circuit and said communications module while monitoring for movement of a subject's head, when at least one of no correlation or movement of the subject's head occurs, said processor is configured to trigger an alert to be shown on said display; and wherein the directions are generated by said processor based on instructions performed by said processor. In a further embodiment to the previous embodiment, the device includes an accelerometer electrically connected to said processor such that said processor is configured to use an output of said accelerometer in conjunction with an output of said camera to determine if the subject's head is stable while said device is moved substantially in a straight line in front of the subject, and said processor is configured to correlate the accelerometer readings from said lens transmitted through said communications circuit and said communications module with the accelerometer signals from said accelerometer on said device, when a difference between the accelerometer signals after normalization for distance travelled by said device and said lens is greater than a threshold, then said processor is configured to trigger the alert to be shown on said display.

In a further embodiment to the first embodiment of the previous paragraph, the lens further includes an iris-facing pupil diameter sensor in electrical communication with said system controller, said iris-facing pupil diameter sensor configured to provide a signal representing pupil diameter; said device further includes a light source controllable by said processor, and said test protocol includes said processor activating said light source, said system controller measuring a before and after light source activation of said pupil diameter with said pupil diameter sensor, said system controller transmitting said measurements to said processor through said antennas, said processor comparing said measurements to determine pupil dilation, and said processor sending an alert to said display when at least one of the pupil dilation exceeds a dilation threshold and the pupil dilation is less than an undilated threshold. In a further embodiment, the contact lens further includes a photodetector in communication with said system controller; and wherein said system controller configured to use outputs of said photodetector to detect a light level of said light source. In a further embodiment to the first embodiments in the previous paragraph, the lens further includes an iris-facing pupil diameter sensor in electrical communication with said system controller, said iris-facing pupil diameter sensor for measuring pupil diameter; said device further includes a light source controllable by said processor, and said test protocol includes said processor displaying instruction on said display directing the wearer to view a bright light, said system controller measuring a before and after light source activation of said pupil diameter with said pupil diameter sensor, said system controller transmitting said measurements to said processor through said antennas, said processor comparing said measurements to determine pupil dilation, and said processor sending an alert to said display when at least one of the pupil dilation exceeds a dilation threshold and the pupil dilation is less than an undilated threshold. In a further embodiment, the contact lens further includes a photodetector in communication with said system controller; and wherein said system controller configured to use outputs of said photodetector to detect a light level of said light source.

In a further embodiment to the embodiments in the previous two paragraphs, the sensor includes at least one accelerometer; and the test protocol is prompted by detection of a possible concussion when said system controller determines an acceleration of a head of the wearer exceeds a concussion threshold based on a signal received from said accelerometer. In a further embodiment to the embodiments in this paragraph and the previous two paragraphs, the test protocol includes having a wearer of the lens focus on a place on a stationary object, turning the wearer's head right or left while having the wearer continue to look at the place, tracking the gaze of the wearer relative to the turning speed of the wearer's head to determine whether the differential is within a predetermined threshold, alerting at least one of the wearer through said alert mechanism and/or through transmitting an alert signal to said device to display an alert on said display. In a further embodiment, the eye movement sensor system includes at least one accelerometer; and the differential is determined based on a signal from said at least one accelerometer where the signal equaling zero is confirmation of tracking of the place on the wall by the wearer while when the signal is a non-zero value the wearer has a delay in tracking the place on the wall. In a further embodiment to the previous two embodiments, the test protocol further includes storing on said device data from said test protocol for later use in a verification study.

In at least one embodiment, the system for conducting a test protocol on a wearer of at least one contact lens includes at least one powered ophthalmic contact lens having an iris-facing pupil diameter sensor configured to output a signal representing pupil diameter; at least one forward-facing photodetector; an alert mechanism; a system controller in communication with said iris-facing pupil diameter sensor and said at least one photodetector, the system controller configured to monitor outputs of said iris-facing pupil diameter sensor, monitor said at least one forward-facing photodetector for a detected light exceeding a brightness threshold, compare the output of the iris-facing pupil diameter sensor from before and after detection of the light exceeding the brightness threshold, when the difference between outputs of the iris-facing pupil diameter sensor exceeds a dilation threshold or is less than an undilated threshold, sending a signal to said alert mechanism. In a further embodiment, the alert mechanism alerts the user in response to the signal from the system controller.

### BRIEF DESCRIPTION OF THE DRAWINGS

The foregoing and other features and advantages of the invention will be apparent from the following, more particular description of preferred embodiments of the invention, as illustrated in the accompanying drawings.
FIGs. 1A-1F illustrate a contact lens having sensor systems in accordance with at least one embodiment of the present invention.
FIG. 2A illustrates a diagrammatic representation of two eyelid position sensors having a communication channel for synchronizing operation between two eyes in accordance with at least one embodiment of the present invention.
FIG. 2B illustrates a diagrammatic representation of one eyelid position sensor having a communication channel for communicating with an external device in accordance with at least one embodiment of the present invention.
FIGs. 3A-3C illustrate flowcharts for medical monitoring methods in accordance with embodiments of the present invention.
FIG. 4 illustrates a graphical representation of light incident on the surface of the eye versus time, illustrating a possible involuntary blink pattern recorded at various light intensity levels versus time and a usable threshold level based on some point between the maximum and minimum light intensity levels in accordance with at least one embodiment of the present invention.
FIG. 5 is a state transition diagram of an eyelid position sensor system in accordance with at least one embodiment of the present invention.
FIG. 6 illustrates a diagrammatic representation of a photodetection path utilized to detect and sample received light signals in accordance with at least one embodiment of the present invention.
FIG. 7 illustrates a block diagram of digital conditioning logic in accordance with at least one embodiment of the present invention.
FIG. 8 illustrates a block diagram of digital detection logic in accordance with at least one embodiment of the present invention.
FIG. 9 illustrates a timing diagram in accordance with at least one embodiment of the present invention.
FIG. 10 illustrates a diagrammatic representation of a digital system controller in accordance with at least one embodiment of the present invention.
FIGs. 11A-11G illustrate timing diagrams for automatic gain control in accordance with at least one embodiment of the present invention.
FIG. 12 illustrates a diagrammatic representation of light-blocking and light-passing regions on an integrated circuit die in accordance with at least one embodiment of the present invention.
FIG. 13 illustrates a diagrammatic representation of an electronic insert, including a blink detector, for a powered contact lens in accordance with at least one embodiment of the present invention.
FIGs. 14A and 14B illustrate diagrammatic representations of eyelid position sensors in accordance with at least one embodiment of the present invention.
FIG. 15A illustrates a diagrammatic representation of an electronic system incorporated into a contact lens for detecting eyelid position in accordance with at least one embodiment of the present invention.
FIG. 15B illustrates an enlarged view of the electronic system of FIG. 15A.
FIG. 16 illustrates a diagrammatic representation of outputs from eyelid position sensors in accordance with at least one embodiment of the present invention.
FIG. 17A illustrates a diagrammatic representation of another electronic system incorporated into a contact lens for detecting eyelid position in accordance with at least one embodiment of the present invention.
FIG. 17B illustrates an enlarged view of the electronic system of FIG. 17A.
FIG. 18A-18C illustrate diagrammatic representations of an eyelid position detecting system in accordance with at least one embodiment of the present invention.
FIG. 18D illustrates an enlarged view of the electronic system of FIGs. 18A-18C.
FIG. 19A illustrates a diagrammatic representation of a pupil position and convergence detection system incorporated into a contact lens in accordance with at least one embodiment of the present invention.
FIG. 19B is an enlarged view of the pupil position and convergence detection system of FIG. 19A.
FIG. 19C illustrates an overlay of an X, Y, and Z axes on the eye.
FIG. 20 illustrates a diagrammatic representation of two pupil position and convergence sensors having a communication channel for synchronizing operation between two eyes in accordance with at least one embodiment of the present invention.
FIG. 21 illustrates a diagrammatic representation of a plot of the correlation between pupil convergence and focal distance.
FIG. 22A illustrates a diagrammatic, front perspective representation of the eyes of an individual gazing to the right.
FIG. 22B illustrates a diagrammatic, top perspective representation of the eyes of FIG. 22A.
FIG. 23 illustrates a diagrammatic representation of the geometry associated with various gaze directions in two dimensions in accordance with at least one embodiment of the present invention.
FIG. 24 illustrates a diagrammatic representation of a powered ophthalmic lens having a first pupil diameter sensor positioned on eye in accordance with at least one embodiment of the present invention.
FIG. 25 illustrates a diagrammatic representation of a powered ophthalmic lens having a second pupil diameter sensor positioned on eye in accordance with at least one embodiment of the present invention.
FIG. 26 illustrates a plot of an example of ambient light and pupil diameter versus time.
FIG. 27 illustrates a diagrammatic representation of a powered ophthalmic lens having pulse oximetry components in accordance with at least one embodiment of the present invention.
FIG. 28 illustrates a diagrammatic representation of a powered ophthalmic lens having pulse oximetry components in accordance with at least one second embodiment of the present invention.
FIG. 29 illustrates a block diagram of an insertion sensor embodiment in accordance with at least one embodiment of the present invention.
FIG. 30 illustrates a block diagram of a generic system having multiple sensors, a system controller and an alert mechanism, wherein an activation decision is made based on the output of two or more sensors in accordance with the present invention.
FIG. 31 illustrates a flow chart of a method by which a system controller determines if the state of an alert mechanism is to be changed based upon sensor inputs in accordance with at least one embodiment of the present invention.
FIG. 32 illustrates a block diagram of a storage box in accordance with at least one embodiment of the present invention.

### DETAILED DESCRIPTION OF THE PREFERRED EMBODIMENTS

Conventional contact lenses are polymeric structures with specific shapes to correct various vision problems as briefly set forth above. To achieve enhanced functionality, various circuits and components may be integrated into these polymeric structures. For example, control circuits, microprocessors, communication devices, power supplies, sensors, light-emitting diodes, and miniature antennas may be integrated into contact lenses via custom-built optoelectronic components to not only correct vision, but to enhance vision as well as provide additional functionality as is explained herein. Electronic and/or powered contact lenses may be designed to provide enhanced vision via zoom-in and zoom-out capabilities, or just simply modifying the refractive capabilities of the lenses. Electronic and/or powered contact lenses may be designed to enhance color and resolution, to display textual information, to translate speech into captions in real time, to offer visual cues from a navigation system, and to provide image processing and internet access. The lenses may be designed to allow the wearer to see in low light conditions. The properly designed electronics and/or arrangement of electronics on lenses may allow for projecting an image onto the retina, for example, without a variable focus optic lens, provide novelty image displays and even provide wakeup alerts. In addition, sensors built into the lenses may be utilized to detect light incident on the eye to compensate for ambient light conditions or for use in determining blink patterns and whether the wearer is a desired medical state.

The powered or electronic contact lens of at least one exemplary embodiment includes the necessary elements to monitor the wearer with or without elements to correct and/or enhance the vision of patients with one or more of the above described vision defects or otherwise perform a useful ophthalmic function. The electronic contact lens may have a variable-focus optic lens, an assembled front optic embedded into a contact lens or just simply embedding electronics without a lens for any suitable functionality. The electronic lens of the present invention may be incorporated into any number of contact lenses as described above. In addition, intraocular lenses may also incorporate the various components and functionality described herein. However, for ease of explanation, the disclosure will focus on an electronic contact lens intended for single-use daily disposability.

The present invention may be employed in a powered ophthalmic lens or powered contact lens having an electronic system, which actuates a variable-focus optic or any other device or devices configured to implement any number of numerous functions that may be performed. The electronic system includes one or more batteries or other power sources, power management circuitry, one or more sensors, clock generation circuitry, control algorithms and circuitry, and lens driver circuitry. The complexity of these components may vary depending on the required or desired functionality of the lens. Alternatively, the contact lens may just monitor the wearer in at least one embodiment.

Control of an electronic or a powered ophthalmic lens may be accomplished through a manually operated external device that communicates with the lens, such as a hand-held remote unit, a storage container, or cleaning box. For example, a fob may wirelessly communicate with the powered lens based upon manual input from the wearer. Alternately, control of the powered ophthalmic lens may be accomplished via feedback or control signals directly from the wearer. For example, sensors built into the lens may detect blinks, blink patterns, eyelid closures, and/or eye movement. Based upon the pattern or sequence of blinks and/or movement, the powered ophthalmic lens may change operation state. A further alternative is that the wearer has no control over operation of the powered ophthalmic lens. In at least one embodiment, the lens control can be used to 1) begin a medical monitoring session and/or protocol test and/or 2) mark and/or store sensor data. In at least one embodiment, these lens controls are examples of input means for receiving an input from the wearer (or user).

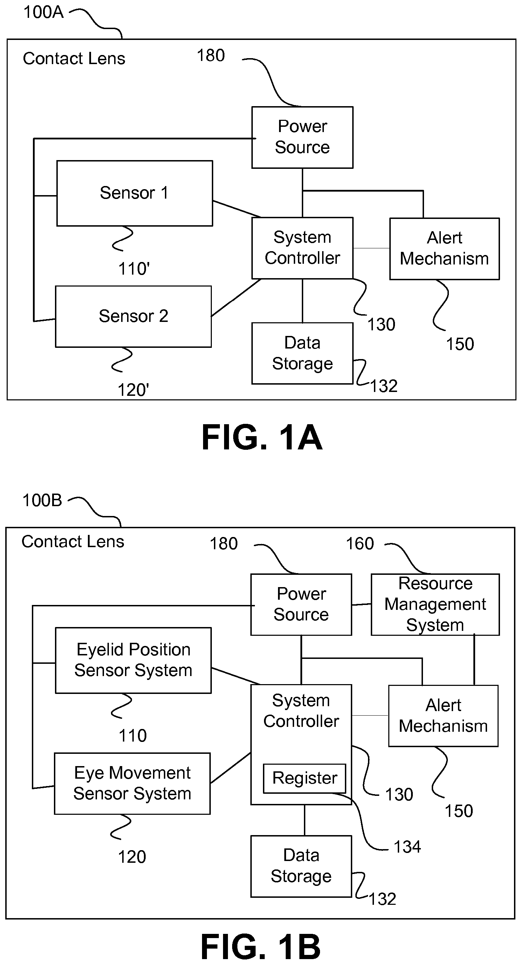

In at least one exemplary embodiment, the contact lens includes at least one sensor 110 in electrical communication with a system controller 130 to allow for the monitoring of the wearer of the contact lens and/or alerting the wearer when a medical condition detected by the at least one sensor arises. In a further exemplary embodiment, there are at least two sensors 110', 120' (110, 120) monitoring the wearer of the contact lens 100A-100F as illustrated in FIGs. 1A-1F. Examples of sensors as will be developed further in this disclosure include: an eyelid position sensor system, an eye movement sensor system, a pupil diameter sensor, a bioimpedance sensor, a pulse oximeter, a salinity sensor, a biosensor, strain and/or pressure sensors, and a temperature sensor.

The system controller 130 in at least one exemplary embodiment uses at least one predetermined threshold for comparing at least one data sample of the at least one sensor to determine whether a medical condition has arisen. In another exemplary embodiment, the system controller 130 makes use of at least one problem template (or pattern) to which a series of data samples or alternatively one data sample from the at least one sensor are compared against to determine whether a medical condition has arisen, for example based on a match to the pattern and/or a threshold being met, exceeded or less than resulting in the problem template being satisfied. In at least one exemplary embodiment, the problem template includes only at least one threshold. In an alternative exemplary embodiment, both thresholds and patterns are used by the system controller 130. In at least one exemplary embodiment as illustrated in FIG. 1A, the system controller 130 is in electrical communication with a data storage 132 that stores the threshold(s) and/or template(s). In at least one exemplary embodiment, a plurality of problem templates may include any combination of patterns and thresholds. Examples of data storage 132 include memory such as persistent or non-volatile memory, volatile memory, and buffer memory, a register(s), a cache(s), programmable read-only memory (PROM), and flash memory.

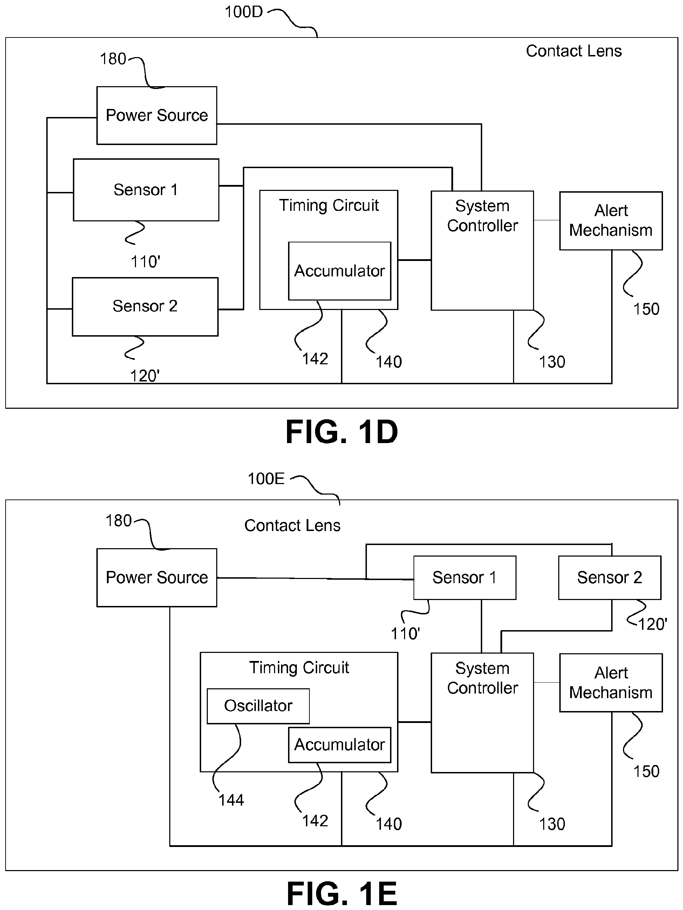

The system in FIG. 1A also includes an alert mechanism 150 that receives an output from the system controller 130. The alert mechanism 150 may include any suitable device for implementing a specific alert to the wearer based upon a received command signal from the system controller 130. For example, if a set of data samples matches a problem template, the system controller 130 may enable the alert mechanism 150, such as a light (or light array) to pulse a light or cause a physical wave to pulsate into the wearer's retina (or alternatively across the lens) or to log data regarding the state of the wearer. Further examples of the alert mechanism 150 include an electrical device; a mechanical device including, for example, piezoelectric devices, transducers, vibrational devices, chemical release devices with examples including the release of chemicals to cause an itching, irritation or burning sensation, and acoustic devices; a transducer providing optic zone modification of an optic zone of the contact lens such as modifying the focus and/or percentage of light transmission through the lens; a magnetic device; an electromagnetic device; a thermal device; an optical coloration mechanism with or without liquid crystal, prisms, fiber optics, and/or light tubes to, for example, provide an optic modification and/or direct light towards the retina; an electrical device such as an electrical stimulator to provide a mild retinal stimulation or to stimulate at least one of a corneal surface and one or more sensory nerves of the cornea; or any combination thereof. In an alternative exemplary embodiment, the alert mechanism 150 sends an alert to an external device. The alert mechanism 150 receives a signal from the system controller 130 in addition to power from the power source 180 and produces some action based on the signal from the system controller 130. For example, if the output signal from the system controller 130 occurs during one operation state, then the alert mechanism 150 may alert the wearer that a medical condition has arisen. In an alternate embodiment, the signal output by the system controller 130 during another operation state, then the alert mechanism 150 will record the information in memory for later retrieval. In a still further alternative exemplary embodiment, the signal will cause the alert mechanism 150 to alarm and store information. In an alternative exemplary embodiment, the system controller 130 stores the data in the memory (e.g., data storage 132) associated with the system controller 130 and does not use the alert mechanism 150 for data storage and in at least one exemplary embodiment, the alert mechanism 150 is omitted from the illustrated embodiments of FIGs. 1A-1F. In at least one exemplary embodiment there is a clock such as the timing circuit 140 in FIG. 1D that is capable of providing a time stamp. As set forth above, the powered lens of the present invention may provide various functionality; accordingly, one or more alert mechanisms may be variously configured to implement the functionality.

FIG. 1A also illustrates a power source 180, which supplies power for numerous components in the system. The power may be supplied from a battery, energy harvester, or other suitable means as is known to one of ordinary skill in the art. Essentially, any type of power source 180 may be utilized to provide reliable power for all other components of the system. In an alternative exemplary embodiment, communication functionality is provided by an energy harvester that acts as the receiver for the time signal, for example in an alternative embodiment, the energy harvester is a solar cell or a radio frequency (RF) receiver, which receives both power and a time-base signal (or indication). In a further alternative exemplary embodiment, the energy harvester is an inductive charger, in which power is transferred in addition to data such as RFID. In one or more of these alternative embodiments, the time signal could be inherent in the harvested energy, for example N*60 Hz in inductive charging or lighting.

In at least one exemplary embodiment as illustrated in FIG. 1B, the system controller 130 includes a register 134 for storing data samples from the at least one sensor 110/120. In a further exemplary embodiment, there is an individual register for each sensor present on the contact lens being used to monitor a medical condition. In a still further exemplary embodiment, there is an individual register for each sensor present on the contact lens. The use of a register in at least one embodiment allows for the comparison of data with a problem template with or without a mask. In an alternative exemplary embodiment, other data storage is used instead of a register(s). In an alternative embodiment, the register 134 is part of the data storage 132.

FIG. 1B also illustrates a medical monitoring system according to at least one exemplary embodiment. The illustrated system includes a contact lens 100B having an eyelid position sensor system 110, an eye movement sensor system 120, a system controller 130 and an alert mechanism 150. The sensor systems 110, 120 are in electrical communication with the system controller 130, which in turn is in electrical communication with the alert mechanism 150. In at least one exemplary embodiment, the alert mechanism 150 includes an accumulator connected to a memory. In at least one embodiment, the alert mechanism 150 is consolidated with the system controller 130. FIG. 1B also illustrates a power source 180 that, in at least one embodiment, provides power to the other components of the system. FIG. 1B illustrates an optional resource management system 160, which will be discussed later.

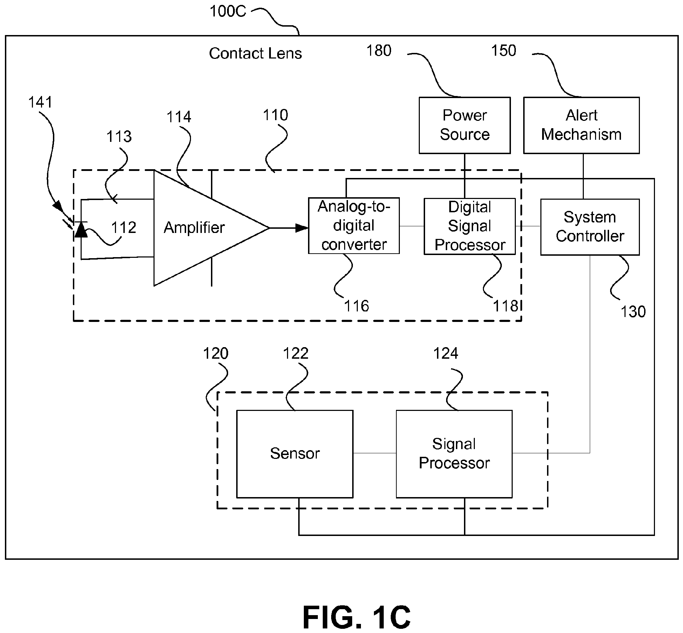

FIG. 1C illustrates, in block diagram form, a contact lens 100C in accordance with at least one exemplary embodiment. In the illustrated embodiment, the contact lens 100C includes an eyelid position system 110, an eye movement sensor system 120, a system controller 130, an alert mechanism 150, and a power source 180.

The illustrated eyelid position sensor system 110 in FIG. 1C includes at least one sensor in electrical communication with a signal processing component(s). The at least one sensor allows for the detection of eyelid closure and may take a variety of forms as is discussed later in this disclosure. The illustrated eyelid position system 110 includes a photosensor 112, an amplifier 114, an analog-to-digital converter (or ADC) 116, and a digital signal processor 118.

The illustrated eye movement sensor system 120 in FIG. 1C includes at least one sensor in electrical communication with a signal processor. The at least one sensor may take a variety of forms as is discussed later in this disclosure. Examples include an accelerometer and a transducer. The illustrated eye movement sensor system 120 includes a sensor 122 and a signal processor 124 such as an acquisition sampling signal conditioner.

In an alternative exemplary embodiment, an integrated circuit or other electrical component that houses the system controller also houses the signal processing of the two sensor systems.

When the contact lens 100C is placed onto the front surface of a user's eye the electronic circuitry of the blink detector system may be utilized to implement the blink detection in at least one exemplary embodiment. The photosensor 112, as well as the other circuitry, is configured to detect blinks, various blink patterns produced by the user's eye, and/or level of eyelid closure.

In this example embodiment, the photosensor 112 may be embedded into the contact lens 100C and receives ambient light 141, converting incident photons into electrons and thereby causing a current, indicated by arrow 113, to flow into the amplifier 114. The photosensor or photodetector 112 may include any suitable device. In one exemplary embodiment, the photosensor 112 includes a photodiode. In at least one embodiment, the photodiode is implemented in a complimentary metal-oxide semiconductor (CMOS process technology) to increase integration ability and reduce the overall size of the photosensor 112 and the other circuitry. The current 113 is proportional to the incident light level and decreases substantially when the photodetector 112 is covered by an eyelid. The amplifier 114 creates an output proportional to the input, with gain, and may function as a transimpedance amplifier which converts input current into output voltage. The amplifier 114 may amplify a signal to a usable level for the remainder of the system, such as giving the signal enough voltage and power to be acquired by the ADC 116. For example, the amplifier 114 may be necessary to drive subsequent blocks since the output of the photosensor 112 may be quite small and may be used in low-light environments. The amplifier 114 may be implemented as a variable-gain amplifier, the gain of which may be adjusted by the system controller 130, in a feedback arrangement, to maximize the dynamic range of the system. In addition to providing gain, the amplifier 114 may include other analog signal conditioning circuitry, such as filtering and other circuitry appropriate to the photosensor 112 and amplifier 114 outputs. The amplifier 114 may include any suitable device for amplifying and conditioning the signal output by the photosensor 112. For example, the amplifier 114 may include a single operational amplifier or a more complicated circuit having one or more operational amplifiers. The photosensor 112 may be a switchable array of photodiodes, and the amplifier 114 may be an integrator. As set forth above, the photosensor 112 and the amplifier 114 are configured to detect and isolate blink sequences based upon the incident light intensity received through the eye and convert the input current into a digital signal usable ultimately by the system controller 130. In at least one exemplary embodiment, the system controller 130 is preprogrammed or preconfigured to recognize various blink sequences, blink patterns, an/or eyelid closures (partial or complete) in various light intensity level conditions and provide appropriate control of the contact lens and/or an appropriate output signal to the alert mechanism 150. In at least one exemplary embodiment, the system controller 130 also includes associated memory.

In this exemplary embodiment, the ADC 116 may be used to convert a continuous, analog signal output from the amplifier 114 into a sampled, digital signal appropriate for further signal processing. For example, the ADC 116 may convert an analog signal output from the amplifier 114 into a digital signal that may be usable by subsequent or downstream circuits, such as a digital signal processor 118. The digital signal processor 118 may be utilized for digital signal processing, including one or more of filtering, processing, detecting, and otherwise manipulating/processing sampled data to permit incident light detection for downstream use. The digital signal processor 118 may be preprogrammed with the blink sequences and/or blink patterns described above along with a blink sequence indicating prolonged eyelid closure or eyelid drift. The digital signal processor 118, also in at least one exemplary embodiment, includes associated memory, which in at least one embodiment stores template and masks sets to detect, for example blink patterns for each operation state as selected by the system controller 130. The digital signal processor 118 may be implemented utilizing analog circuitry, digital circuitry, software, or a combination thereof. In the illustrated embodiment, it is implemented in digital circuitry. The ADC 116 along with the associated amplifier 114 and digital signal processor 118 are activated at a suitable rate in agreement with the sampling rate previously described, for example every one hundred (100) ms, which is subject to adjustment in at least one exemplary embodiment.

In at least one exemplary embodiment, any suitable device that allows for detection of movement of the eye and more particularly the pupil may be utilized as the sensor 122, and more than a single sensor 122 may be utilized. The output of the sensor 122 is acquired, sampled, and conditioned by signal processor 124. The signal processor 124 may include any number of devices including an amplifier, a transimpedance amplifier, an analog-to-digital converter, a filter, a digital signal processor, and related circuitry to receive data from the sensor 122 and generate output in a suitable format for the remainder of the system. The signal processor 124 may be implemented utilizing analog circuitry, digital circuitry, software, and/or a combination thereof In at least one exemplary embodiment, the signal processor 124 is co-designed with the sensor 122, for example, circuitry for acquisition and conditioning of an accelerometer are different than the circuitry for a muscle activity sensor or optical pupil tracker. The output of the signal processor 124 in at least one exemplary embodiment is a sampled digital stream and may include absolute or relative position, movement, detected gaze in agreement with convergence, or other data. System controller 130 receives input from the position signal processor 124 and uses this information, in conjunction with input from the eyelid position sensor system, to monitor the wearer.

In at least one exemplary embodiment, the signal processors 118 and 124 are combined into (or fabricated as) one signal processor.

Furthermore, the system controller 130 may control other aspects of a powered contact lens depending on input from the digital signal processor 118 and/or the signal processor 124, for example changing the focus or refractive power of an electronically controlled lens through an actuator.

In at least one exemplary embodiment, the system controller 130 will determine the operation state of the lens based on a received blink pattern, for example, to initiate or terminate monitoring although in an alternative embodiment other operational states are possible simultaneously or separately. Further to this embodiment or alternatively, the operation state will determine a set of templates and masks to be used by the digital signal processor 118 in that operation state along with control what the alert mechanism 150 does in response to an output from the system controller 130 detecting the wearer has a medical condition.

The system controller 130 uses the signal from the photosensor chain; namely, the photosensor 112, the amplifier 114, the ADC 116 and the digital signal processing system 118, to compare sampled light levels to determine eyelid closure and/or blink activation patterns.

FIG. 1D illustrates the system depicted in FIG. 1A where a contact lens 100D further includes a timing circuit 140. The timing circuit 140 provides a clock signal for operation of the electronic components on the contact lens requiring a clock signal. The timing circuit 140 in at least one exemplary embodiment includes an accumulator 142 for tracking the passing of time. An example of an accumulator is a register acting as a counter. In an alternative exemplary embodiment, the accumulator 142 is set to a value approximating the time in the future when the alarm is to be provided to the wearer and works in reverse counting down from that value, which leads to the system controller 130 performing a comparison of the reading to zero to determine when to send the alert signal. In alternative exemplary embodiments, the timing circuit 140 as illustrated in contact lens 100E in FIG. 1E may include an oscillator 144 having a crystal, for example quartz, a resistor-capacitor (RC), an inductor-capacitor (LC), and/or a relaxation circuitry. In a further exemplary embodiment, the oscillator frequency is maintained by a variable capacitor including a selectable array of capacitors, a varactor diode, and/or a variable resistor. In at least one exemplary embodiment, a register in electrical communication with the oscillator is adjusted, and the contents of the register are then decoded to provide adjustment of variable components leading to adjustment of the oscillator frequency.

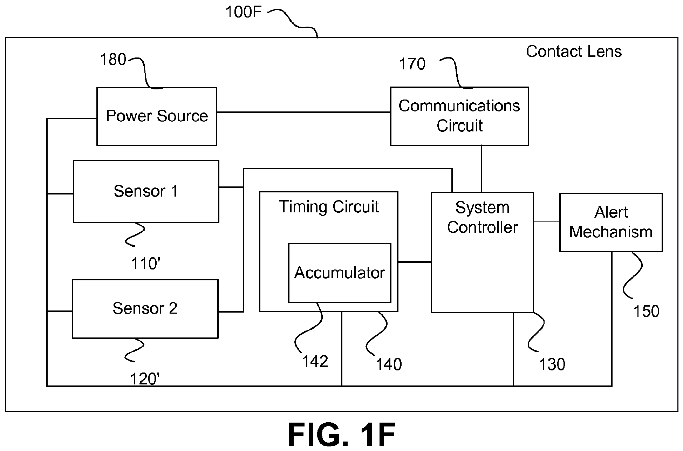

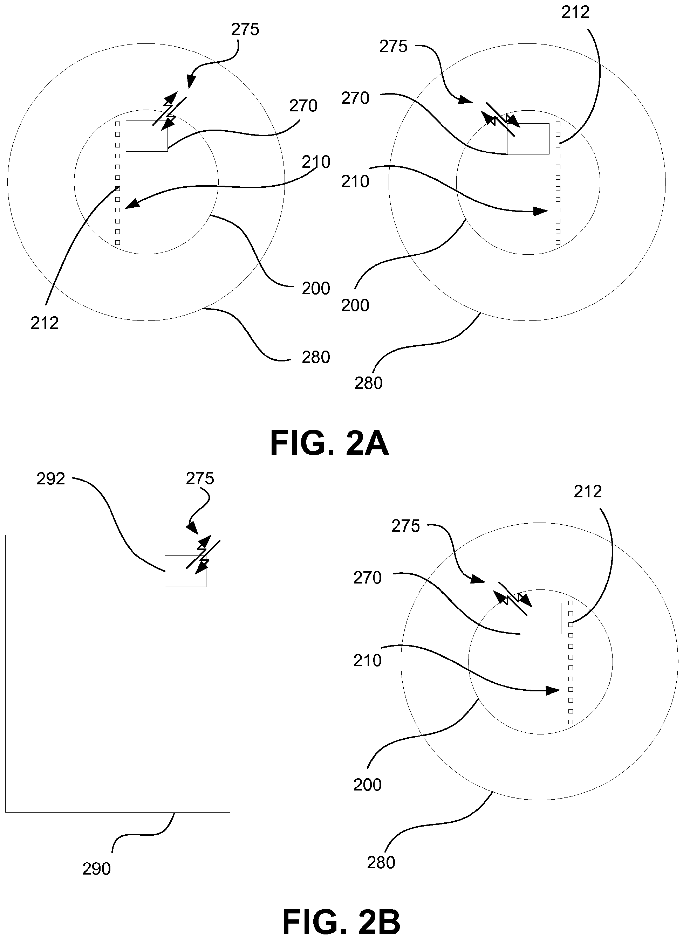

FIG. 1F illustrates the system depicted in FIG. 1A where a contact lens 100F further includes a communications circuit 170. The communications circuit 170 facilitates communication between the system controller 130 and another contact lens (as discussed, for example in connection with FIG. 2A) and/or an external device (as discussed, for example in connection with FIG. 2B). Examples of the external device include a fob, a cellular telephone, a smartphone, a smartwatch, a computer, and a mobile computing device including a tablet. The communications circuit 170 in at least one exemplary embodiment includes an antenna and a receiver. In a further alternate exemplary embodiment, the communications circuit 170 may include a transmitter in addition to the receiver or a transceiver. In a further alternative exemplary embodiment, the communications circuit 170 facilitates communication via radiofrequency (e.g., Bluetooth, ANT, or a custom protocol), sonic, ultrasonic, and light. One possible sonic/ultrasonic approach would be to use a speaker (e.g., a built-in speaker in a smartphone) to provide an audio signal towards a transducer or other receiver on the lens. One possible light approach other than a fob is to use a display (e.g., smartphone display, tablet display, computer display, or a television) to introduce a subliminal flash containing data for the lens to receive. In at least one exemplary embodiment, the communications circuit 170 and/or the eyelid position sensor system are examples of a user input, which in at least one exemplary embodiment is used to trigger the system controller 130 to store data and/or mark data.

In at least one exemplary embodiment, the timing circuit 140, the resource management system 160, and the communications circuit 170 are used in different combinations with the other elements including at least one sensor and with each other.

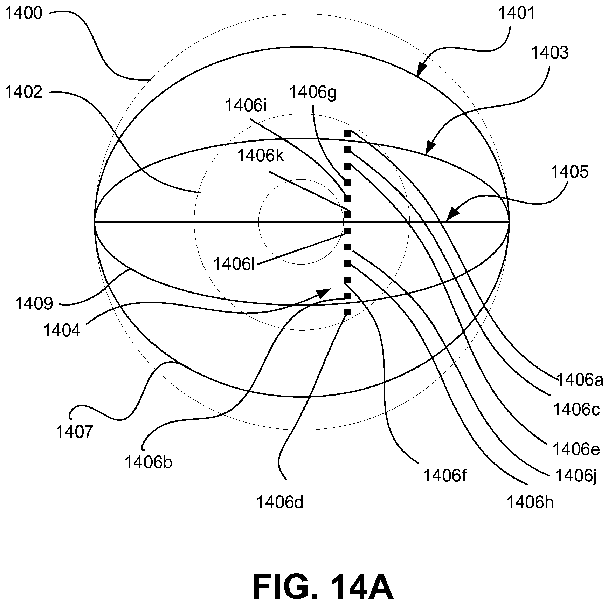

FIG. 2A illustrates a system in which two eyes 280 are at least partially covered with contact lenses 200. Sensor arrays 210 are present in both of the contact lenses 200 to determine lid position, as described with respect to FIGs. 14A-15B and 17A-18B subsequently. In this exemplary embodiment, the contact lenses 200 each include an electronic communication component 270, which is an example of a communications circuit 170 in FIG. 1F. Electronic communication component 270 in each contact lens 200 permits two-way communication to take place between the contact lenses 200. The electronic communication components 270 may include transmitters, receivers, RF transceivers, antennas, interface circuitry for photosensors 212 which comprise the sensor arrays 210, and associated or similar electronic components. The communication channel represented by line 275 may include RF transmissions at the appropriate frequency and power with an appropriate data protocol to permit effective communication between the contact lenses 200. Transmission of data between the two contact lenses 200 may, for example, verify that both lids have closed in order to detect a true, purposeful blink rather than a wink or involuntary blink. The transmission may also allow a system to determine if both eyelids have closed by a similar amount, for example, that which is associated with a user reading up-close; the pupils are substantially the same size; and/or the gaze direction of the eyes. Data transmission 275 may also take place from and/or to an communications component 292 in an external device 290, for example, spectacle glasses, or a smartphone (or other processor based system) as illustrated, for example in FIG. 2B. In at least one embodiment, the electronic communication components 270, for example, allow for the transmission of data to and receiving a response from the smartphone (or other external device) 290 having a communications component 292. As such the electronic communication components 270 may be present on just one lens in at least one alternative embodiment.

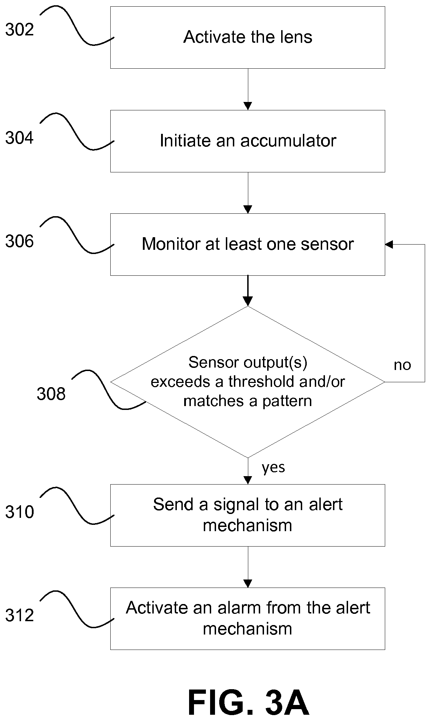

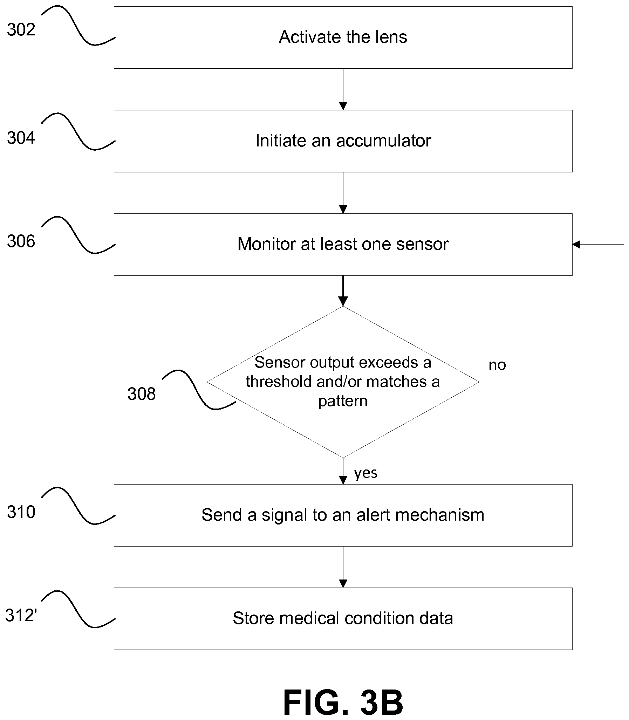

FIGs. 3A and 3B are flow charts that illustrate methods for medical monitoring with a powered ophthalmic lens. As discussed in this disclosure, there are a variety of ways to activate the powered ophthalmic lens, 302. In at least one exemplary embodiment when a timing circuit providing a time is not present and in response to activation of the powered ophthalmic lens, an accumulator is initiated on the lens to track a passage of time, 304. The system controller monitors at least one sensor at a first sampling rate, 306. The system controller determines whether the sensor output(s) exceeds/matches a threshold/pattern, 308. In at least one exemplary embodiment, when the threshold is exceeded or the pattern matched, then this is an indication of a medical condition having occurred and the system controller sends a signal to the alert mechanism, 310. Otherwise, the sensor is still monitored. FIG. 3A continues by having the alert mechanism activate an alarm, 312. Examples of the alarm are as discussed previously in this disclosure. In at least one exemplary embodiment the alarm continues while the method returns to further monitoring of the wearer. In an alternative embodiment, the alarm step is continued until receiving an instruction to stop and/or a determination that the medical condition has subsided.

FIG. 3B is identical to the method of FIG. 3A, but continues by having the alert mechanism store data relating to the medical condition, 312'. Examples of storing data include recording the current sensor data, storing the contents of a buffer or register containing the current and recent sensor data, and storing a threshold value if the threshold is adjustable. The storing the data may be with or without a time stamp from, for example the accumulator or the timing circuit. In at least one exemplary embodiment, the method returns to further monitoring of the wearer.

In an alternative exemplary embodiment, the pattern is used to predict when a medical condition is about to start to provide an alert to the wearer of the oncoming medical condition to allow the wearer to take appropriate action prior to the medical condition occurring. In a further alternative exemplary embodiment, the alert would be provided to another device. An example where this would be useful is in the situation where the wearer surfers from seizures, the alarm would give the person time to get in a predetermined position, insert a mouth implement, inform someone nearby, or other action to better protect his or her self during the seizure.

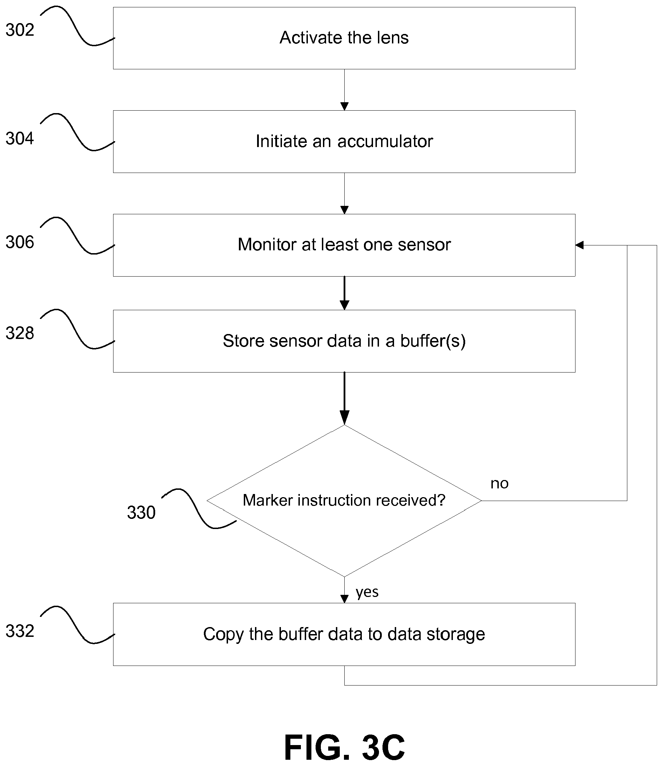

FIG. 3C illustrates an alternative method for medical monitoring with a powered ophthalmic lens. As discussed in this disclosure, there are a variety of ways to activate the powered ophthalmic lens, 302. In at least one exemplary embodiment when a timing circuit providing a time is not present and in response to activation of the powered ophthalmic lens, an accumulator is initiated on the lens to track a passage of time, 304. The system controller monitors at least one sensor at a first sampling rate, 306. The system controller stores the reading from the at least one sensor in a buffer(s), 328. The system controller, upon receiving a marking instruction, 330, copies the contents of the buffer(s) to data storage with or without using the alert mechanism along with a time stamp, 332. The time stamp may be obtained, for example, from the accumulator or the timing circuit. In at least one exemplary embodiment, the method returns to further monitoring of the at least one sensor.

Based on this disclosure, it should be understood that any of these methods may further include a termination step based on an instruction from, for example, the wearer; a resource management system; etc. In an alternative exemplary embodiment, the limitation of an accumulator is omitted when time stamps are not desired for a particular implementation.

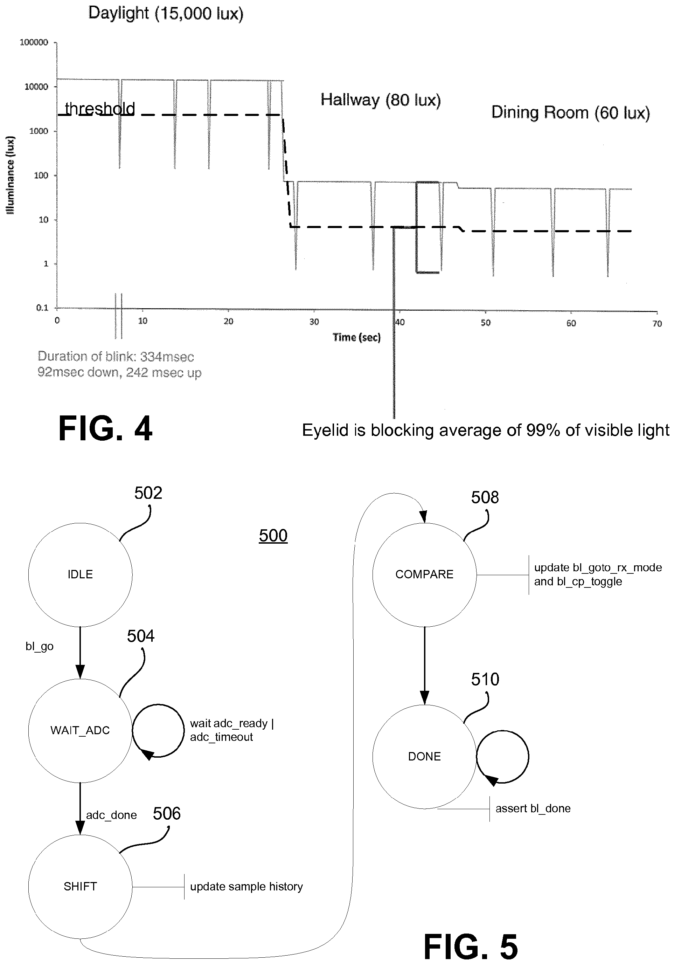

Referring to FIG. 4, a graphical representation of blink pattern samples recorded at various light intensity levels versus time and a usable threshold level is illustrated. Accordingly, accounting for various factors may mitigate and/or prevent error in detecting blinks when sampling light incident on the eye, such as accounting for changes in light intensity levels in different places and/or while performing various activities. Additionally, when sampling light incident on the eye, accounting for the effects that changes in ambient light intensity may have on the eye and eyelid may also mitigate and/or prevent error in detecting blinks, such as how much visible light an eyelid blocks when it is closed in low-intensity light levels and in high-intensity light levels, or used as part of a test protocol. In other words, in order to prevent erroneous blinking patterns from being utilized to control, the level of ambient light is preferably accounted for as is explained in greater detail below.

For example, in a study, it has been found that the eyelid on average blocks approximately ninety-nine (99) percent of visible light, but at lower wavelengths less light tends to be transmitted through the eyelid, blocking out approximately 99.6 percent of visible light. At longer wavelengths, toward the infrared portion of the spectrum, the eyelid may block only thirty (30) percent of the incident light. What is important to note; however, is that light at different frequencies, wavelengths and intensities may be transmitted through the eyelids with different efficiencies. For example, when looking at a bright light source, an individual may see red light with his or her eyelids closed. There may also be variations in how much visible light an eyelid blocks based upon an individual, such as an individual's skin pigmentation. As is illustrated in FIG. 4, data samples of blink patterns across various lighting levels are simulated over the course of a seventy (70) second time interval wherein the visible light intensity levels transmitted through the eye are recorded during the course of the simulation, and a usable threshold value is illustrated. The threshold is set at a value in between the peak-to-peak value of the visible light intensity recorded for the sample blink patterns over the course of the simulation at varying light intensity levels. Having the ability to preprogram blink patterns while tracking an average light level over time and adjusting a threshold may be critical to being able to detect when an individual is blinking, as opposed to when an individual is not blinking and/or there is just a change in light intensity level in a certain area.

The system controller in at least one exemplary embodiment uses a blink detection method to detect characteristics of blinks, for example, is the lid open or closed, the duration of the blink, the inter-blink duration, and the number of blinks in a given time period. In at least one exemplary embodiment, the blink detection method relies on sampling light incident on the eye at a certain sample rate. Pre-determined blink patterns are stored and compared to the recent history of incident light samples. When patterns match, the blink detection method may trigger activity in the system controller, for example, to initiate a test, to initiate a monitoring session, to mark and/or store data, and/or to change operation of the lens. The blink detection method in at least one exemplary embodiment further distinguishes between the pre-determined blink patterns and the eyelid movements associated with a medical condition, drowsiness, sleep onset, or sleep.

Blinking is the rapid closing and opening of the eyelids, and is an essential function of the eye. Blinking protects the eye from foreign objects, for example, individuals blink when objects unexpectedly appear in proximity to the eye. Blinking provides lubrication over the anterior surface of the eye by spreading tears. Blinking also serves to remove contaminants and/or irritants from the eye. Normally, blinking is done automatically, but external stimuli may contribute as in the case with irritants. Spontaneous blinking is a function of the individual and remains constant if the environment does not change. On average an individual blinks 12 -15 times per minute. However, when one is excited, blinking increases as it does when one is bored. Conversely, when concentrating, an individual's blink rate substantially decreases. Individuals also have blink reflexes; namely, a tactile reflex, an optic or dazzle reflex, an auditory reflex and a menace reflex. These blinking reflexes are discussed further subsequently. However, blinking may also be purposeful, for example, for individuals who are unable to communicate verbally or with gestures can blink once for yes and twice for no. The blink detection method and system of at least one exemplary embodiment utilizes blinking patterns that cannot be confused with normal blinking response. In other words, if blinking is to be utilized as a means for controlling an action, then the particular pattern selected for a given action cannot occur at random; otherwise, inadvertent actions may occur. As blink speed and/or frequency may be affected by a number of factors, including fatigue, concentration, boredom, eye injury, medication and disease, blinking patterns for control purposes preferably account for these and any other variables that affect blinking. The average length of involuntary blinks is in the range of about one hundred (100) to four hundred (400) milliseconds. Average adult men and women blink at a rate of ten (10) involuntary blinks per minute, and the average time between involuntary blinks is about 0.3 to seventy (70) seconds. Eyelid movements may also indicate other conditions such as drowsiness, as the eyelids have a general trend towards closing over a period of time or are closed for a period of time indicating that the wearer is asleep.

An exemplary embodiment of the blink detection method may be summarized in the following steps.
1. Define an intentional "blink sequence" that a user will execute for positive blink detection or that is representative of sleep onset.
2. Sample the incoming light level at a rate consistent with detecting the blink sequence and rejecting involuntary blinks.
3. Compare the history of sampled light levels to the expected "blink sequence," as defined by a blink template of values.
4. Optionally implement a blink "mask" sequence to indicate portions of the template to be ignored during comparisons, e.g. near transitions. This may allow for a user to deviate from a desired "blink sequence," such as a plus or minus one (1) error window, wherein one or more of lens activation, control, and focus change can occur. Additionally, this may allow for variation in the user's timing of the blink sequence. In a further exemplary embodiment, the concept of patterns and masks are applied to other sensor data to detect a medical condition using problem patterns and masks. In a further exemplary embodiment, the pattern is a template that in at least one embodiment includes at least one threshold.

A blink sequence may be defined as follows:
1. blink (closed) for 0.5 s
2. open for 0.5 s
3. blink (closed) for 0.5 s

At a one hundred (100) ms sample rate, a twenty (20) sample blink template is given by blink_template = [1,1,1, 0,0,0,0,0, 1,1,1,1,1, 0,0,0,0,0, 1,1].

The blink mask is defined to mask out the samples just after a transition (0 to mask out or ignore samples), and is given by
blink_mask = [1,1,1, 0,1,1,1,1, 0,1,1,1,1, 0,1,1,1,1, 0,1].

Optionally, a wider transition region may be masked out to allow for more timing uncertainty, and is given by
blink_mask = [1,1,0, 0,1,1,1,0, 0,1,1,1,0, 0,1,1,1,0, 0,1].

Alternate patterns may be implemented, e.g. single long blink, in this case a 1.5s blink with a 24-sample template, given by
blink_template = [1,1,1,1,0,0, 0,0,0,0,0,0, 0,0,0,0,0,0, 0,1,1,1,1,1].

A further alternative pattern may be implemented as indicative of sleep, in this case a 2.4s blink (or eyes that have closed for sleep) with a 24-sample template, given by
blink_template = [0,0,0,0,0,0, 0,0,0,0,0,0, 0,0,0,0,0,0, 0,0,0,0,0,0].
In an alternative embodiment, this blink_template is used without a blink_mask.

It is important to note that the above examples are for illustrative purposes and do not represent a specific set of data.

Detection may be implemented by logically comparing the history of samples against the template and mask. The logical operation is to exclusive-OR (XOR) the template and the sample history sequence, on a bitwise basis, and then verify that all unmasked history bits match the template. For example, as illustrated in the mask samples above, in each place of the sequence of a mask that the value is logic 1, a blink (or sensor state) has to match the mask template in that place of the sequence. However, in each place of the sequence of a mask that the value is logic 0, it is not necessary that a blink (or other sensor state) matches the mask template in that place of the sequence. For example, the following Boolean algorithm equation, as coded in MATLAB^{®} (MathWorks, Natick, Massachusetts), may be utilized
matched = not (mask) | not (xor (template, test_sample)),
wherein test_sample is the sample history. The matched value is a sequence with the same length as the template, sample history and mask. If the matched sequence is all logic 1's, then a good match has occurred. Breaking it down, not (xor (template, test_sample)) gives a logic 0 for each mismatch and a logic 1 for each match. Logic "oring" with the inverted mask forces each location in the matched sequence to a logic 1 where the mask is a logic 0. Accordingly, the more places in a mask template where the value is specified as logic 0, the greater the margin of error in relation to a person's blinks (or other sensor state) is allowed. It is also important to note that the greater the number of logic 0's in the mask template, the greater the potential for false positive matched to expected or intended patterns. It should be appreciated that a variety of expected or intended patterns may be programmed into a device with one or more active at a time and in at least one embodiment control the use of particular patterns to be used in a particular operation state. More specifically, multiple expected or intended patterns may be utilized for the same purpose or functionality, or to implement different or alternate functionality. For example, one pattern may be utilized to cause the lens to change operation state, terminate the monitoring, and/or initiate the monitoring. The blink detection in at least one embodiment also can detect when the eyelids remain closed, which would be detected as a continuous blink.

FIGs. 5-18D provide examples of eyelid position sensor systems (or blink detection sensor systems). In at least one exemplary embodiment, the eyelid position sensor systems use blink detection to determine whether the eyelid is closed and remains closed over a period of time.

FIG. 5 illustrates a state transition diagram 500 for a blink detection system in accordance with at least one embodiment. The system starts in an IDLE state 502 waiting for an enable signal b1_go to be asserted. When the enable b1_go signal is asserted, for example, by an oscillator and control circuit which pulses b1_go at a one hundred (100) ms rate commensurate with the blink sampling rate, the state machine then transitions to a WAIT_ADC state 504 in which an ADC is enabled to convert a received light level to a digital value. The ADC asserts an adc_done signal to indicate its operations are complete, and the system or state machine transitions to a SHIFT state 506. In the SHIFT state 506 the system pushes the most recently received ADC output value onto a shift register to hold the history of blink samples. In some embodiments, the ADC output value is first compared to a threshold value to provide a single bit (1 or 0) for the sample value, in order to minimize storage requirements. The system or state machine then transitions to a COMPARE state 508 in which the values in the sample history shift register are compared to one or more blink sequence templates and masks as described above. If a match is detected, one or more output signals may be asserted, such as one to switch the state of the lens to an asleep operation state or an awake operation state or to signal onset of sleep by the wearer. The system or state machine then transitions to the DONE state 510 and asserts a bl_done signal to indicate its operations are complete.

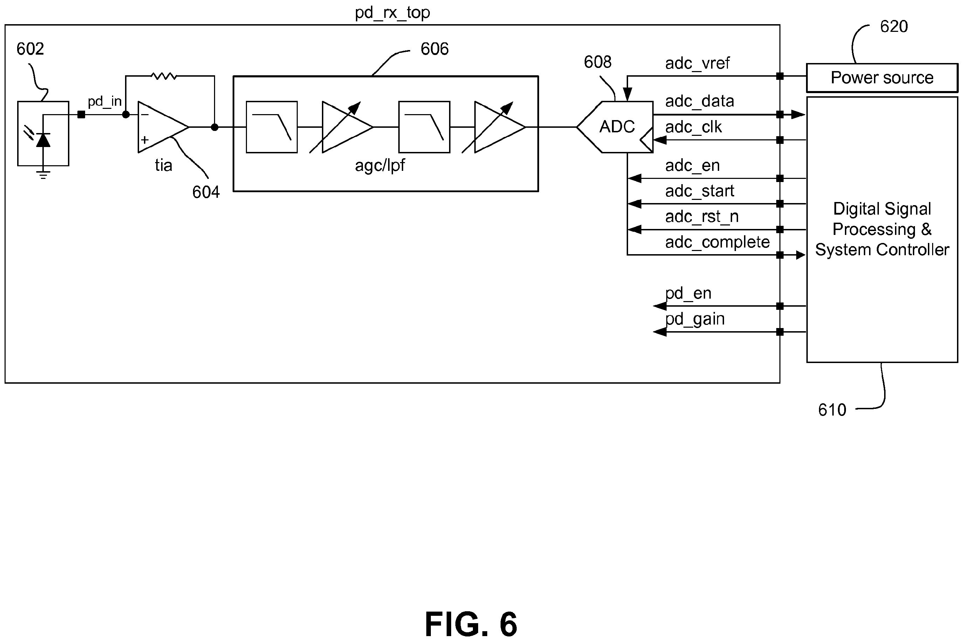

FIG. 6 illustrates a photosensor or photodetector signal path pd_rx_top that may be used to detect and sample received light levels. The signal path pd_rx_top may include a photodiode 602, a transimpedance amplifier 604, an automatic gain and low pass filtering stage 606 (AGC/LPF), and an ADC 608. The adc_vref signal is input to the ADC 608 from the power source 620 (*see, e.g.,* power source 180 in FIGs. 1A-1F) or alternately it may be provided from a dedicated circuit inside the analog-to-digital converter 608. The output from the ADC 608, adc_data, is transmitted to the digital signal processing and system controller block 118/130 (*see* FIG. 1C). Although illustrated in FIG. 1C as individual blocks 118 and 130, for ease of explanation, the digital signal processor 118 and system controller 130 may be implemented on a single block 610. The enable signal, adc_en, the start signal, adc_start, and the reset signal, adc_rst_n are received from the digital signal processing and system controller 610 while the complete signal, adc_complete, is transmitted thereto. The clock signal, adc_clk, may be received from a clock source external to the signal path, pd_rx_top, or from the digital signal processing and system controller 610. It is important to note that the adc_clk signal and the system clock may be running at different frequencies. It is also important to note that any number of different ADCs may be utilized in accordance with at least one embodiment which may have different interface and control signals but which perform a similar function of providing a sampled, digital representation of the output of the analog portion of the photosensor signal path. The photodetect enable, pd_en, and the photodetect gain, pd_gain, are received from the digital signal processing and system controller 610.

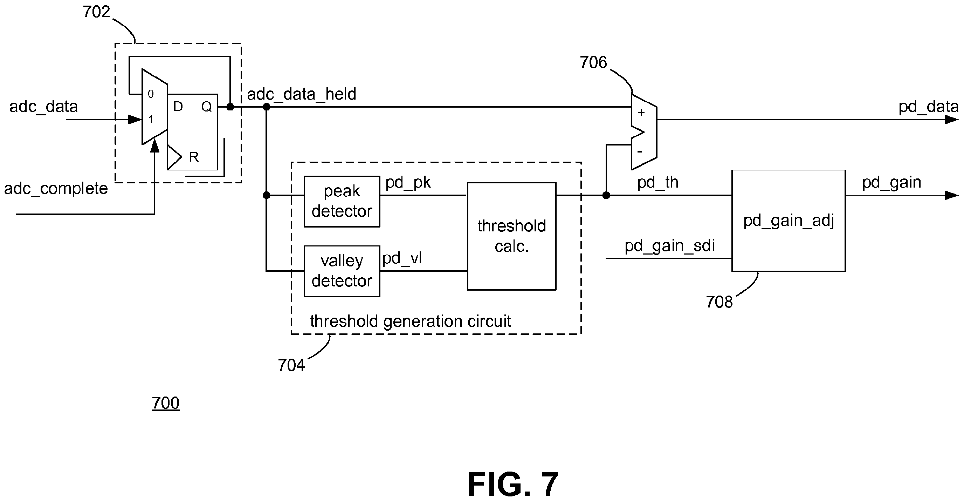

FIG. 7 illustrates a block diagram of digital conditioning logic 700 that may be used to reduce the received ADC signal value, adc_data, to a single bit value pd_data. The digital conditioning logic 700 may include a digital register 702 to receive the data, adc_data, from the photodetection signal path pd_rx_top to provide a held value on the signal adc_data_held. The digital register 702 is configured to accept a new value on the adc_data signal when the adc_complete signal is asserted and to otherwise hold the last accepted value when the adc_complete signal is received. In this manner the system may disable the photodetection signal path once the data is latched to reduce system current consumption. The held data value may then be averaged, for example, by an integrate-and-dump average or other averaging methods implemented in digital logic, in the threshold generation circuit 704 to produce one or more thresholds on the signal pd_th. The held data value may then be compared, via comparator 706, to the one or more thresholds to produce a one-bit data value on the signal pd data. It will be appreciated that the comparison operation may employ hysteresis or comparison to one or more thresholds to minimize noise on the output signal pd_data. The digital conditioning logic may further include a gain adjustment block pd_gain_adj 708 to set the gain of the automatic gain and low-pass filtering stage 606 in the photodetection signal path via the signal pd_gain, illustrated in FIG. 6, according to the calculated threshold values and/or according to the held data value. It is important to note that in this exemplary embodiment six bit words provide sufficient resolution over the dynamic range for blink detection while minimizing complexity. FIG. 7 illustrates an alternative embodiment that includes providing a pd_gain_sdi control signal from, for example, the serial data interface that allows one to override the automatic gain control determined by gain adjustment block pd_gain_adj 708.

In one exemplary embodiment, the threshold generation circuit 704 includes a peak detector, a valley detector and a threshold calculation circuit. In this embodiment, the threshold and gain control values may be generated as follows. The peak detector and the valley detector are configured to receive the held value on signal adc_data_held. The peak detector is further configured to provide an output value, pd_pk, which quickly tracks increases in the adc_data_held value and slowly decays if the adc_data_held value decreases. The operation is analogous to that of a classic diode envelope detector, as is well-known in the electrical arts. The valley detector is further configured to provide an output value pd_v1 which quickly tracks decreases in the adc_data_held value and slowly increases to a higher value if the adc_data_held value increases. The operation of the valley detector is also analogous to a diode envelope detector, with the discharge resistor tied to a positive power supply voltage. The threshold calculation circuit is configured to receive the pd_pl and pd_v1 values and is further configured to calculate a mid-point threshold value pd_th_mid based on an average of the pd_pk and pd_v1 values. The threshold generation circuit 704 provides the threshold value pd th based on the mid-point threshold value pd_th_mid.

The threshold generation circuit 704 may be further adapted to update the values of the pd_pk and pd_v1 levels in response to changes in the pd gain value. If the pd_gain value increases by one step, then the pd_pk and pd_v1 values are increased by a factor equal to the expected gain increase in the photodetection signal path. If the pd_gain value decreases by one step, then the pd_pk and pd_val values are decreased by a factor equal to the expected gain decrease in the photodetection signal path. In this manner the states of the peak detector and valley detectors, as held in the pd_pk and pd_v1 values, respectively, and the threshold value pd_th as calculated from the pd_pk and pd_v1 values are updated to match the changes in signal path gain, thereby avoiding discontinuities or other changes in state or value resulting only from the intentional change in the photodetection signal path gain.

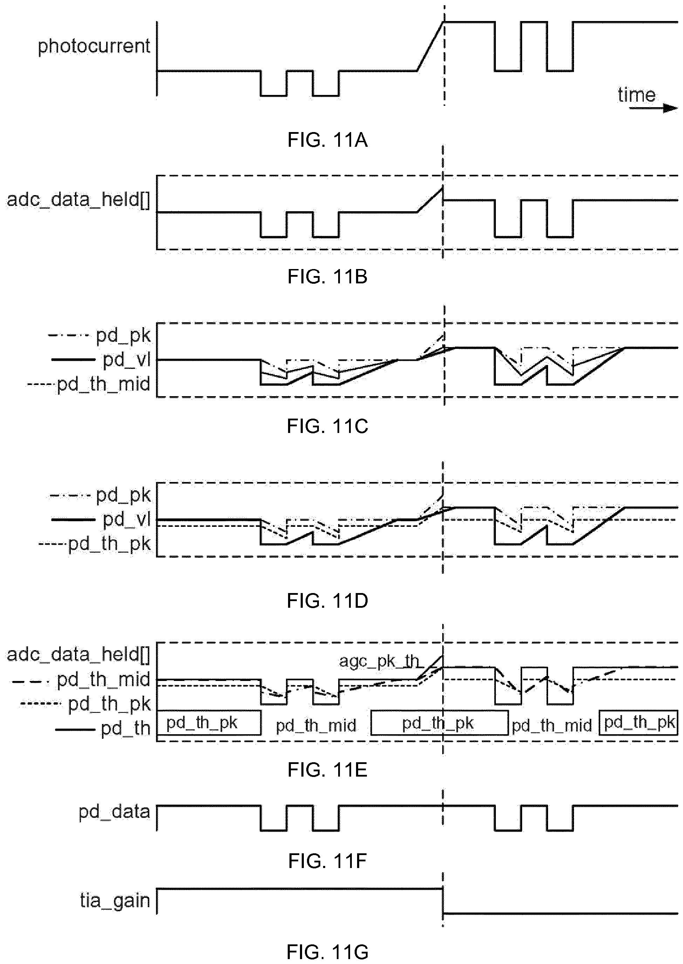

In a further exemplary embodiment of the threshold generation circuit 704, the threshold calculation circuit may be further configured to calculate a threshold value pd_th_pk based on a proportion or percentage of the pd_pk value. In at least one embodiment the pd_th_pk may be advantageously configured to be seven eighths of the pd_pk value, a calculation which may be implemented with a simple right shift by three bits and a subtraction as is well-known in the relevant art. The threshold calculation circuit may select the threshold value pd_th to be the lesser of pd_th_mid and pd_th_pk. In this manner, the pd_th value will never be equal to the pd_pk value, even after long periods of constant light incident on the photodiode which may result in the pd_pk and pd_v1 values being equal. It will be appreciated that the pd_th_pk value ensures detection of a blink after long intervals. The behavior of the threshold generation circuit is further illustrated in FIGs. 11A-11G, as discussed subsequently.

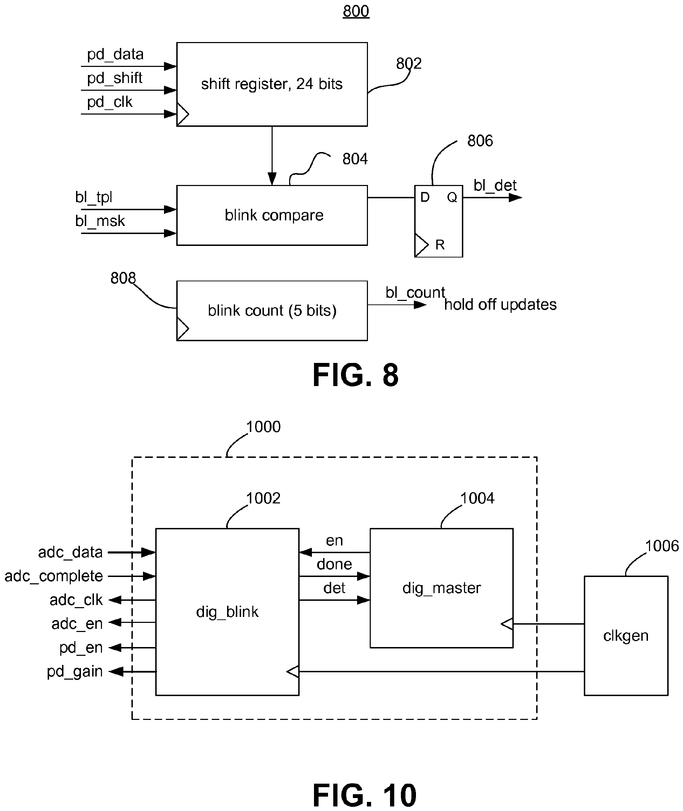

FIG. 8 illustrates a block diagram of digital detection logic 800 that may be used to implement a digital blink detection algorithm in accordance with at least one exemplary embodiment. The digital detection logic 800 may include a shift register 802 adapted to receive the data from the photodetection signal path pd_rx_top, FIG. 6, or from the digital conditioning logic, FIG. 7, as illustrated here on the signal pd_data, which has a one bit value. The shift register 802 holds a history of the received sample values, here in a 24-bit register. The digital detection logic 800 further includes a comparison block 804, adapted to receive the sample history and one or more templates bl tpl and masks bl-mask based on operation state (if necessary), and is configured to indicate a match to the one or more templates and masks on one or more output signals that may be held for later use. In at least one embodiment, the operation state determines the set of templates bl_tpl and masks bl_mask to be used by the comparison block 804. In at least one set of the templates bl_tpl, there is at least one sleep template representative of the wearer falling asleep. In an alternative exemplary embodiment, the digital detection logic 800 includes a comparison block, adapted to contain one or more sleep templates, and is configured to indicate a match to the one or more templates and masks on one or more output signals that may be held for later use. In such an alternative exemplary embodiment, the lens does not have asleep and awake operation states.

The output of the comparison block 804 is latched via a D flip-flop 806. The digital detection logic 800 may further include a counter 808 or other logic to suppress successive comparisons that may be on the same sample history set at small shifts due to the masking operations. In a preferred embodiment the sample history is cleared or reset after a positive match is found, thus requiring a full, new matching sequence to be sampled before being able to identify a subsequent match. The digital detection logic 800 may still further include a state machine or similar control circuitry to provide the control signals to the photodetection signal path and the ADC. In some embodiments the control signals may be generated by a control state machine that is separate from the digital detection logic 800. This control state machine may be part of the digital signal processing and system controller 610 (*see* FIG. 6).

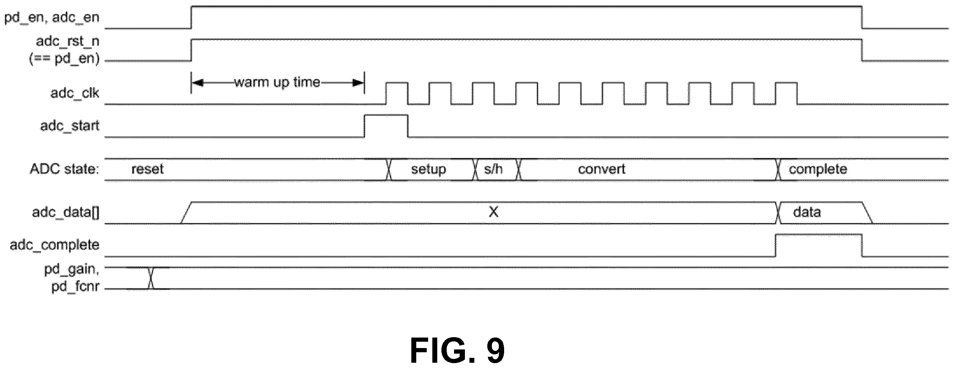

FIG. 9 illustrates a timing diagram of the control signals provided from a detection subsystem to an ADC 608 (FIG. 6) used in a photodetection signal path. The enable and clock signals adc_en, adc_rst_n and adc_clk are activated at the start of a sample sequence and continue until the analog-to-digital conversion process is complete. In one embodiment the ADC conversion process is started when a pulse is provided on the adc_start signal. The ADC output value is held in an adc_data signal and completion of the process is indicated by the analog-to-digital converter logic on an adc_complete signal. Also illustrated in FIG. 9 is the pd_gain signal which is utilized to set the gain of the amplifiers before the ADC. This signal is shown as being set before the warm-up time to allow the analog circuit bias and signal levels to stabilize prior to conversion.

FIG. 10 illustrates a digital system controller 1000 having a digital blink detection subsystem dig_blink 1002. The digital blink detection subsystem dig_blink 1002 may be controlled by a master state machine dig_master 1004 and may be adapted to receive clock signals from a clock generator clkgen 1006 external to the digital system controller 1000. The digital blink detection subsystem dig_blink 1002 may be adapted to provide control signals to and receive signals from a photodetection subsystem as described above. The digital blink detection subsystem dig_blink 1002 may include digital conditioning logic and digital detection logic as described above, in addition to a state machine to control the sequence of operations in a blink detection algorithm. The digital blink detection subsystem dig_blink 1002 may be adapted to receive an enable signal from the master state machine 1004 and to provide a completion or done indication and a blink detection indication back to the master state machine 1004.

In an alternative exemplary embodiment to the embodiment illustrated in FIG. 10, a time clock is connected to the clock generator 1006 (in FIG. 10) to track time since the lens began operation and provide a time stamp signal to the data manager in an embodiment where the data manager records data regarding the initiation and termination of sleep by the wearer such that when data is transmitted (or sent) from the lens to an external device using, for example, at least one electronic communication component, the external device is able to determine what time periods the wearer was asleep while wearing the lens by reverse calculating the time of day based on the time stamp from the lens and the current time on the external device when the data is transmitted as compared to the logged time stamps.

FIGs. 11A-11G depict waveforms to illustrate the operation of the threshold generation circuit and automatic gain control (FIG. 7). FIG. 11A illustrates an example ofphotocurrent versus time as might be provided by a photodiode in response to varying light levels. In the first portion of the plot, the light level and resulting photocurrent are relatively low compared to in the second portion of the plot. In both the first and second portions of the plot a double blink is seen to reduce the light and photocurrent. Note that the attenuation of light by the eyelid may not be one hundred (100) percent, but a lower value depending on the transmission properties of the eyelid for the wavelengths of light incident on the eye. FIG. 11B illustrates the adc_data_held value that is captured in response to the photocurrent waveform of FIG. 11A. For simplicity, the adc_data_held value is illustrated as a continuous analog signal rather than a series of discrete digital samples. It will be appreciated that the digital sample values will correspond to the level illustrated in FIG. 11B at the corresponding sample times. The dashed lines at the top and bottom of the plot indicate the maximum and minimum values of the adc_data and adc_data_held signals. The range of values between the minimum and maximum is also known as the dynamic range of the adc_data signal. As discussed below, the photodection signal path gain is different (lower) in the second portion of the plot. In general, the adc_data_held value is directly proportional to the photocurrent, and the gain changes only affect the ratio or the constant of proportionality. FIG. 11C illustrates the pd_pk, pd vl and pd_th_mid values calculated in response to the adc_data_held value by the threshold generation circuit. FIG. 11D illustrates the pd_pk, pd_v1 and pd_th_pk values calculated in response to the adc_data_held value in some embodiments of the threshold generation circuit. Note that the pd_th_pk value is always some proportion of the pd_pk value. FIG. 11E illustrates the adc_data_held value with the pd_th_mid and pd_th_pk values. Note that during long periods of time where the adc_data_held value is relatively constant the pd_th_mid value becomes equal to the adc_data_held value as the pd_v1 value decays to the same level. The pd_th_pk value always remains some amount below the adc_data_held value. Also illustrated in FIG. 11E is the selection of pd_th where the pd th value is selected to be the lower of pd_th_pk and pd_th_mid. In this way the threshold is always set some distance away from the pd_pk value, avoiding false transitions on pd data due to noise on the photocurrent and adc_data held signals. FIG. 11F illustrates the pd_data value generated by comparison of the adc_data_held value to the pd_th value. Note that the pd_data signal is a two-valued signal which is low when a blink is occurring. FIG. 11G illustrates a value of tia_gain versus time for these example waveforms. The value of tia_gain is set lower when the pd_th starts to exceed a high threshold shown as agc_pk_th in FIG. 11E. It will be appreciated that similar behavior occurs for raising tia_gain when pd_th starts to fall below a low threshold. Looking again at the second portion of each of FIGs. 11A-11E the effect of the lower tia_gain is clear. In particular note that the adc_data_held value is maintained near the middle of the dynamic range of the adc_data and adc_data_held signals. Further, it is important to note that the pd_pk and pd_v1 values are updated in accordance with the gain change as described above such that discontinuities are avoided in the peak and valley detector states and values due solely to changes in the photodetection signal path gain.

Additional exemplary embodiments of blink detection may allow for more variation in the duration and spacing of the blink sequence, for example, by timing the start of a second blink based on the measured ending time of a first blink rather than by using a fixed template or by widening the mask "don't care" intervals (0 values).

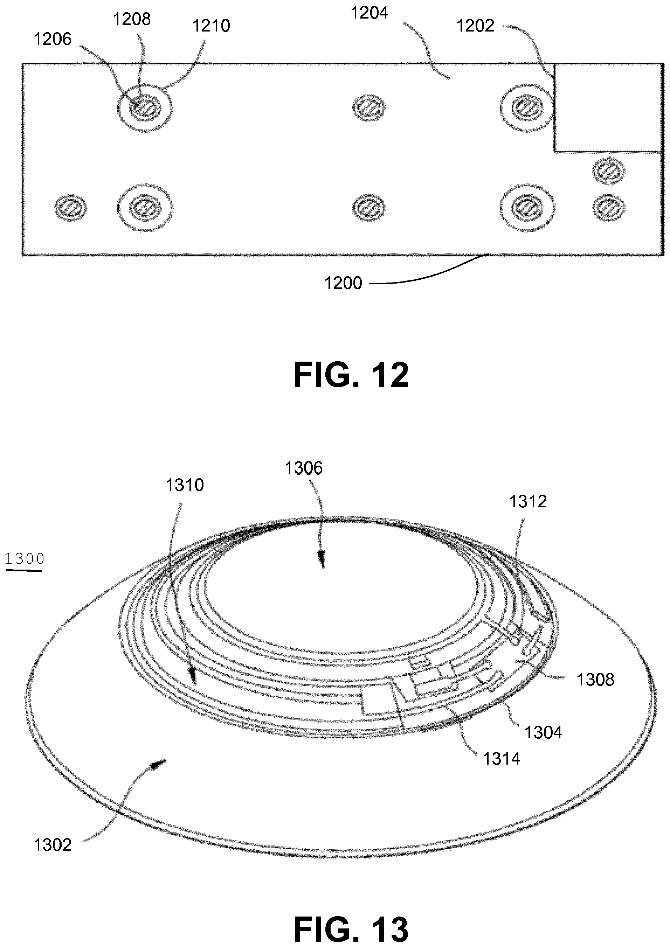

FIG. 12 illustrates light-blocking and light-passing features on an integrated circuit die 1200. The integrated circuit die 1200 includes a light passing region 1202, a light blocking region 1204, bond pads 1206, passivation openings 1208, and light blocking layer openings 1210. The light-passing region 1202 is located above the photosensors (not illustrated), for example, an array of photodiodes implemented in the semiconductor process. In at least one embodiment, the light-passing region 1202 permits as much light as possible to reach the photosensors thereby maximizing sensitivity. This may be done through removing polysilicon, metal, oxide, nitride, polyimide, and other layers above the photoreceptors, as permitted in the semiconductor process utilized for fabrication or in post-processing. The light-passing area 1202 may also receive other special processing to optimize light detection, for example, an anti-reflective coating, filter, and/or diffuser. The light-blocking region 1204 may cover other circuitry on the die which does not require light exposure. The performance of the other circuitry may be degraded by photocurrents, for example, shifting bias voltages and oscillator frequencies in the ultra-low current circuits required for incorporation into contact lenses, as mentioned previously. The light-blocking region 1204 is formed with a thin, opaque, reflective material, for example, aluminum or copper already used in semiconductor wafer processing and post-processing. If implemented with metal, the material forming the light-blocking region 1204 must be insulated from the circuits underneath and the bond pads 1206 to prevent short-circuit conditions. Such insulation may be provided by the passivation already present on the die as part of normal wafer passivation, e.g. oxide, nitride, and/or polyimide, or with other dielectric added during post-processing. Masking permits light blocking layer openings 1210 so that conductive light-blocking metal does not overlap bond pads on the die. The light-blocking region 1204 is covered with additional dielectric or passivation to protect the die and avoid short-circuits during die attachment. This final passivation has passivation openings 1208 to permit connection to the bond pads 1206.

In an alternative exemplary embodiment where the contact lens includes tinting capabilities, the light-passing region 1202 is at least partially overlapping with the region of the contact lens capable of being tinted. Where the photosensors are present in both the tinting region and non-tinting regions of the contact lens, it allows for a determination of the amount of light being blocked by the tinting. In a further exemplary embodiment, the entire light-passing region 1202 is present in the tinting region.

FIG. 13 illustrates a contact lens with an electronic insert having a blink detection system. The contact lens 1300 (have to change this in drawing) includes a soft plastic portion 1302 which provides an electronic insert 1304. This insert 1304 includes a lens 1306 which is activated by the electronics, for example, focusing near or far depending on activation. Integrated circuit 1308 mounts onto the insert 1304 and connects to batteries 1310, lens 1306, and other components as necessary for the system. In at least one embodiment, the integrated circuit 1308 includes a photosensor 1312 and associated photodetector signal path circuits. The photosensor 1312 faces outward through the lens insert and away from the eye, and is thus able to receive ambient light. The photosensor 1312 maybe implemented on the integrated circuit 1308 (as shown) for example as a single photodiode or array ofphotodiodes. The photosensor 1312 may also be implemented as a separate device mounted on the insert 1304 and connected with wiring traces 1314. When the eyelid closes, the lens insert 1304 including photodetector 1312 is covered, thereby reducing the light level incident on the photodetector 1312. The photodetector 1312 is able to measure the ambient light to determine if the user is blinking or not. Based on this disclosure one of ordinary skill in the art should appreciate that photodetector 1312 may be replaced or augmented by the other sensors discussed in this disclosure.

It will be appreciated that blink detection and/or sleep detection may be implemented in digital logic or in software running on a microcontroller. The algorithm logic or microcontroller may be implemented in a single application-specific integrated circuit (ASIC) with photodetection signal path circuitry and a system controller, or it may be partitioned across more than one integrated circuit.

In accordance with another exemplary embodiment, a powered or electronic ophthalmic lens may incorporate an eyelid or lid position sensor. It is known that the eyelids protect the globe in a number of ways, including the blink reflex and the tear spreading action. The blink reflex of the eyelids prevents trauma to the globe by rapidly closing upon a perceived threat to the eye. Blinking also spreads tears over the globe's surface to keep it moist and rinse away bacteria and other foreign matter. But the movement of the eyelids may also indicate other actions or functions at play beyond being used to alert an individual (or wearer) wearing an electronic ophthalmic lens that an alarm has been activated.

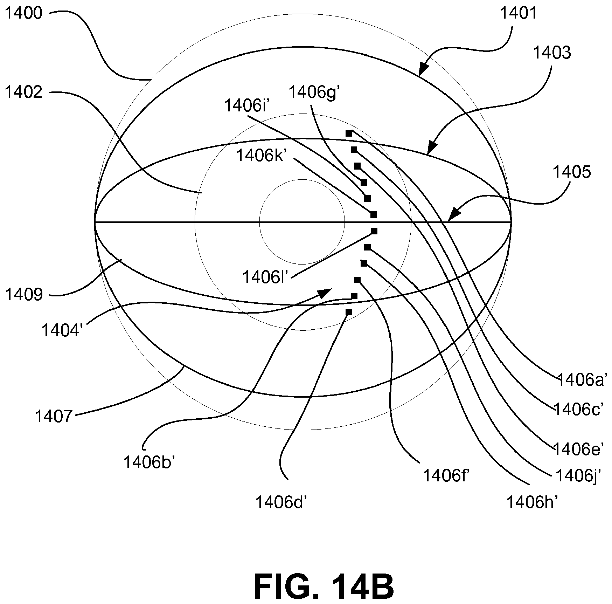

Referring now to FIG. 14A, there is illustrated an eyelid position sensor system on an eye 1400. The system is incorporated into a contact lens 1402. The top and bottom eyelids are shown, with the top lid having possible locations 1401, 1403, and 1405 in order of increasing closure. The bottom eyelid is also illustrated with levels of closure corresponding to the top lid; namely, locations 1407, 1409 and 1405. When the eyelids are closed, they occupy the same position; namely, 1405. The contact lens 1402 in accordance with this exemplary embodiment includes a sensor array 1404. This sensor array 1404 includes one or more photosensors. In this embodiment, the sensor array 1404 includes twelve (12) photosensors 1406a - 14061. With the top lid at position 1401 and the bottom lid at position 1407, all photosensors 1406a - 14061 are exposed and receive ambient light, thereby creating a photocurrent which may be detected by an electronic circuit described herein. With the lids partially closed at positions 1403 and 1409, the top and bottom photosensors 1406a and 1406b are covered, receive less light than the other photosensors 1406c - 14061, and output a correspondingly lower current which may be detected by the electronic circuit. With the lids totally closed in position 1405, all sensors 1406a - 14061 are covered with a corresponding reduction in current. This system may be used to detect lid position by sampling each photosensor in the sensor array and using the photocurrent output versus sensor position to determine lid position, for example, if the upper and lower eyelids do not fully open after blinks indicating potential onset of sleep or fatigue. It will be appreciated that the photosensors should be placed in suitable locations on the contact lens, for example, providing enough sample locations to reliably determine lid position while not obstructing the clear optic zone (roughly the area occupied by a dilated pupil.) This system may also be used to detect blinks by routinely sampling the sensors and comparing measurements over time. In an alternative exemplary embodiment, photosensors 1406a'-14061' of a sensor array 1404' form an arcuate pattern around the pupil while being vertically spaced from each other as illustrated, for example, in FIG. 14B. Under either of the illustrated embodiments, one of ordinary skill in the art should appreciate that a number other than 12 may be used in the sensor array. Further examples include a number in a range of 3 through 15 (including the end points in at least one embodiment), and more particularly a number in a range of 4 through 8 (including the end points in at least one embodiment).

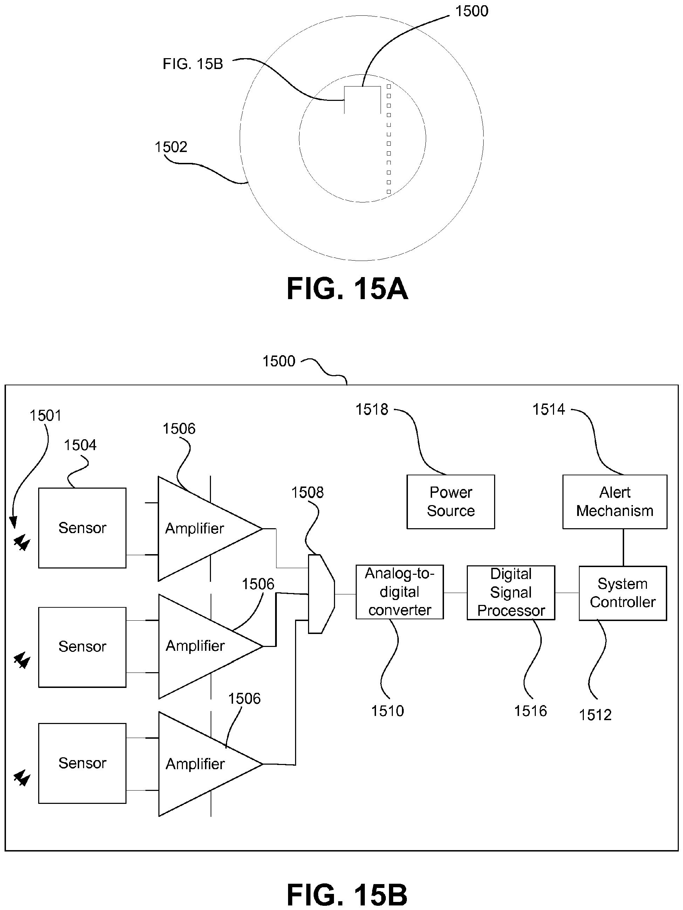

FIGs. 15A and 15B illustrate an electronic system 1500 in which lid position photosensors, as set forth above, are used to trigger activity in a contact lens 1502 or more specifically, a powered or electronic ophthalmic lens. FIG. 15A shows the electronic system 1500 on the lens 1502, and FIG. 15B is an exploded view of the system 1500. Light 1501 is incident onto one or more photosensors 1504 as previously described with respect to FIGs. 14A and 14B. These photosensors 1504 may be implemented with photodiodes, cadmium sulfide (CdS) sensors, or other technologies suitable for converting ambient light into current. Depending on the choice of photosensors 1504, amplifiers 1506 or other suitable circuitry may be required to condition the input signals for use by subsequent or downstream circuits. A multiplexer 1508 permits a single analog-to-digital converter (or ADC) 1510 to accept inputs from multiple photosensors 1504. The multiplexer 1508 may be placed immediately after the photosensors 1504, before the amplifiers 1506, or may not be used depending on considerations for current consumption, die size, and design complexity. Since multiple photosensors 1504 are needed at various positions on the eye to detect lid position, sharing downstream processing components (for example amplifiers, an analog-to-digital converter, and digital signed system controllers) may significantly reduce the size needed for the electronic circuitry. The amplifiers 1506 create an output proportional to the input, with gain, and may function as transimpedance amplifiers which convert input current into output voltage. The amplifiers 1506 may amplify a signal to a usable level for the remainder of the system, such as giving the signal enough voltage and power to be acquired by the ADC 1510. For example, the amplifiers 1506 may be necessary to drive subsequent blocks since the output of the photosensors 1504 may be quite small and may be used in low-light environments. Amplifiers 1506 may also be implemented as variable-gain amplifiers, the gain of which may be adjusted by a system controller 1512 to maximize the dynamic range of the system 1500. In addition to providing gain, the amplifiers 1506 may include other analog signal conditioning circuitry, such as filtering and other circuitry appropriate to the photosensor 1504 and amplifier 1506 output. The amplifiers 1506 may be any suitable device for amplifying and conditioning the signal output by the photosensor 1504. For example, the amplifiers 1504 may simply be a single operational amplifier or a more complicated circuit including one or more operational amplifiers.

As set forth above, the photosensors 1504 and the amplifiers 1506 are configured to detect incident light 1501 at various positions on the eye and convert the input current into a digital signal usable ultimately by the system controller 1512. In at least one exemplary embodiment, the system controller 1512 is preprogrammed to sample each photosensor 1504 on the eye to detect lid position and provide an appropriate output signal to an alert mechanism 1514. The system controller 1512 also includes associated memory. The system controller 1512 may combine recent samples of the photosensors 1504 to preprogrammed patterns correlating to lid open and squinting positions. The system 1500 may need to differentiate between eyelid position changes, normal changes in ambient light, shadows, and other phenomena. This differentiation may be accomplished through proper selection of the sampling frequency, amplifier gain, and other system parameters, optimization of sensors placement in the contact lens, determination of lid position patterns, recording ambient light, comparing each photosensor to adjacent and all photosensors, and other techniques to discern lid position uniquely.

In at least one exemplary embodiment, the ADC 1510 may be used to convert a continuous, analog signal output from the amplifiers 1506 through the multiplexer into a sampled, digital signal appropriate for further signal processing. For example, the ADC 1510 may convert an analog signal output from the amplifiers 1506 into a digital signal that may be useable by subsequent or downstream circuits, such as a digital signal processing system or microprocessor 1516. A digital signal processing system or digital signal processor 1516 may be utilized for digital signal processing, including one or more of filtering, processing, detecting, and otherwise manipulating/processing sampled data to permit incident light detection for downstream use. The digital signal processor 1516 may be preprogrammed with various lid position and/or closure patterns. The digital signal processor 1516 also includes associated memory in at least one embodiment. The digital signal processor 1516 may be implemented utilizing analog circuitry, digital circuitry, software, and/or preferably a combination thereof. The ADC 1510 along with the associated amplifiers 1506 and digital signal processor 1516 are activated at a suitable rate in agreement with the sampling rate previously described, for example, every one hundred (100) ms.

A power source 1518 supplies power for numerous components including the eyelid position sensor system 1500. The power source 1518 may also be utilized to supply power to other components in the contact lens. The power may be supplied from a battery, energy harvester, or other suitable means as discussed previously. Essentially, any type of power source 1518 may be utilized to provide reliable power for all other components of the system. A lid position sensor array pattern, processed from analog to digital, may enable activation of the system controller 1512 or a portion of the system controller 1512. Furthermore, the system controller 1512 may control other aspects of a powered contact lens depending on input from the digital signal system controller 1508, for example, activating the alert mechanism 1514.

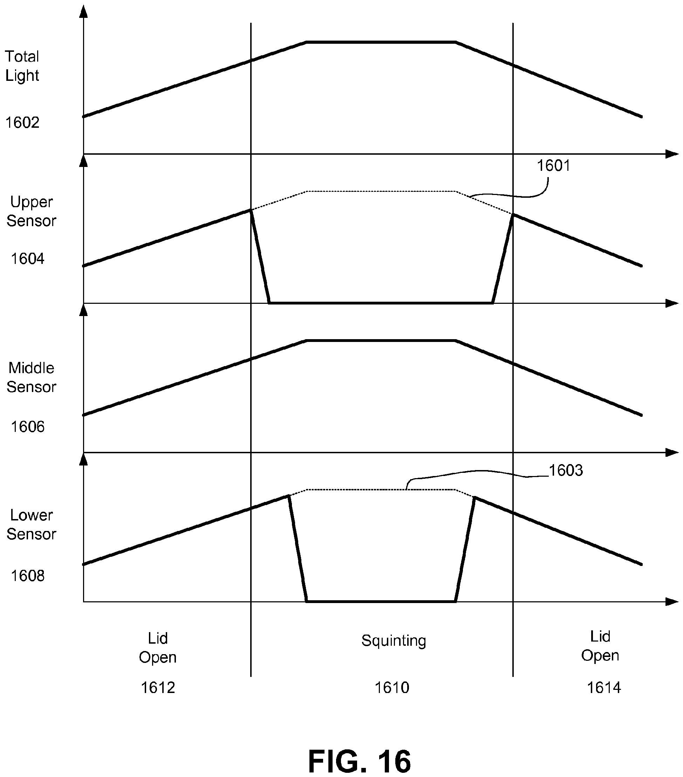

Referring now to FIG. 16 there is illustrated an output characteristic for three photosensors positioned at three different vertical positions on the contact lens. The output characteristics may represent the current proportional to incident light on each photosensor or may represent a downstream signal, for example, digital sampled data values versus time at the output of the ADC (element 1510 in FIG. 15B). Total incident light 1602 increases, holds steady, and then decreases, for example, when walking from a dark room to a bright hallway then back to a dark room. All three photosensors 1604, 1606, and 1608 would output a signal similar to that of the ambient light if the eyelid remained open, illustrated by dotted lines 1601 and 1603 for photosensors 1604 and 1608. In addition to the ambient light level 1602 changing, partial closure of the eyelids is indicated by position 1610, different than that of the lid open positions 1612 and 1614. When the lid partially closes, upper photosensor 1604 becomes covered by the upper eyelid and outputs a correspondingly lower level due to obstruction of the photosensor by the eyelid. Despite ambient light 1602 increasing, photosensor 1604 receives less light and outputs a lower signal due to the partially closed eyelid. Similar response is observed with photosensor 1608 which becomes covered. Middle sensor 1606 is not covered during squinting and thus continues to see the light level increase, with a corresponding increase in output level. While this example illustrates one particular case, it should be apparent how various configurations of sensor position and eyelid movement could be detected.

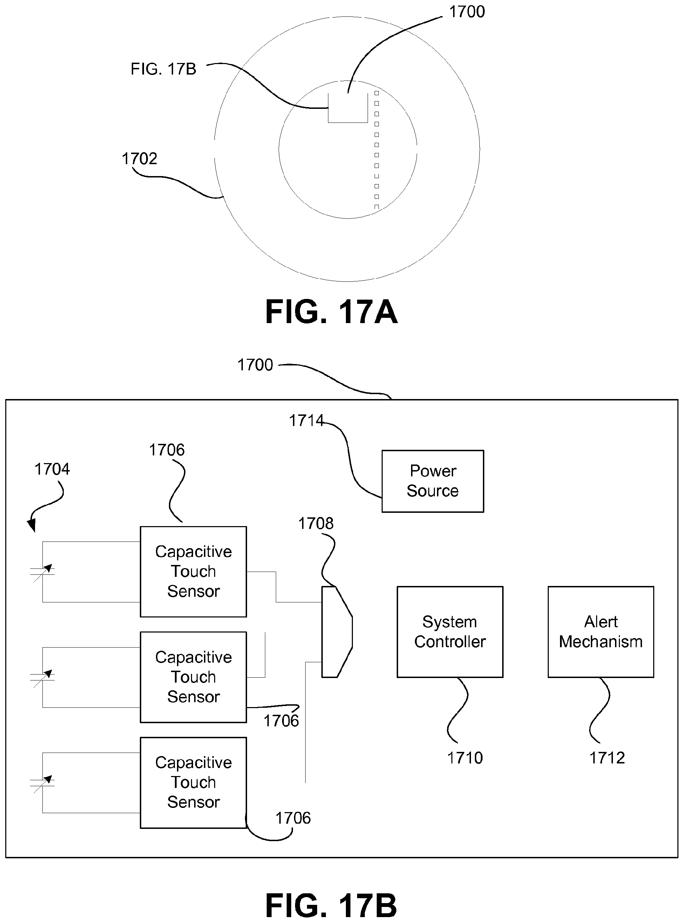

FIGs. 17A and 17B illustrate an alternative detection system 1700 incorporated into a contact lens 1702. FIG. 17A illustrates the system 1700 on the lens 1702 and FIG. 17B illustrates an exploded view of the system 1700. In this exemplary embodiment, capacitive touch sensors 1704 are utilized instead of photosensors. Capacitive touch sensors are common in the electronics industry, for example, in touch-screen displays. The basic principle is that a capacitive touch sensor (or variable capacitor) 1704 is implemented in a physical manner such that the capacitance varies with proximity or touch, for example, by implementing a grid covered by a dielectric. Sensor conditioners 1706 create an output signal proportional to the capacitance, for example, by measuring the change in an oscillator having the variable capacitor or by sensing the ratio of the variable capacitor to a fixed capacitor with a fixed-frequency AC signal. The output of the sensor conditioners 1706 may be combined with a multiplexer 1708 to reduce downstream circuitry. In this exemplary embodiment, the necessary signal conditioning circuitry as described above with respect to FIG. 15B is omitted for simplicity. A system controller 1710 receives inputs from the capacitance sensor conditioner 1706 via the multiplexor 1708, for example, by activating each sensor in order and recording the values. It may then compare measured values to pre-programmed patterns and historical samples to determine lid position. It may then activate a function in an alert mechanism 1712, for example, causing a variable-focus lens to change to a closer focal distance. The capacitor touch sensors 1704 may be laid out in a physical pattern similar to that previously described for the photodetectors, but in at least one embodiment would be optimized for detecting changes in capacitance with lid position. The sensors, and for that matter the whole electronic system, would be encapsulated and insulated from the saline contact lens environment. As the eyelid covers a sensor 1704, the change in capacitance would be detected rather than the change in ambient light previously described. FIG. 17B also illustrates the inclusion of a power source 1714 in at least one embodiment, which could take a variety of forms as previously discussed.

It is important to note that ADC's and digital signal processing circuitry may be utilized in accordance with the capacitive touch sensors if needed as illustrated with respect to the photosensors of FIG. 15B. In an alternative exemplary embodiment, the capacitive touch sensors are any pressure sensor. In a further exemplary embodiment, there is a combination of photosensors and pressure sensors on the lens.

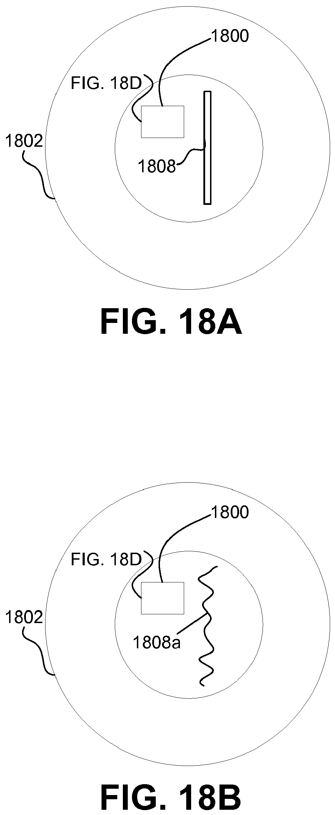

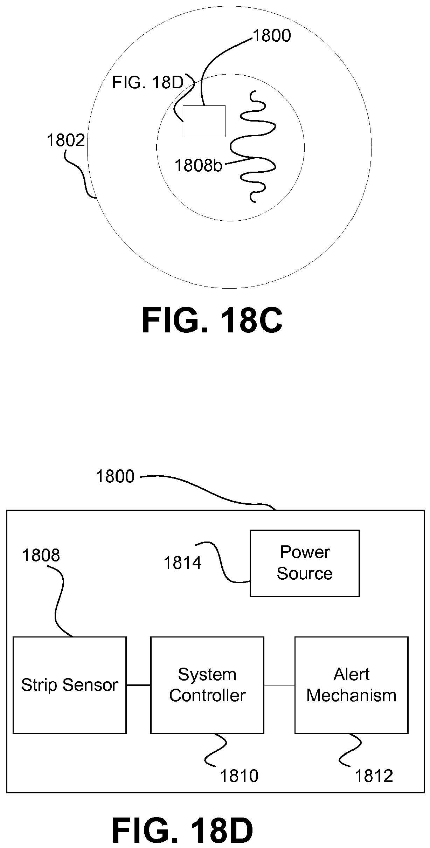

FIGs. 18A-18D illustrate an alternative exemplary embodiment where the lid position sensor system is a sensor having a strip 1808, 1808a, 1808b that covers a plurality of vertical points along the contact lens 1802 that works in conjunction with circuit 1800. One example of a sensor that may have a strip configuration is a capacitance sensor. FIG. 18A illustrates an example where the strip 1808 is substantially straight on the contact lens 1802. Although the strip 1808 is illustrated as being orientated parallel to a line bisecting the contact lens 1802, it may have an angled orientation relative to the bisecting line or have an arcuate shape. FIG. 18B illustrates an example where the strip 1808a takes a serpentine path along the contact lens 1802. In the embodiment illustrated in FIG. 18C, the serpentine configuration of strip 1808b will increase the change in capacitance detected by the circuit 1800 as the eyelids approach a closed state. The level of capacitance change will translate to the amount of eyelid closure. Another example of a sensor that may have a strip configuration is a piezoelectric pressure transducer with a diaphragm and a base having a strip configuration. As the eyelids close, additional pressure will be applied by the eyelids against the piezoelectric pressure transducer thus allowing for a determination of the level of eyelid closure. The continuous sensing along the vertical axis provides an improved granularity over a plurality of sensors thus providing improved measurement of the eyelid location. FIG. 18D illustrates an electrical circuit that may be used in conjunction with strip sensors 1808, 1808a, 1808b that includes a system controller 1810, an alert mechanism 1812 and a power source 1814 like those previously discussed. In a further alternative exemplary embodiment, there are multiple strips present. An advantage of an angled and/or serpentine strip configuration is that lid position may still be detected even if the contact lens is orientated incorrectly on the wearer's eye.

The activities of the digital signal processing block and system controller (1516 and 1512 in FIG. 15B, respectively, system controller 1710 in FIG. 17B, and system controller 1810 in FIG. 18D) depend on the available sensor inputs, the environment, and user reactions. The inputs, reactions, and decision thresholds may be determined from one or more of ophthalmic research, pre-programming, training, and adaptive/learning algorithms. For example, the general characteristics of eyelid movement may be well-documented in literature, applicable to a broad population of users, and pre-programmed into system controller. However, an individual's deviations from the general expected response and/or changes in blink frequency may be recorded in a training session or part of an adaptive/learning algorithm which continues to refine the response in operation of the electronic ophthalmic device. In one embodiment, the user may train the device by activating a handheld fob, which communicates with the device, when the user desires near focus. A learning algorithm in the device may then reference sensor inputs in memory before and after the fob signal to refine internal decision algorithms. This training period could last for one day, after which the device would operate autonomously with only sensor inputs and not require the fob.

In an alternative exemplary embodiment, the system further includes an eye movement sensor system. In a further exemplary embodiment, if the system controller receives readings from the eye movement sensor system that the wearer is prone and from the eyelid position sensor system that the eyelids are closed, then the type of alarm may be adjusted to reflect the wearer is asleep. In a further exemplary embodiment, the alarm is started at a lower level of intensity that grows over a period of time to provide a gentler alert to the wearer. In an alternative exemplary embodiment, the alarm provided is an escalated alarm.

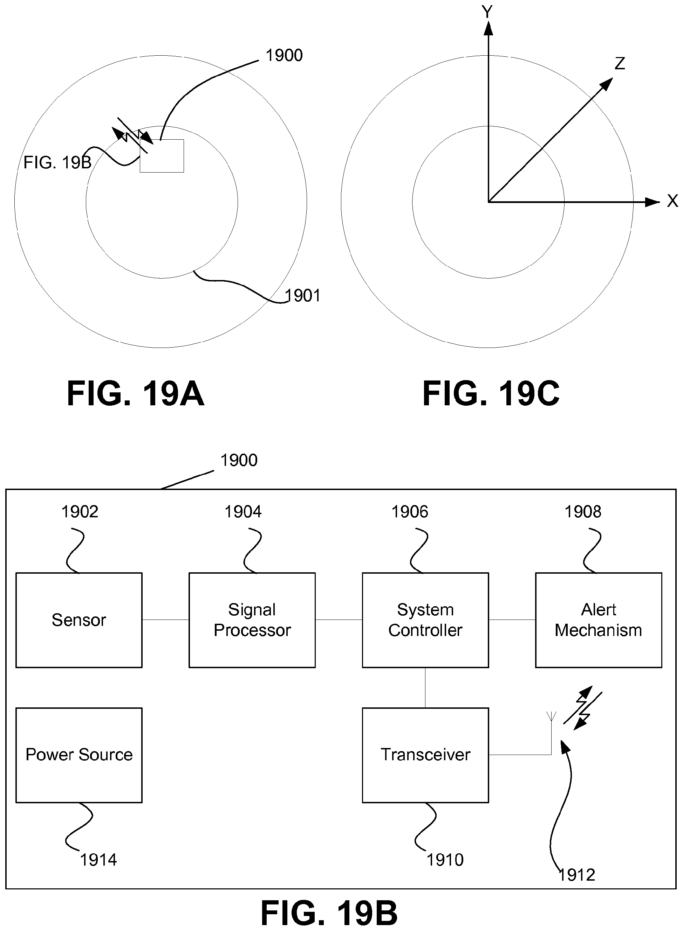

FIGs. 19A and 19B illustrate an exemplary eye movement sensor system 1900 for detecting movement of the eye. Sensor 1902 detects the movement and/or position of the pupil or, more generally, the eye. The sensor 1902 may be implemented as a multi-axis accelerometer on a contact lens 1901. With the contact lens 1901 being affixed to the eye and generally moving with the eye, an accelerometer on the contact lens 1901 may track eye movement. It is important to note that any suitable device may be utilized as the sensor 1902, and more than a single sensor 1902 may be utilized. The output of the sensor 1902 is acquired, sampled, and conditioned by signal processor 1904. The signal processor 1904 may include any number of devices including an amplifier, a transimpedance amplifier, an analog-to-digital converter, a filter, a digital signal processor, and related circuitry to receive data from the sensor 1902 and generate output in a suitable format for the remainder of the components of the eye movement sensor system 1900. The signal processor 1904 may be implemented utilizing analog circuitry, digital circuitry, software, and/or a combination thereof. In at least one exemplary embodiment, the signal processor 1904 and the sensor 1902 are fabricated on the same integrated circuit die. The sensor circuitry for acquisition and conditioning of an accelerometer is different than the circuitry for a muscle activity sensor or optical pupil tracker. The output of the signal processor 1904 in at least one exemplary embodiment is a sampled digital stream and may include absolute or relative position, movement, detected gaze in agreement with convergence, or other data. System controller 1906 receives input from the signal processor 1904 and uses this information, in conjunction with other inputs, to determine information regarding the position of the eye. System controller 1906 may both trigger the activity of sensor 1902 and the signal processor 1904 while receiving output from them.

System controller 1906 uses input data from the signal processor 1904 and/or transceiver 1910 to decide if the wearer is lying down (or prone) based on the orientation of the sensor 1902 based on orientation on an X, Y, and Z axes when no eye movement is detected. If the axes are as illustrated in FIG. 19C, then when the accelerometer detects stable acceleration in the X axis in either direction or in the Z axis in either direction, then the wearer's head has a horizontal orientation. When the accelerometer detects stable acceleration in the Y axis in the negative direction, then the wearer's head is vertical. When the accelerometer detects stable acceleration in the Y and Z axes with or without a stable acceleration in the X axis, then the wearer's head is tilted forward.

In a further exemplary embodiment, the system controller 1906 uses data from the sensor 1902 in conjunction with the data from a timing circuit to calculate acceleration-deceleration forces for the wearer. When the acceleration exceeds a concussion threshold, which is an example of a problem template, or, in an alternative embodiment, when the calculated force exceeds a concussion force, the system controller triggers an alert with the alert mechanism 1908 and/or begins a concussion test protocol. In a further embodiment, the system controller 1906 is provided an approximate weight of the wearer prior to calculation of acceleration-deceleration force. In a still further embodiment, the system controller 1906 adds the calculated acceleration-deceleration force to a cumulative forces value. When the cumulative forces value exceeds a repetitive concussion threshold, which in at least one embodiment is a constant value while in another embodiment is a variable number adjusting over time. In at least one embodiment where a storage box is used, the cumulative forces value is uploaded to the next pair of contact lens to provide long-term tracking of forces.

FIG. 19B illustrate an optional transceiver 1910, briefly described above, that receives and/or transmits communication through antenna 1912. This communication may come from an adjacent contact lens, spectacle lenses, or other devices. The transceiver 1910 may be configured for two-way communication with the system controller 1906. Transceiver 1910 may contain filtering, amplification, detection, and processing circuitry as is common in transceivers. The specific details of the transceiver 1910 are tailored for an electronic or powered contact lens, for example, the communication may be at the appropriate frequency, amplitude, and format for reliable communication between eyes, low power consumption, and to meet regulatory requirements. Transceiver 1910 and antenna 1912 may work in the radio frequency (RF) band, for example 2.4 GHz, or may use light for communication. Information received from transceiver 1910 is input to the system controller 1906, for example, information from an adjacent lens which indicates orientation. The system controller 1906 may also transmit data from, for example, the alert mechanism 1908, to the transceiver 1910, which then transmits data over the communication link via antenna 1912.

The system controller 1906 may be implemented as a state machine, on a field-programmable gate array, in a microcontroller, or in any other suitable device. Power for the system 1900 and components described herein is supplied by a power source 1914.

The eye movement sensor system 1900 in at least one exemplary embodiment is incorporated and/or otherwise encapsulated and insulated from the saline contact lens 1901 environment.

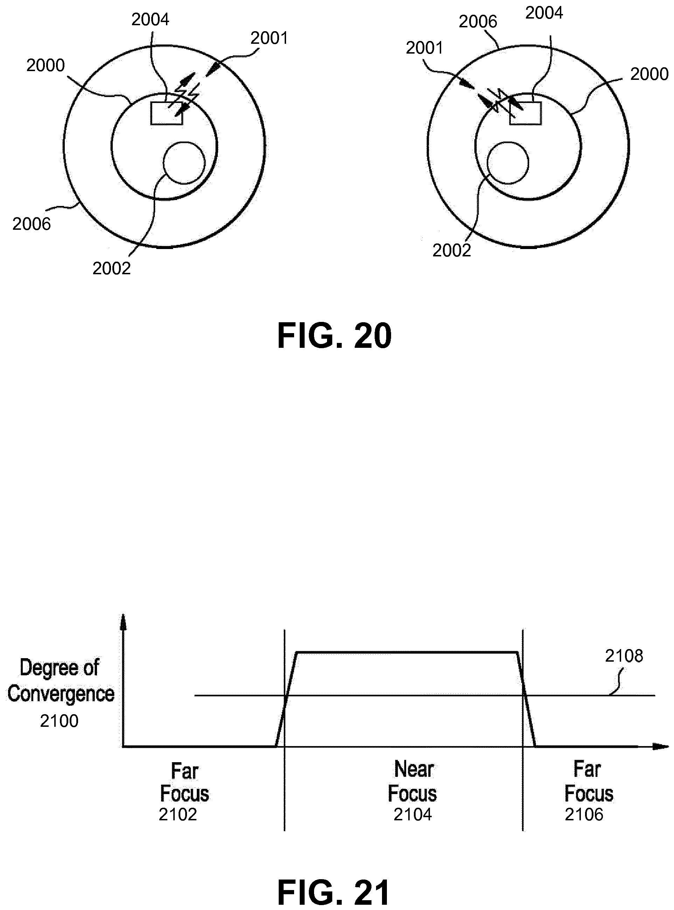

FIG. 20 illustrates a system by which the convergence or divergence of two pupils and/or gaze may be sensed and communicated between a pair of contact lenses 2000. Pupils 2002 are illustrated converged for near object viewing. Pupil position and convergence detection systems 2004 incorporated within contact lenses 2000 that are positioned on eyes 2006 track the position of the pupils 2002 and/or the contact lenses 2000, for example, with iris-facing photodetectors to observe the pupils 2002 or with accelerometers to track movement of the eyes 2006 and hence the pupils 2002. For the purposes of this disclosure, iris-facing means facing in the direction towards the wearer's eye, and in a further embodiment iris-facing includes having the component present on the side of the contact lens that contacts the wearer's eye. The pupil position and convergence detector systems 2004 may include several components forming a more complex system, for example, a 3-axis accelerometer, signal conditioning circuitry, a controller, memory, power supply, and a transceiver as is described in detail subsequently. Communication channel 2001 between the two contact lenses 2000 allows the pupil position and convergence detection systems 2004 to synchronize on pupil position. Communication may also take place with an external device, for example, spectacle glasses or a smartphone. Communication between the contact lenses 2000 is important to detect convergence. For example, without knowing the position of both pupils 2002, simply gazing down to the left may be detected as convergence by the right eye since the pupil 2002 has similar movement for both actions. However, if the right pupil is detected moving down to the left while the pupil of the left eye is detected moving down to the right, convergence may be construed. Communication between the two contact lenses 2000 may take the form of absolute or relative position, or may simply be a "convergence suspected" signal if the eye moves in the expected direction of convergence. In this case, if a given contact lens detects convergence itself and receives a convergence indication from the adjacent contact lens, it may activate a change in stage, for example, switching a variable-focus or variable power optic equipped contact lens to the near distance state to support reading. Other information useful for determining the desire to accommodate (focus near), for example, lid position and ciliary muscle activity, may also be transmitted over the communication channel 2001 if the contact lenses are so equipped. It should also be appreciated that communication over the channel 2001 could include other signals sensed, detected, or determined by each lens 2006 and used for a variety of purposes, including vision correction, vision enhancement, entertainment, and novelty.

In at least one exemplary embodiment, the system illustrated in FIG. 19B has as sensor 1902 that is a sensor that detects the movement and/or position of the pupil or, more generally, the eye. The sensor 1902 may be implemented as a multi-axis accelerometer in a contact lens 1901. With the contact lens 1901 being affixed to the eye and generally moving with the eye, an accelerometer on the contact lens 1901 may track eye movement. The sensor 1902 may also be implemented as an iris-facing camera or sensor which detects changes in images, patterns, or contrast to track eye movement. Alternately, the sensor 1902 may include neuromuscular sensors to detect nerve and/or muscle activity which moves the eye in the socket. There are six muscles attached to each eye globe which provide each eye with a full range of movement and each muscle has its own unique action or actions. These six muscles are innervated by one of the three cranial nerves (CN). The six extra-ocular muscles are the medial rectus which is innervated or controlled by CN3 (oculomotor), the inferior rectus muscle also innervated by CN3, the lateral rectus which is innervated or controlled by CN6 (abducens), the superior rectus which is innervated or controlled by CN3, the superior oblique which is innervated or controlled by CN4 (trochlear) and the inferior oblique which is innervated or controlled by CN3. It is important to note that any suitable device may be utilized as the sensor 1902, and more than a single sensor 1902 may be utilized. The output of the sensor 1902 is acquired, sampled, and conditioned by signal processor 1904. In at least one exemplary embodiment, system controller 1906 receives input from the signal processor 1904 and uses this information, in conjunction with other inputs, to control the electronic contact lens 1901. A transceiver 1910 receives and/or transmits communication through antenna 1902. This communication may come from an adjacent contact lens, spectacle lenses, or other devices. Information received from transceiver 1910 is input to the system controller 1906, for example, information from an adjacent lens which indicates convergence or divergence. System controller 1906 uses input data from the signal processor 1904 and/or transceiver 1910 to determine gaze direction. The system controller 1906 may also transmit data to the transceiver 1910, which then transmits data over the communication link via antenna 1912. In at least one exemplary embodiment, the pupil position and convergence detection system 1900 is incorporated and/or otherwise encapsulated and insulated from the saline contact lens 1901 environment.

FIG. 21 illustrates an example of correlation between convergence 2100 and focal length states 2102, 2104, and 2106 as is commonly documented in the ophthalmic literature. When in the far focus state 2102 and 2106, the degree of convergence is low. When in the near focus state 2104, the degree of convergence is high. A threshold 2108 may be set in the system controller (e.g., element 1906 of FIG. 19) to change the state of the electronic ophthalmic lens, for example, focusing a variable optic with add power when the threshold is passed going positive then focusing the variable optic with no add power when the threshold is passed going negative.

Eye tracking is the process of determining either or both where an individual is looking, point of gaze, or the motion of an eye relative to the head. An individual's gaze direction is determined by the orientation of the head and the orientation of the eyes. More specifically, the orientation of an individual's head determines the overall direction of the gaze while the orientation of the individual's eyes determines the exact gaze direction which in turn is limited by the orientation of the head. Information of where an individual is gazing provides the ability to determine the individual's focus of attention and this information.

It is important to note, that eye tracking in accordance with the present invention may be set up for gross or fine tracking monitoring.

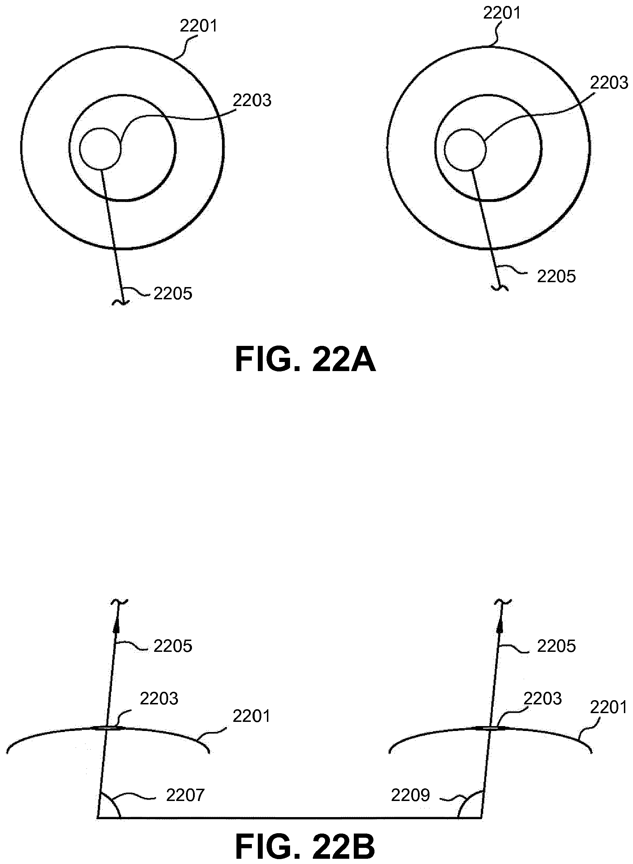

FIGs. 22A and 22B illustrate a pair of eyes 2201 observing an object (not illustrated) to the right of the user. FIG. 22A illustrates a front perspective of the eyes 2201, whereas FIG. 22B illustrates a top perspective of the eyes 2201. The position to the right is used for illustrative purposes, but it should be appreciated that the object under observation could be at any visible point in three-dimension space with the corresponding changes in eye gaze. As illustrated, via exaggeration, pupils 2203 both face toward the right. Lines 2205 drawn between the pupils 2203 and the object under observation are almost parallel since the object is illustrated to be much farther from the eyes 2201 than the distance between the eyes 2201. Angle 2207 is less than ninety (90) degrees whereas angle 2209 is greater than ninety (90) degrees. These angles are in contrast to angles were both ninety (90) degrees, when gazing straight ahead at a distant object, or both less than ninety degrees, when gazing straight ahead at a nearby object. As is illustrated in two dimensions, the angle may be used to determine gaze position or, more generally, samples of eye movement may be utilized to determine absolute and relative position and movement of eye gaze.

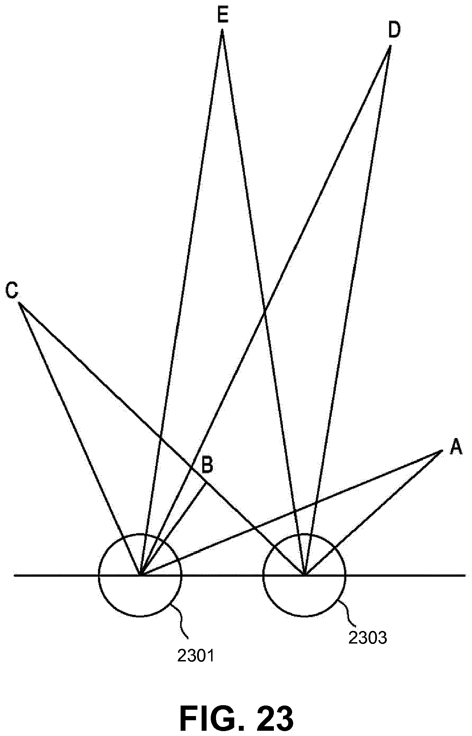

FIG. 23 illustrates the geometric systems associated with various gaze directions. FIG. 23 is a top view. Eyes 2301 and 2303 are shown gazing upon various targets labeled A, B, C, D, and E. A line connects each eye 2301 and 2303 to each target. A triangle is formed by each of the two lines connecting the eyes 2301 and 2303 with a given target in addition to a line connecting both eyes 2301 and 2303. As may be seen in the illustration, the angles between the direction of gaze in each eye 2301 and 2303 and the line between the two eyes 2301 and 2303 varies for each target. These angles may be measured by the sensor system, determined from indirect sensor measurements, or may only be shown for illustrative purposes. Although shown in two-dimensional space for simplicity of illustration, it should be apparent that gaze occurs in three-dimensional space with the corresponding addition of an additional axis. Targets A and B are shown relatively near to the eyes 2301 and 2303, for example, to be read with near-focus accommodation. Target A is to the right of both eyes 2301 and 2303, hence both eyes 2301 and 2303 are pointing right. Measuring the angle formed anti-clockwise between the horizontal axis, illustrated collinear with the line connecting the two eyes 2301 and 2303, and direction of gaze, both angles are acute for target A. Now referring to target B the eyes 2301 and 2303 are converged on a target in front of and between both eyes 2301 and 2303. Hence the angle, previously defined as anti-clockwise from the horizontal axis and the direction of gaze, is obtuse for the right eye 2303 and acute for the left eye 2301. A suitable sensor system will differentiate the positional difference between targets A and B with suitable accuracy for the application of concern. Target C is shown at intermediate distance for the special case of the right eye 2303 having the same direction of gaze and angle as target B. The gaze direction varies between targets B and C allowing a gaze direction determination system using inputs from both eyes 2301 and 2303 to determine the direction of gaze. Further, a case could be illustrated where another target F (not illustrated) lies above target B in three-dimensional space. In such an example, projected into the two-dimensional illustration shown in FIG. 23, the angles from the horizontal axis would be identical to those illustrated for target B. However, the angles normal to the page extending in three-dimensional space would not be equal between the targets. Finally, targets D and E are shown as distant objects. These examples illustrate that as the object under gaze is farther away, the angular difference at the eyes 2301 and 2303 between distant points becomes smaller. A suitable system for detecting gaze direction would have sufficient accuracy to differentiate between small, distant objects.

The direction of gaze may be determined by any number of suitable devices, for example, with iris-facing photodetectors to observe the pupils or with accelerometers to tack the movement of the eyes. Neuromuscular sensors may also be utilized. By monitoring the six extra-ocular muscles that control eye movement, the precise direction of gaze may be determined. A memory element to store prior position and/or acceleration may be required in addition to a position computation system considering present and past sensor inputs. In addition, the system illustrated in FIGs. 19A and 19B are equally applicable to the gaze and tracking system of the present invention. In at least one exemplary embodiment, the system controller is programmed to account for gazing geometries in three-dimensional space.

It is known in the art of optometry that the eyes do not remain completely stable when gazing at a stationary object. Rather, the eyes quickly move back and forth. A suitable system for detecting gaze position would include the necessary filtering and/or compensation to account for visual physiology. For example, such a system may include a low-pass filter or an algorithm specially tuned to a user's natural eye behaviors.

The activities of the acquisition sampling signal processing block and system controller (1904 and 1906 in FIG. 19B, respectively) depend on the available sensor inputs, the environment, and user reactions. The inputs, reactions, and decision thresholds may be determined from one or more of ophthalmic research, pre-programming, training, and adaptive/learning algorithms. For example, the general characteristics of eye movement may be well-documented in literature, applicable to a broad population of users, and pre-programmed into system controller. However, an individual's deviations from the general expected response may be recorded in a training session or part of an adaptive/learning algorithm which continues to refine the response in operation of the electronic ophthalmic device. In at least one exemplary embodiment, the user may train the device by activating a handheld fob, which communicates with the device, when the user desires near focus. A learning algorithm in the device may then reference sensor inputs in memory before and after the fob signal to refine internal decision algorithms. This training period could last for one day or any other suitable time period, after which the device would operate autonomously with only sensor inputs and not require the fob.

In at least one exemplary embodiment, the powered or electronic ophthalmic lens includes an iris-facing pupil diameter sensor. The size of the pupils and changes thereof; namely, dilation and constriction, may be utilized to control one or more aspects of the electronic or powered contact lens. In other words, signals output from the pupil sensor may be input to a system controller which in turn takes a specific action based upon the input and outputs a signal to an alert mechanism.

The iris is the partition between the anterior and posterior chambers of the eye. The iris is connected to the ciliary muscle as is the crystalline lens. The iris is formed from two muscles that regulate the central opening thereof, commonly referred to as the pupil. Similar to the shutter of a camera, the pupil, through the actions of the two muscles, controls the amount of light entering the eye. The size of the pupil varies with age, the color of the iris, and refractive error if any; however, a number of other factors may affect the size of the pupils at any given time. The iris constantly reacts to light and emotion, and thus any sensors have to account for these normal fluctuations as is explained in greater detail subsequently along with other reasons that the pupil may change in size. In addition, pupil size may be a good diagnostic tool for certain conditions, including cranial nerve damage.

The pupils may become dilated from the use of certain agents, for example, a cycloplegic drug such as atropine. The pupils may become dilated as a result of paralysis of the third cranial nerve. The pupil may be dilated and fixed to direct light stimulation and consensual light stimulation after acute narrow-angle glaucoma. Alternately, the pupils may become constricted from the use of glaucoma medications such as pilocarpine. Other drugs, for example, morphine, causes constriction of the pupils. In addition, certain conditions, for example, iritis, interruption of the sympathetic pathways of the eye and irritative lesions of the cornea may also cause constriction or the pupils. Hippus is a spasmodic, rhythmic, but irregular dilation and constriction of the pupils and may be indicative of a number of conditions.

External psychic influences, including surprise, fear and pain also cause the pupils to dilate. Dim light causes the pupils to dilate, whereas bright light causes the pupils to constrict. In addition, when an individual focuses on a near distance object, for example, reading a book, the pupils converge and constrict slightly in what is commonly referred to as the accommodative reflex. Accordingly, since certain factors are known to cause a specific pupillary reaction in otherwise healthy eyes, sensing the reaction of the pupils may be utilized as a control means. For example, if pupil constriction is detected alone or in combination with convergence, then the system controller may send a signal to an actuator to change the state of a variable power-optic incorporated into the powered contact lens.

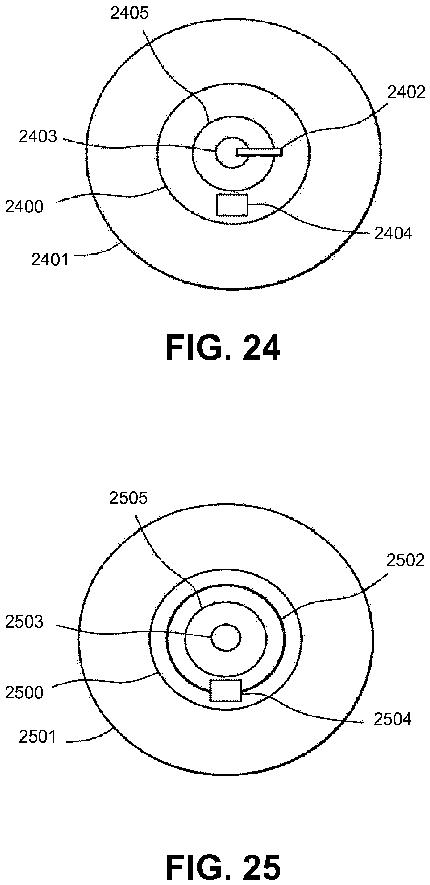

Referring now to FIG. 24, there is illustrated a powered contact lens with a pupil diameter sensor. The contact lens 2400 is positioned on the eye 2401 of an individual. The iris of the eye 2401 is shown in two levels of diameter, constricted 2403 and dilated 2405. The contact lens 2400 covers a portion of the eye 2401 including the iris. The contact lens 2400 includes a first exemplary pupil diameter sensor 2402 and electronic component 2404. The contact lens 2400 may include other sensors as discussed in this disclosure.

In at least one exemplary embodiment, the pupil diameter sensor 2402 is positioned in the contact lens 2400 above the iris. As illustrated, the pupil diameter sensor 2402 is a thin strip covering all possible pupil diameters which permits it to detect all levels of pupil diameter. If implemented as a strip, as in this exemplary embodiment, the strip is thin and transparent, so as not to disrupt light incident on the eye 2401. In at least one embodiment, the pupil diameter sensor 2402 includes an array of photodetectors facing back into or towards the iris. Depending on the pupil diameter, sensors at various distances from the center of the iris will detect different reflected light. For example, when the iris is dilated most of the sensors may detect little light because of the large, dark pupil. Conversely, when the iris is constricted most sensors may detect higher light because of reflection off the iris. It should be appreciated that, for such a sensor, ambient light level and iris color may need to be considered in the system design, for example, by a per-user programming and/or calibration. Such an ambient light sensor may be implemented as a forward-facing photosensor to complement the iris-facing sensors of pupil diameter sensor 2402. To minimize disruption of the optic zone in front of the eye, in at least one exemplary embodiment the pupil diameter sensor 2402 may be implemented using transparent conductors such as indium-tin oxide and small, thin silicon photosensors.

In an alternate exemplary embodiment, the pupil diameter sensor 2402 may be implemented as an array of sensors positioned around the iris to maximize coverage as opposed to just a linear strip. It should be appreciated that other physical configurations are possible to maximize performance, cost, comfort, acceptance, and other metrics.

The pupil diameter sensor 2402 may be integrated with other electronics, may function on its own, or may connect to another device such as a controller portion of the electronic component 2404. In this exemplary embodiment, the system controller samples the pupil diameter sensor 2402 and, depending on results from the pupil diameter sensor 2402, may activate another component in the system (not shown) and/or used to monitor a medical condition or health of the wearer. A power source (not shown) supplies current to the pupil diameter sensor 2402, the controller, and other components of the electronic ophthalmic system.

Such a system may require not only detectors such as those illustrated and described, but also emitters (not shown). Such emitters may, for example, include light-emitting diodes matched to the photosensors of pupil diameter sensor 2402. Alternately, the emitters may include piezoelectric ultrasonic transducers coupled to ultrasonic receivers in the pupil diameter sensor 2402. In yet another exemplary embodiment, the sensors and emitters may create an impedance detection system, for example, by passing a low-current signal through the eye and measuring changes in voltage across the eye.

FIG. 25 illustrates a contact lens 2500 with an alternative pupil diameter sensor. The contact lens 2500 is positioned on the eye 2501 of an individual. The iris of the eye 2501 is shown in two levels of diameter, constricted 2503 and dilated 2505. The contact lens 2500 covers a portion of the eye 2501, including the iris. Rather than the strip or array of detectors partially covering the pupil as described above and illustrated in FIG. 24, the system in FIG. 25 positions the pupil diameter sensor or sensors 2502 outside of the maximum pupil diameter 2505 but still inside the contact lens 2500. This configuration is beneficial because no potential obstruction of the optic zone occurs due to the pupil diameter sensor 2502. The pupil diameter sensor or sensors 2502 may, for example, include a single- or multi-turn coil antenna. Such an antenna may receive electromagnetic radiation from the eye as the muscles controlling the iris contract and relax. It is well-known in the relevant art that muscle and neural activity of the eye may be detected through changes in electromagnetic emissions, for example, with contact electrodes, capacitive sensors, and antennas. In this manner, a pupil diameter sensor based on a muscle sensor may be implemented. The pupil diameter sensor 2502 may also be implemented as one or more contact- or capacitive electrodes designed to measure impedance across the eye. Similar to other proposed systems which use changes in impedance to determine ciliary muscle activity in the eye, and hence a desire to change focal state, impedance may be used to detect changes in pupil diameter. For example, the impedance measured across the iris and pupil may change appreciably depending on pupil diameter. A pupil diameter sensor 2502 placed at the appropriate location on the eye and properly coupled to the eye could detect these changes in impedance and hence pupil diameter. The contact lens 2500 may also include an electronic component 2504 as described above.

In at least one exemplary embodiment, the system illustrated in FIG. 19B has a sensor 1902 that is a pupil diameter sensor(s), as illustrated in FIGS. 24 and 25. Pupil diameter sensor 1902 includes one or more of the pupil diameter sensors as previously described, for example, photosensors, antennas, or impedance sensors. In at least one exemplary embodiment, any emitters necessary to implement or improve the performance of the sensors are included in element 1902 for simplicity. Element 1902 may include multiple sensors, or multiple sensor blocks such as 1902, perhaps implemented in different technologies and sensor methods. Signal processor 1904 is an interface between the sensor 1902 and the system controller 1906. The output of the signal conditioning element 1904 is a signal included of sensor data which is input to the system controller 1906. The system controller 1906, as discussed previously, may consider inputs from multiple sensors 1902 (both in terms of number and type) for providing an output to the alert mechanism 1908. A transceiver 1910 may be included in the system to send data to and/or receive data from external devices, for example a second contact lens mounted on the adjacent eye, spectacle lenses, a smartphone, or another device. Such communication occurs through an antenna 1912, perhaps an electromagnetic antenna or a light-emitting diode/photodiode sensor combination.

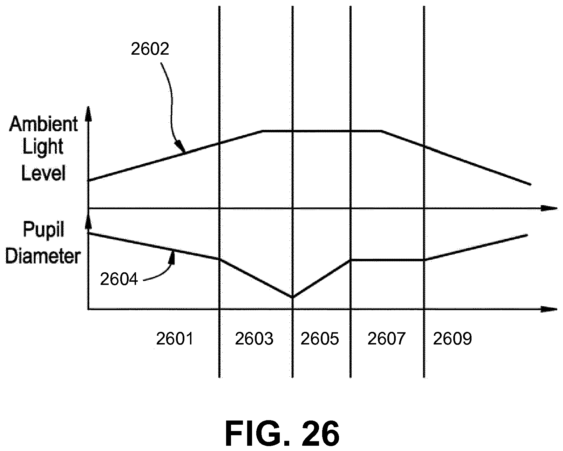

FIG. 26 illustrates ambient light 2602 and pupil diameter 2604 plotted versus time on the x-axis, illustrating how differences between these two measured quantities could be used to activate an electronic ophthalmic device such as a contact lens. During the first time period 2601, ambient light level 2602 is increasing while pupil diameter 2604 is decreasing. Ambient light and pupil diameter may be sensed as previously described, for example, by a forward-facing photodiode and an iris-facing impedance sensor, respectively. As is commonly the case, as ambient light increases in time period 2601 pupil diameter decreases. This is a common reaction which occurs to maintain a relatively constant light intensity on the retina by reducing the aperture of the iris. In time period 2603, the ambient light level 2602 first continues to increase then levels off. However, the pupil diameter 2604 constricts more rapidly than in the previous time period. This is not the classical correlation between ambient light and pupil diameter. This response may be caused by a narrow-angle response of the pupil, perhaps to a book held up close, versus the wide-angle response of an ambient light detector. In this manner, a change in pupil diameter response may be detected and used to activate a function in an electronic ophthalmic device. In time period 2605, the ambient light 2602 continues flat however the pupil diameter 2604 dilates or increases. Again, this may be caused by a specific response in the eye, for example, the accommodation reflex. In time period 2607 there is again a difference between ambient light level 2602, which starts level then decreases, and pupil diameter 2604 which stays flat. Again, this may be used to detect certain responses in the eye and trigger changes in the operation of an electronic ophthalmic device. Finally, in time period 2609 the classical response is again observed similar to that shown in timer period 2601. As the ambient light level 2602 decreases, the pupil diameter 2604 dilates to let in more light.

In at least one exemplary embodiment, the activities of the signal conditioning block and system controller (1904 and 1906 in FIG. 19B, respectively) depend on the available sensor inputs, the environment, and user reactions, for example the ambient light level and pupil diameter as illustrated in FIG. 26. The inputs, reactions, and decision thresholds may be determined from one or more of ophthalmic research, pre-programming, training, and adaptive/learning algorithms. For example, the general characteristics of pupil dilation versus ambient light may be well-documented in literature, applicable to a broad population of users, and pre-programmed into system controller 1906. However, an individual's deviations from the general expected response, for example the deviations illustrated in time periods 2603, 2605, and 2607 of FIG. 26, may be recorded in a training session or part of an adaptive/learning algorithm which continues to refine the response in operation of the electronic ophthalmic device. In one example embodiment, the user may train the device by activating a handheld fob, which communicates with the device, when the user desires near focus. A learning algorithm in the device may then reference sensor inputs in memory before and after the fob signal to refine internal decision algorithms. This training period could last for one day or any other suitable time period, after which the device would operate autonomously with only sensor inputs and not require the fob.

In a further exemplary embodiment, the pupil dilation sensor is used in combination with photodetector sensors for blink detection to provide a light-triggered pupil dilation test to the wearer. In at least one exemplary embodiment, the system controller monitors a photodetector sensor for a rapid light level change that would be of sufficient size to provoke a dilated pupil. In such an embodiment, the system controller also would be monitoring the pupil dilation sensor(s) for pupil size so that there is a comparison possible to detect pupil size change when a rapid light level change is detected. The system controller would have a template that would include a light change threshold and a pupil dilation threshold for comparison to data stored in, for example, registers or buffer memory(ies) in the system controller. When the light change threshold is met and the pupil dilation threshold is not met, then the system controller will determine that a medical condition has occurred for the wearer. The change would be running a comparison with a recent sensor reading to the current sensor reading. In at least one exemplary embodiment, the recent sensor reading is a reading within a predetermined time period to compensate for an implementation where there is a high sampling rate of the sensors. In an alternative exemplary embodiment, the system controller may also store relevant data when both thresholds are exceeded particularly in a medical logging implementation.

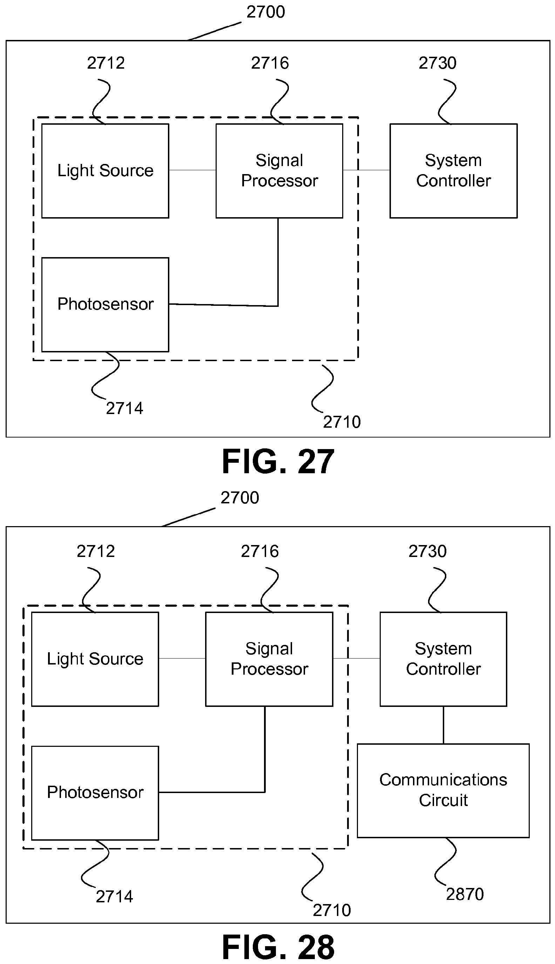

In at least one exemplary embodiment, the powered or electronic ophthalmic lens includes a pulse oximeter sensor, which is iris-facing. The pulse oximeter sensor includes at least one light source such as a LED and at least one photosensor for receiving back-reflected light from the eye that originates with the light source. FIGs. 27 and 28 illustrate an exemplary lens 2700 having the pulse oximeter sensor 2710 and a system controller 2730. The illustrated pulse oximeter sensor 2710 includes the at least one light source 2712 and at least one photosensor 2714. Both the light source 2712 and the photosensor 2714 are in electrical communication with a signal processor 2716 that processes the output of the photosensor 2714 and drives the light source 2712 with, for example, an oximeter signal. The signal processor 2716 provides an output to the system controller 2730. In at least one exemplary embodiment, the signal processor 2716 is incorporated into the system controller 2730. As discussed in the various other exemplary embodiments in this disclosure, there may be a variety of other components present on the contact lens 2700 beyond these components.

In at least one exemplary embodiment, the light source includes an infrared light source and/or a near-infrared light source. In at least one exemplary embodiment, when the light source is one light emitter, then it is configured to output light having two wavelengths. The first wavelength is at about 660 nm, while the second wavelength is about 940 nm. In an alternative exemplary embodiment, where the light source includes two light emitters, then the first light emitter will produce a light having a wavelength of about 660 nm and the second light emitter will produce a light having a wavelength in the range of about 890 nm to about 950 nm. In a further exemplary embodiment, the light source or sources have bandwidth in the range of 20 nm to 50 nm.

In at least one exemplary embodiment, the photosensor is selected from any of the photodetectors discussed previously in this disclosure. The photosensor may be matched to the light source, for example having a peak response wavelength close to the peak output wavelength of the source and a similar bandwidth.

In at least one exemplary embodiment as illustrated in FIG. 28, the system further includes a communications circuit 2870 that allows for transmission of the output from the photodetector to be sent to an external device for processing.

In at least one exemplary embodiment, the light source and the photodetector are arranged to perform reflectance pulse oximetry based on their close proximity to each other. In at least one alternative exemplary embodiment, the light source and the photodetector are arranged to be located on opposing edges of the contact lens to provide transmission pulse oximetry by passing the light through the cornea and the iris of the wearer. Their location on opposing edges is that the sensor and the light source are proximate to the edge to allow for sufficient lens material to be present between them and the edge for manufacturing and/or safety considerations.

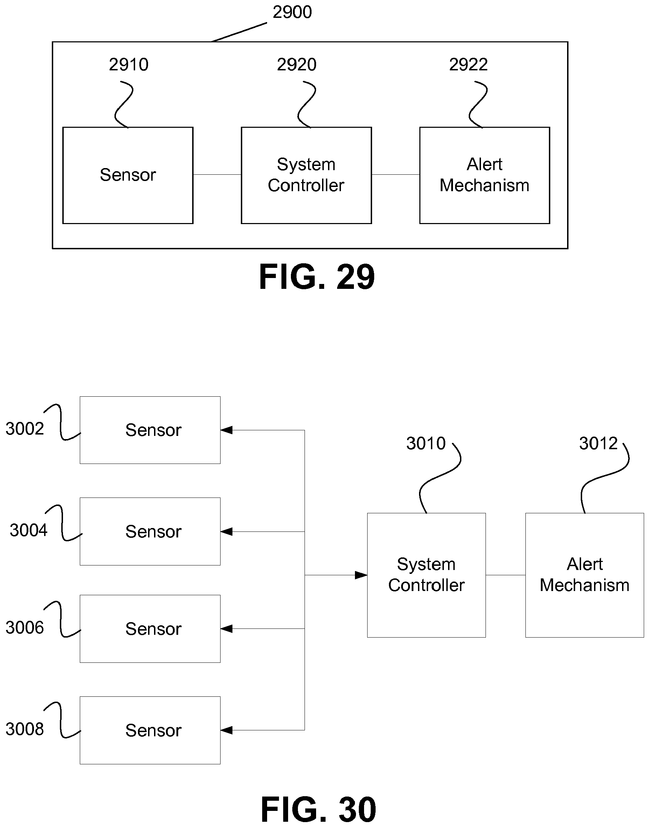

In at least one exemplary embodiment as illustrated in FIG. 29, the contact lens 2900 includes a sensor 2910 to detect at least one of removal from a lens storage case and insertion of the contact lens into the wearer's eye. In at least one exemplary embodiment, insertion of the contact lens into the wearer's eye will activate medical monitoring by the system controller 2920. In a further exemplary embodiment, the insertion will initiate an accumulator in the alert mechanism 2922 to run. In an alternative exemplary embodiment, the removal of the contact lens from the wearer's eye will terminate a medical monitoring by the system controller 2920. Examples of sensors that would provide detection include, but are not limited to, a pressure sensor, a reed switch, a salinity sensor, a biosensor and a capacitive sensor. These sensors, in at least one exemplary embodiment, work in conjunction with a light sensor to detect the presence of light that occurs after removal of the contact lens from the storage container. In a further exemplary embodiment to the sensor embodiments, the sampling rate used to monitor the sensor may be slowed after the detection of the event being monitored to conserve power while allowing for the detection of removal of the contact lens from the eye. In an alternative exemplary embodiment to the prior embodiment, the sensor would be deactivated upon detection of the contact lens being placed on the eye.

The pressure sensor may take a variety of forms. One example is an iris-facing (or rear-facing) pressure sensor connected to the system controller through an analog-to-digital convertor. The iris-facing pressure sensor in at least one exemplary embodiment is partially encapsulated in the contact lens while the analog-to-digital convertor is completely encapsulated in the contact lens and included as part of any circuit board present in the contact lens. The system controller resets the accumulator upon receiving a signal from the pressure sensor in excess of an insertion threshold indicating that data collection should begin by the system controller. The system controller sends a signal to the alert mechanism to store the current accumulator value when the signal from the pressure sensor then falls below the insertion threshold indicating that the contact lens has been removed and further data collection is unnecessary. The system controller samples the pressure sensor at a predetermined schedule only when the system controller detects the eyelid is open. Another example of a pressure sensor is a pressure sensor that will detect the removal of pressure from the saline present in the storage container and would provide a signal to activate the other functionality of the contact lens. A further example of a pressure sensor is a surface acoustic wave resonator with interdigital transducer (IDT). A still further example is a binary contact pressure sensor that either detects pressure or no pressure, but not the level of pressure.

One example is the utilization of a reed switch which completes a circuit in the contact lens that provides power to the rest of the circuit elements by application of pressure from the wearer's eye upon insertion of the contact lens or the removal of pressure when the contact lens is removed from the storage container for use. Upon the respective event occurring, the reed switch would close and complete the circuit to provide an electrical connection between the system controller and the power supply. Another example of a reed switch use in the system is to provide a binary output upon the switch being activated with the binary output providing an indication of the switch being closed (or open depending on the orientation of the switch) as opposed to completing a circuit.

A salinity sensor or biosensor in at least one exemplary embodiment would detect salinity or another chemical present in tear fluid. Examples of the substances that could be monitored include, but are not limited to, a pathogen, a biomarker, an active agent, and a chemical. One example of a biosensor is a resistance tab, in electrical communication with system the controller, which is capable of binding with the substance being monitored resulting in an increasing or decreasing resistance as the amount of substance present increases and/or decreases. Another example is a reactive tube(s) that contains a substance, material, or mixture that may react with a specific molecule where a reaction will be indicative of the presence of a chemical being monitored. Yet another example is a biosensor in which a surface is functionalized to have an affinity for a certain substance, and an electrical property of the sensor, for example, capacitance or voltage, varies in response to the presence of the substance to which the sensor is functionalized. In at least one exemplary embodiment, where a chemical being monitored relates to a concentration of some substance in the tear fluid, the reaction may occur directly with that substance or may occur with a separate substance that may indicate concentration of the monitored substance. In other examples, because other electroactive biological components may affect the conductivity within a particular tube, the tube may be lined with or include a selective barrier to minimize interference with the other substances than the substance being monitored. Alternatively to a tube having an increasing conductivity in response to the presence of the monitored substance, the tube may instead have an increasing resistivity in the presence of the monitored substance. A further example will have the hollow tube include material that is selectively permeable or attractive to a specific substance or chemical. Under any of these examples, it may be possible to provide a graduated indication of the level of the substance beyond a binary output. In at least one exemplary embodiment, the salinity sensor and/or the biosensor is one of the sensors 110', 120' in FIGs. 1A and 1D-1F used by the system controller 130 to monitor at least one medical condition or state of the wearer.

The capacitive sensor may be rear facing or forward facing. In at least one exemplary embodiment, the sensor would be an iris-facing sensor to allow for contact by the wearer's eye. In a further exemplary embodiment, once a contact causes a change in capacitance above an insertion threshold indicating that the contact lens has been inserted, the sensor is deactivated or has its sampling rate decreased. If, however, the sensor was forward facing, then contact by one of the eyelids that would change the capacitance above the insertion threshold would confirm insertion of the contact lens. In a further exemplary embodiment, the forward-facing capacitive sensor would also be used for detection of the position of the eyelids.

In complex systems which may include multiple sensors, such as powered ophthalmic lenses having a number of electronic components, it is possible in at least one embodiment to reduce the potential for initiating false actions or false positive triggering of a sleep determination. In accordance with another alternative exemplary embodiment, this exemplary embodiment is directed to a decision making process and/or voting scheme which utilizes input from multiple sensors to substantially reduce the possibility of changing the state of the powered ophthalmic lens based upon inaccurate, incomplete or erroneous information, changing physiologic conditions, as well as noise and/or interference from internal and external sources. For example, in medical monitoring, the control system should not determine onset of a medical condition such as seizure based upon a random blinking pattern due to eye irritation or the like. Likewise in medical monitoring, a determination of concussion or mental impairment should not be confused with slowed eye movements (drowsiness) or fixed eye movements (daydreaming). However, with input from a single sensor or erroneous information from the single sensor or other sensors, incorrect decisions may be made by the system controller. For example, without knowing the pressure applied to the ophthalmic lens, simply closing the eye lids might trigger a sleep determination despite the wearer rubbing their eyes and applying a pressure greater than lid pressure on a pressure sensor(s). In a powered ophthalmic lens having an eyelid position sensor, eyelid movement may also be utilized as a trigger for making a sleep determination. For example, when an individual gazes down to focus on a near distance object, the eyelids tend to droop and thus it may be utilized to change the state of the ophthalmic lens. Once again, if only a single input is utilized, a false action may take place due to the fact that the person is sleepy and their eyelids drooped. All of these sensors may be utilized as triggers for action to be implemented by various systems incorporated into an electronic or powered ophthalmic lens, and all of them independently or in limited combination are potentially subject to error. In addition to the sensors already mentioned which are intended to detect certain aspects directly related to determining sleep onset, other sensors may be used to improve state-change sensors by monitoring ambient conditions, noise, and interference. For example, ambient light may be monitored to improve the accuracy of blink detection, lid position, and pupil diameter sensors. Such sensors may be utilized to augment other sensors, for example, by subtracting common mode noise and interference. Sensor inputs may be used to record history readings (an example of historical data) which are then considered by a complex decision algorithm, for example, one which considers both accelerometer inputs and eye muscle contraction to determine pupil position. Utilizing the voting scheme in accordance with at least one exemplary embodiment may reduce the likelihood of error in determining whether the wearer has fallen asleep and may also allow more precise measurements. In other words, for any given determination to be made, there are sensors that may be utilized to check corroborating evidence or to augment input for a given determination by a primary sensor. It is also important to note that the sensed data, in addition to or in alternate use, may simply be utilized as part of a collection process rather than as a triggering event. For example, the sensed data may be collected, logged and utilized in treating medical conditions. In other words, it should also be appreciated that a device utilizing such a sensor may not change state in a manner visible to the user; rather the device may simply log data.

Referring now to FIG. 30, there is illustrated a generic system in which sensors 3002, 3004, 3006 and 3008 are used to determine if a medical condition has arisen. The sensors 3002, 3004, 3006 and 3008 may include any number of potential inputs including blink action, lid position, pupil position, contact lens orientation, external lens pressure, biosensors, bioimpedance, temperature, pulse oximeter, and the like. The number and type of sensors is determined by the application and the wearer. Each sensor 3002, 3004, 3006 and 3008 may have its own signal conditioning contained within the sensor block, a dedicated block, or within the system controller 3010. The system controller 3010 accepts inputs from each sensor 3002, 3004, 3006 and 3008. It then performs routines to process and compare the input data. Based on these inputs, the system controller 3010 determines if the alert mechanism 3012 should record any readings. For example, the combination of eyelid droop, low ambient light, and vertical lens orientation may trigger the system controller 3010 to determine the wearer is drowsy and to signal the alert mechanism 3012 to increase the sampling rate of at least one sensor system being used to make the determination. Likewise, the combination of eyelid closure, vertical orientation for the wearer, and external eyelid pressure may trigger the system controller 3010 to determine no sleep onset and continue regular operation. The combination of lid closure, horizontal orientation for the wearer may trigger the system controller 3010 to determine sleep onset and to signal the alert mechanism to record data as the sleep is likely intentional sleep given the wearer's orientation. Inputs from various sensors may also be utilized to alter the configuration of the system controller to improve decision making performance, for example, if ambient light decreases, the controller may increase the gain of a photosensor. The system controller may also turn sensors on and/or off, increase and/or decrease sampling rates, and make other changes to the system to optimize performance. Utilizing inputs from multiple sensors in conjunction with an algorithm that weights and/or votes on various scenarios tends to minimize false positive, thereby tending to make the overall system more reliable.

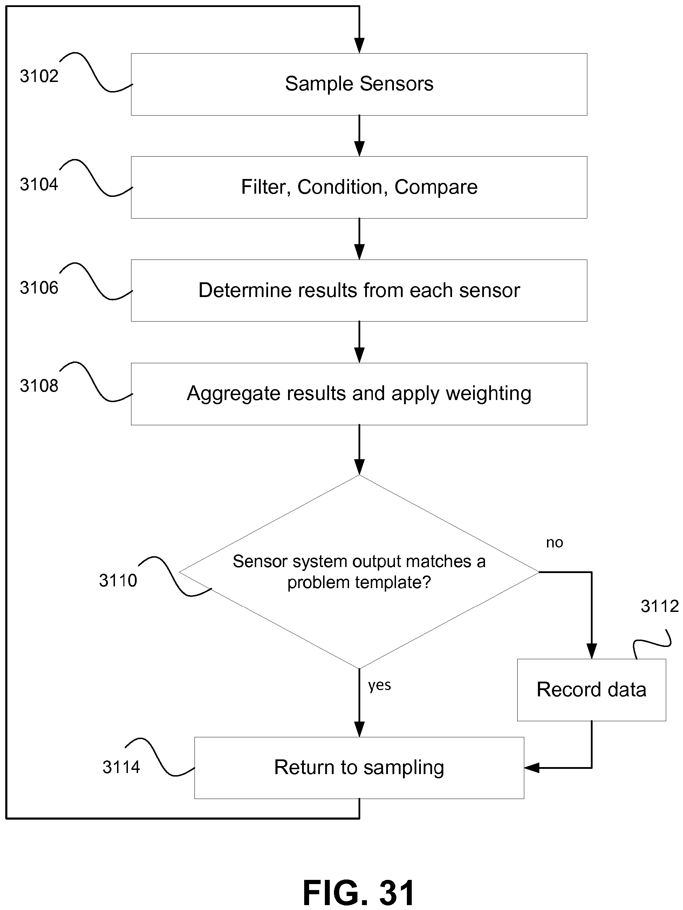

FIG. 31 illustrates a flow chart of a method by which a system controller, for example, system controller 3010 illustrated in FIG. 30, operates to sample sensors and determine whether a medical condition has arisen. The first step is to sample the sensors, 3102. This may require triggering other elements to activate, warm-up, calibrate, take readings, condition, and output data. The system controller may also provide configuration information to each sensor based on programmed values and current data, for example, the gain of a photosensor amplifier based on the history of incident light, or these settings may be determined by other elements in the system. Then the method performs filtering and additional conditioning, 3104, for example, digital as opposed to analog filtering, along with a comparison to baseline or reference results. One purpose of this step is to properly condition the input data for the next step so that an accurate, repeatable decision may be made. Then the results are determined from each sensor, 3106, for example, the lid position and emitter-detector response. This determination may involve comparison to a pre-programmed or variable threshold, comparison to a specific pattern, or any other determination. The results are aggregated from the previous step and weighted, 3108. A decision is made whether a medical condition has arisen using, for example, a problem template, 3110. This step in at least one exemplary embodiment may involve per-user training and preferences or historical data, ensuring all sensors have been sampled before deciding, and various weights applied to the results of each sensor. In at least one embodiment, a decision is made that is predictable and repeatable in the presence of real-world noise and interference. If a decision is made regarding a medical condition as described above, then recording data, 3112, may begin. Regardless of the decision, returning the system to sampling so another set of measurements and determination may take place, 3114. The total time required to execute the process in FIG. 31 in at least one exemplary embodiment is short enough such that the system is responsive to user inputs similar to how individuals naturally interact with their environments.

It should be appreciated that each sensor input may vary for reasons other than monitoring. For example, the eye impedance may vary over time due to changes in body hydration, salt intake, level of exertion, or other means. Likewise, pupil diameter may vary due to changes in ambient light levels. Thus, it should be apparent that combining multiple sensor inputs reduces the chances of false positive triggering by requiring more than one input to correlate with a desired change in focal length or by using certain sensor inputs to augment other sensors.

It should also be apparent that the problem templates such as thresholds and/or problem patterns for each sensor and the combination of sensors used to monitor the wearer depends on many variables such as safety, response time, and user preferences. The specific programming of the voting scheme may be based on clinical observations of a number of subjects and individual programming tailored to a specific user based in at least one exemplary embodiment on, for example, recent sensor readings and/or historical data, which may be downloaded onto the lens(es). Parameters in the voting scheme may be dependent on sensor inputs, for example, the threshold and gain setting for blink detection may vary with ambient light.

In an alternative exemplary embodiment, the system further includes a memory preservation controller that is in electrical communication with the power source and the system controller. In at least one exemplary embodiment, the memory preservation controller is an example of the resource management system 160 discussed in connection with FIG. 1B. The memory preservation controller, at a predetermined frequency, tests the power source to determine the level of energy that remains. When the remaining energy falls below a predetermined energy threshold, the memory preservation controller sends an instruction to the system controller to no longer sample the sensor and to send a signal causing the recording by the alert mechanism of the current time and/or accumulator value. The power then is provided to maintain the data in memory and/or data storage present on the contact lens. In a further exemplary embodiment when the power supply finds the available energy level below a low-energy threshold, the system will perform at least one of the following: reducing the sampling rate of at least one sensor, terminating further sampling of at least one sensor, terminating further monitoring of the power supply, storing a time stamp representing low-energy based on the current value in the accumulator or timing circuit, removing power from at least one sensor, sampling at least one sensor at a second sampling rate that is slower than the first sampling rate, powering a memory where the readings are stored, or any combination of these. Based on this disclosure one of ordinary skill in the art should appreciate that a particular implementation may have just one of these options available and that this is contemplated to be covered by the at least one of language.

The predetermined energy threshold is based on an estimate of the power required to maintain a power supply to any memory or data storage device. In a further exemplary embodiment, the threshold is adjusted based on the current run time of the lens while still facilitating an estimated period of power for the memory and/or data storage. One example of how to adjust the threshold over time is to decrement a register for each passing of a predetermined time as measured by sampling periods in the contact lens.

In a further exemplary embodiment, the energy level test is done in conjunction with the sampling of the sensor(s) to compare the energy level of the power source to the threshold under maximum load of the lens as occurs when a sensor(s) is providing a reading(s). If the energy level for the power source is below a threshold, then there is a high likelihood that an upcoming sensor sampling, prior to the next energy level test, will drain the power source such that the sensor(s) will provide an incorrect reading because of insufficient power being available and/or stored data will become corrupted thus leading to a data set that is unreliable.

In a modified alternative exemplary embodiment, the memory preservation controller places an artificial load on the power source during periods of non-sampling of the sensor(s). Example sampling time periods include but are not limited to 1 minute, 2 minutes, 5 minutes, 10 minutes, 15 minutes, 20 minutes, and 30 minutes. Other examples of testing the power source include, but are not limited to, obtaining a loaded voltage, introducing a special test waveform to pulse current out the battery and measuring voltage drop with the comparison of the results being compared to a predetermined threshold that in a further exemplary embodiment may be adjusted downward in view of expected remaining run time.

In a further alternative exemplary embodiment, the memory preservation controller monitors the data manager to determine remaining space. When the remaining space in memory of the data manager is less than a free space threshold, the memory preservation controller sends a signal to the system controller to do at least one of the following: terminate sampling the sensor(s) to avoid creating additional data for storage, send a signal to the data storage to set a flag of memory full and to shift the currently stored data to provide additional space using a first in first out approach, and remove power from the system controller and the sensor(s) leaving power being supplied to just the data storage. Other examples include storing a time stamp representing low memory based on the current value in the accumulator, reducing the sampling rate for at least one sensor, terminating further sampling of at least one sensor, storing future readings from at least one sensor over the earliest stored readings in the memory, deleting the stored sensor readings associated with the lowest accumulator reading and shifting the remaining stored sensor and accumulator readings in the memory, and any combination of these examples.

In a further exemplary embodiment to the above exemplary embodiments, the memory preservation controller and/or the resource management system is part of the system controller.

In at least one exemplary embodiment, the electronics and electronic interconnections are made in the peripheral zone of a contact lens rather than in the optic zone. In accordance with an alternative exemplary embodiment, it is important to note that the positioning of the electronics need not be limited to the peripheral zone of the contact lens. All of the electronic components described herein may be fabricated utilizing thin-film technology and/or transparent materials. If these technologies are utilized, the electronic components may be placed in any suitable location as long as they are compatible with the optics.

An intraocular lens or IOL is a lens that is implanted in the eye and replaces the crystalline lens. It may be utilized for individuals with cataracts or simply to treat various refractive errors. An IOL typically comprises a small plastic lens with plastic side struts called haptics to hold the lens in position within the capsular bag in the eye. Any of the electronics and/or components described herein may be incorporated into IOLs in a manner similar to that of contact lenses.

In at least one exemplary embodiment, the system further includes a storage box. The storage box in at least one embodiment includes a housing with a base and a cover that are connected along one edge to facilitate opening the cover relative to the base to allow for deposit of the contact lens into a cavity in the housing. In alternative exemplary embodiments, the storage box may include disinfecting, monitoring, reordering and external connectivity functionality. The disinfecting functionality would allow for the lenses to be used over an extended period of time by the wearer.

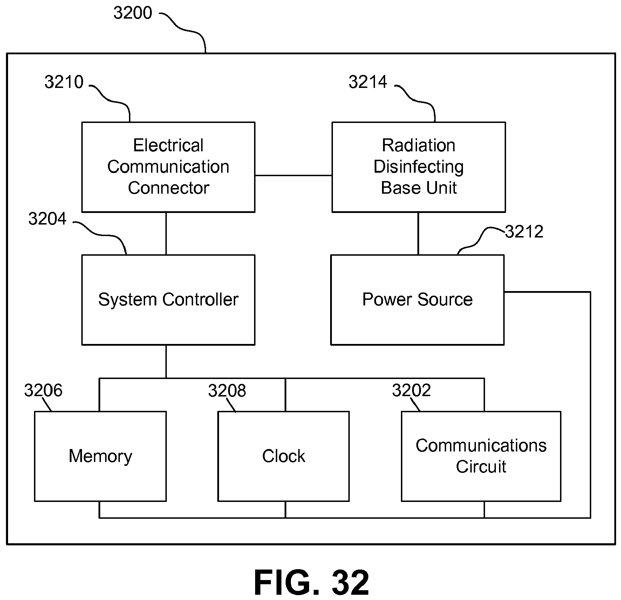

FIG. 32 illustrates an example storage box having a housing 3200, a communication system 3202, a system controller 3204, a memory 3206, a clock (or timing circuit) 3208, an electrical communication connector 3210, and a power source 3212. In an alternative exemplary embodiment, the storage box includes a radiation disinfecting base unit 3214 contained within a housing 3200 which in at least one exemplary embodiment includes a base and a cover. The electrical communication connector 3210 may include a universal serial bus (USB) connector or other type of connector. The connector may include a terminal for transferring one or both of data and electrical power. In some exemplary embodiments, the electrical communication connector 3210 provides power to operate the radiation disinfecting base unit 3214. Some embodiments may also include one or more batteries 3212 or other power storage device. In some exemplary embodiments, the batteries 3212 include one or more lithium-ion batteries or other rechargeable device. The power storage devices may receive a charging electrical current via the electrical communication connector 3210. In at least one battery embodiment, the radiation disinfecting base unit 3214 is operational via stored power in the batteries 3212.

In at least one exemplary embodiment, the communication system 3202 includes an antenna such as a radio-frequency identification (RFID) antenna for interacting with inserted lenses and the system controller 3204 electrically communicating with said antenna. In at least one exemplary embodiment, the system controller 3204 is in electrical communication with at least one memory device or element 3206, which in at least one embodiment is flash memory like that used in a memory stick. Examples of the interaction include wireless recharging of the power source on one or both lenses, transferring of current time, transferring an alarm time, transferring data stored on the lens(es) to memory in (or in communication with) the storage box, and transferring templates and masks based on wearer-specific characteristics from the storage box to at least one lens. In an alternative exemplary embodiment, the antenna is used to communicate with an external device such as a computer or smart phone.

In at least one exemplary embodiment, the system controller 3204 is configured to translate and/or format the data received from the at least one lens to change the time stamp information into actual times based on the current accumulator reading at the time of data transfer as correlated to the current time on the storage box from the clock 3208. In an alternative exemplary embodiment, the storage box sends a signal to the lens to reset the accumulator to zero and the processor records in memory the time that the accumulator was reset to zero, or alternatively updates the accumulator to the correct time. After reinsertion of the lens into the storage box, the processor notes the current time and determines the number of sampling cycles. In the embodiments where the sampling cycles are of different lengths depending on what is being sampled and/or operational state of the lens(es) since removal of the lens(es), the storage box normalizes the sample periods over the time difference between removal of the lens(es) from the storage box and return of the lens(es) to the storage box as measured by the storage box. Alternatively when the sampling cycles are of different lengths, the storage box sends a signal to the contact lens to adjust its oscillator in an amount related to the time drift exhibited by the contact lens and in a further exemplary embodiment the storage box updates the time on the accumulator on the contact lens. In an alternative exemplary embodiment, the above described processing is performed on an external device such as a computer.

In some exemplary embodiments, the electrical communication connector 3210 may include a simple source of AC or DC current. In such embodiments, the power source 3212 may be omitted as power is provided through the electrical communication connector 3210.

In at least one exemplary embodiment, the contact lens will collect medical condition-related data over the period of time (e.g., 8 hours, 12 hours, 16 hours, 24 hours, a day of wearing). When the contact lens is placed into the storage box (or another device with similar functionality), the data is downloaded from the lens to the storage box for analysis and processing by the storage box or a computer in communication with the storage box. An example of the analysis and processing is determining whether a medical condition arose during the period of time.

In at least one exemplary embodiment, there is a test protocol for a wearer (or subject) of at least one contact lens using an external device. In at least one exemplary embodiment, the contact lens may have a variety of component combinations as discussed in this disclosure. For purposes of this discussion, the contact lens will include an eye movement sensor system, a system controller and a communications circuit configured for two-way communication with the external device. In at least one exemplary embodiment, the external device will include a processor configured to run a test protocol, a camera in communication with the processor, a display in communication with the processor and configured to display images and directions, and a communications module configured to have two-way communication with the contact lens. In a further exemplary embodiment, the camera and the display will be facing the same direction. In at least one exemplary embodiment, the system controller is configured to determine movement of the eye and/or gaze direction based on a spatial location output from the eye movement sensor system and to output a control signal based on the determination. In at least one exemplary embodiment, the output of the system controller is a signal formatted for transmission to the external device for processing and determination of the location of the eye. In at least one exemplary embodiment, the processor and the system controller together perform the test protocol. As should be understood based on this disclosure, the eye movement sensor system could be augmented by other sensors or replaced by one or more other sensors.

In at least one exemplary embodiment, the test protocol causes the processor to correlate movement of the external device by the contact lens wearer (or another individual) with the received eye location and/or gaze information transmitted by the system controller through the communications circuit and module. The processor utilizes the image data captured by the camera to monitor movement of the subject's head. When at least one of no correlation or movement of the subject's head occurs, the processor is configured to trigger an alarm, for example, activation of the display, the speaker, the flash, or a combination or sending a signal to a further device. The processor displays directions to the subject on movement of the external device and maintaining visual viewing of the external device while not moving their head. In an alternative exemplary embodiment, the external device turns its flash on to provide a light for the contact lens wearer to follow or displays a message or an image on the display. In an alternative exemplary embodiment, a person other than the contact lens wearer moves the external device, which in such an embodiment would allow use of an external device where the camera and display are facing opposite directions. In a further alternative exemplary embodiment, the external device uses instead of or in addition to the display the audio speaker to provide the directions.

In a further exemplary embodiment, where the external device includes an accelerometer, the processor is configured to use an output of the accelerometer in conjunction with an output of the camera to determine if the wearer's head is stable while the external device is moved substantially in a straight line in a horizontal plane in front of the wearer. This accelerometer data is compared to location/movement information from the contact lens to determine if any difference exceeds a threshold that if exceeded would trigger an alarm. In at least one exemplary embodiment, the contact lens data is normalized relative to the distance travelled by the external device or vice-versa to take into account that the external device will travel a greater absolute distance relative to the movement of the wearer's eye.

In an alternative exemplary embodiment, the wearer is instructed to focus on a stationary object and, while maintaining focus on that stationary object, to turn his or her head left or right. A monitoring system tracks the gaze of the wearer relative to the turning speed of the wearer's head to determine whether the differential is within a predetermined turning threshold, and initiates an alert when the turning threshold is exceeded. Examples of the alert initiation including triggering the alert mechanism or sending an alert signal to the external device or another device, which in at least one embodiment would in turn provide an alert to the wearer and/or another person. In a further exemplary embodiment, the contact lens uses an output from at least one accelerometer where the differential is determined based on a signal from the accelerometer where the signal equaling zero is confirmation that tracking of the stationary object by the wearer while when the signal is a non-zero value the wearer has a delay in tracking the stationary object. In at least one exemplary embodiment, this test protocol is performed without the external device.

The above test protocol may be used in diagnosing, for example, a stroke. The ability or inability to track an object and/or a fixed point may be a sign of other medical conditions.

A further test protocol includes testing the pupil dilation of the wearer's eye that may be used independently or in conjunction with the prior test protocol. The contact lens will include an iris-facing pupil diameter sensor in communication with the system controller. The external device will include a light source such as the flash that is controllable by the processor. The test protocol including activating the light source by the processor, measuring before and after light source activation by the system controller the pupil diameter, transmitting the pupil diameter measurements to the processor, determining the different pupil dilations by the processor, and triggering an alert when at least one pupil dilation exceeds a dilation threshold or is less than an undilated threshold. In a further exemplary embodiment, the contact lens includes a photodetector to measure the light level of the output of the light sensor to confirm that a requisite level has been meet after activation. In an alternative exemplary embodiment, the external device provides instructions to look at a bright light (instead of activating the light source) and the system controller uses the photodetector detecting the light level to confirm it is bright enough to trigger dilation. In a further alternative exemplary embodiment, the contact lens with a photodetector monitors the environmental light level and when a bright light is detected as being observed by the wearer, the system controller determines if the change in pupil diameter between before and after exceeds the dilation threshold. In a further alternative exemplary embodiment, the system controller uses an iris-facing light source on the contact lens to be activated and provide a light with sufficient brightness to trigger pupil dilation in the average wearer.

Pupil dilation may be used in detecting, for example, a concussion or intoxication of the contact lens wearer. In addition, as briefly described above, pupillary response may be utilized to diagnose cranial nerve damage. More specifically, in much the same manner as an eye-care professional can assess the pupil and iris, the sensors may be utilized to check the direct light reflex, the consensual light reflex and the convergence/accommodation reflex. For example, with Argyll Robertson disease, the pupil constricts to accommodate but will not react to light. The ability of the contact lens to detect the amount of change in pupil dilation in response to a bright light (or a rapid change in environmental light) shining into the eye of the contact lens wearer can be used to determine if a concussion has occurred or whether the wearer is intoxicated. If the change in pupil dilation is excessive in response to a change in light, then this is indicative of light sensitivity that is indicative of possibly other medical conditions. In at least one implementation, a contact lens wearer may check to see is they are possibly intoxicated before driving based on the dilation test. As mentioned above, the pupil dilation test can be used along with the test protocol for tracking a point with the eyes as the point and/or head move.

The various test protocols and monitoring capabilities of the contact lens may be initiated, terminated, etc. by input from the user through use of blinks, light or other wireless communication, and insertion/removal of the contact lens.

The above-described contact lens and combinations of sensors may be used for a variety of purposes and detection of medical conditions, some of which are discussed above.

As a corollary to the pupil dilation test, pupil constriction may be measured including the time it takes to have the pupil readjust after a bright light is shined on it. If the adjustment time is too long, then this may be indicative of a concussion or intoxication.

When the contact lens includes an iris-facing light source and an eyelid position sensor system, then the light source may be used to shine a light into the iris to provoke a corneal reflex by the eye. The quickness of the closing of the eyelid may be compared to a lid closure threshold to determine if the response was quick enough and within normal response times.

When the contact lenses include an eye movement sensor, the contact lens may be used to track eye movement and eye gaze to determine if there is agreement between the wearer's eyes. If there is not agreement between the eyes, then this is indicative of nystagmus. In at least one exemplary embodiment, the eye movement, focus and gaze would be tested using the external device as discussed above. The external device would provide the outcome of the test in terms of whether the eyes refocused as the target was moved to different distances from the eyes to see if the eyes refocused and moved along a substantially horizontal plane about the eyes to see if both eyes tracked the target without moving the head.

When the contact lens includes a biosensor, the biosensor may be used to detect the level of sodium present in the tear fluid and/or the amount of tear fluid present on the eye. When the sodium level in the tear fluid exceeds a sodium threshold or the level of tear fluid present is below a tear threshold, then the wearer may be suffering from dehydration.

When the contact lens includes a temperature sensor, the system controller is able to monitor the wearer's body temperature for a decreasing temperature before it reaches hypothermia levels and provide an alarm to the wearer. Conversely, the system controller is able to monitor for an increasing temperature before it reaches hyperthermia levels and provide an alarm to the wearer.

Although shown and described in what is believed to be the most practical embodiments, it is apparent that departures from specific designs and methods described and shown will suggest themselves to those skilled in the art and may be used without departing from the spirit and scope of the invention. The present invention is not restricted to the particular constructions described and illustrated, but should be constructed to cohere with all modifications that may fall within the scope of the appended claims.

Additional exemplary embodiments are set out below:
1. A powered ophthalmic lens, the powered ophthalmic lens comprising:
   a contact lens; and
   an eyelid position sensor system at least partially encapsulated in the contact lens, said eyelid position sensor system configured to detect vertical eyelid position and a signal conditioner configured to sample each individual sensor in said sensor system to detect eyelid position and provide an output lid signal;
   an eye movement sensor system at least partially encapsulated in the contact lens, said eye movement sensor system including at least one movement sensor to track and determine eye position and a signal conditioner cooperatively associated with said movement sensor and configured to track and determine eye position in spatial coordinates based on information from the output of said movement sensor and provide an output movement signal;
   a system controller in electrical communication with said eyelid position sensor system and said eye movement sensor system, said system controller having an associated memory containing a plurality of problem templates and at least two sets of registers for storing data received from said eyelid position sensor system and said eye movement sensor system, said system controller configured to compare the received output lid signal data and the output movement signal data to said plurality of problem templates and produce a control signal when at least one problem template is satisfied, and
   at least one alert mechanism in electrical communication with said system controller, said alert mechanism configured to receive the output control signal and capable of at least one of providing an alert and storing data.
2. The powered ophthalmic lens according to Embodiment 1, wherein at least one of the plurality of problem templates is based on historical data for an intended wearer of said lens.
3. The powered ophthalmic lens according to Embodiment 1, further comprising:
   a user input in electrical communication with said system controller; and
   a storage memory in electrical communication with said system controller, and
   wherein said system controller includes a buffer memory for storing a plurality of signals from said eyelid position sensor system and said eye movement sensor system such that upon receipt of a signal from said user input, the system controller copies the data in the buffer memory into said storage memory.
4. The powered ophthalmic lens according to Embodiment 3, wherein said user input includes a receiver capable of receiving wireless input originating with an individual to store the data present in said buffer memory.
5. The powered ophthalmic lens according to Embodiment 1, further comprising:
   a receiver in electrical communication with said system controller, said receiver configured to receive a data request from an external device; and
   a transmitter in electrical communication with said system controller and said storage memory, and
   wherein said system controller in response to a received data request, transmits the contents of said storage memory through said transmitter to the external device.
6. The powered ophthalmic lens according to Embodiment 1, wherein when said system controller determines an oscillating signal from said eye movement sensor system, said system controller copies the data in the buffer memory into a storage memory.
7. The powered ophthalmic lens according to Embodiment 1, wherein said eye movement sensor system includes at least one of at least one photodetector positioned to capture an image of the eye; at least one iris-facing camera configured to detect changes in images, patterns, or contrast to track eye movement; at least one accelerometer to track movement of at least one of the eye or the contact lens; and at least one neuromuscular sensor configured to detect neuromuscular activity associated with eye movement.
8. The powered ophthalmic lens according to Embodiment 1, wherein the eye movement sensor system further comprises a signal processor configured to receive signals from said movement sensor, perform digital signal processing, and output one or more to the system controller.
9. A lens pair comprising:
   the powered ophthalmic lens according to Embodiment 1, wherein the eye movement sensor system further comprises a communication system for communication with at least a second contact lens,
   said second contact lens having
   an eye movement sensor system incorporated into the contact lens, the eye movement sensor system including at least one sensor to track and determine eye position and a signal conditioner cooperatively associated with the sensor and configured to track and determine eye position in spatial coordinates based on information from the sensor output and provide an output movement signal;
   a system controller in electrical communication with said eye movement sensor system, and
   a communication system for communicating the output of the eye movement sensor system to said first contact lens.
10. The lens pair according to Embodiment 9, wherein when said system controller in said first contact lens detects divergence of lines of vision of the wearer's eyes, said system controller sends the control signal to said alert mechanism.
11. The lens pair according to Embodiment 9, wherein each lens further includes a rear-facing pupil diameter sensor in electrical communication with said system controller, said rear-facing pupil diameter sensor for measuring pupil diameter;
   said system controller of said second lens is configured to transmit said pupil diameter measurement via said communication systems to said system controller of said first lens such that said first lens system controller is configured to determine whether the measured pupil dilations of the wearer's eye are substantially similar, when the pupil dilations are different, the first system controller configured to send the output control signal to said alert mechanism.
12. The powered ophthalmic lens according to Embodiment 1, wherein when said system controller detects a change in pupil size not in response to a change in environmental light condition as detected by said eyelid position sensor system and where the pupil size is based on at least one signal from said eye movement sensor system, said system controller sends the control signal to said alert mechanism.
13. The powered ophthalmic lens according to Embodiment 1, wherein when said system controller detects a stable accelerometer reading in a direction indicative that a wearer is in a prone position after a rapid acceleration in that direction where the readings are from said eye movement sensor system, said system controller sends the control signal to said alert mechanism.
14. The powered ophthalmic lens according to Embodiment 1, wherein the spatial coordinates are in three dimensions.
15. The powered ophthalmic lens according to Embodiment 1, wherein
   said movement sensor includes at least one accelerometer; and
   said system controller compares each signal from said at least one accelerometer against a threshold, when any signal exceeds the threshold, said system controller sends the control signal to said alert mechanism.
16. The powered ophthalmic lens according to Embodiment 1, further comprising:
   an iris-facing light source is in electrical communication with said system controller; and
   at least one iris-facing photosensor arranged to receive reflected light back from the eye where said light originates from said light source, said at least photosensor is in electrical communication with said system controller;
   a transmitter in electrical communication with said system controller, and
   wherein said system controller is configured to send an oximeter signal to said light source and receive a signal from said at least one photosensor, which received signal is transmitted to an external device for processing by said system controller through said transmitter.
17. The powered ophthalmic lens according to Embodiment 1, wherein said system controller is configured to use more than one system sensor to confirm any determination by said system controller of a need for the output control signal to be sent to said alert mechanism.
18. A powered ophthalmic lens, the powered ophthalmic lens comprising:
   a contact lens; and
   a first sensor in said contact lens;
   at least one second sensor in said contact lens;
   a system controller in electrical communication with said first sensor and said at least one second sensor, said system controller having an associated memory containing a plurality of problem templates and at least two sets of registers for storing data received from said sensors, said system controller configured to compare the received sensor data to said plurality of problem templates and produce a control signal when a match occurs, and
   at least one alert mechanism in electrical communication with said system controller, said alert mechanism configured to receive the output control signal and capable of at least one of providing an alert and storing data.
19. The powered ophthalmic lens according to Embodiment 18, wherein said first sensor and/or said at least one second sensor is selected from a group consisting of an eyelid position sensor system, an eye movement sensor system, a biosensor, a bioimpedance sensor, a temperature sensor, and pulse oximeter.
20. A powered ophthalmic lens comprising:
   a contact lens
   an iris-facing light source in said contact lens;
   at least one iris-facing photosensor arranged to receive reflected light back from the eye where said light originates from said light source; and
   a system controller in electrical communication with said iris-facing light source and said at least one iris-facing photosensor, said system controller configured to process at least one signal from said iris-facing photosensor and correlate the processed signal with at least one signal sent to said iris-facing light source.
21. The powered ophthalmic lens according to Embodiment 20, further comprising a transmitter in electrical communication with said system controller, and
   wherein said system controller is configured to send the correlated signals via said transmitter to an external device for processing.
22. The powered ophthalmic lens according to Embodiment 20, wherein said iris-facing light source and said at least one iris-facing photosensor are spaced from each other such that said iris-facing light source and said at least one iris-facing photosensor are proximate to opposing edges of said contact lens.
23. The powered ophthalmic lens according to Embodiment 20, wherein said iris-facing light source includes a first light emitter transmitting a light having a wavelength of about 660 nm and a second light emitter transmitting a light having a wavelength of between about 890 nm and about 950 nm.
24. A system for conducting a test protocol on a wearer of at least one contact lens, said system comprising:
   a device having
   a processor configured to run a test protocol,
   a camera connected to said processor,
   a display connected to said processor and configured to display images generated by said processor,
   communications module; and
   at least one powered ophthalmic contact lens having
   an eye movement sensor system including a sensor to determine and track eye position, said eye movement sensor system configured to output a spatial location of the eye,
   a system controller cooperatively associated with the sensor, the system controller configured to determine movement of the eye based on the spatial location output from said eye movement sensor system, said system controller is further configured to output a control signal based on the determination, and
   communications circuit configured to facilitate communication with said communications module of said device during performance of the test protocol; and
   wherein said processor performs the test protocol in conjunction with said system controller.
25. The system according to Embodiment 24, wherein
   said control signal produced by said system controller includes gaze direction information;
   said test protocol correlates movement of said device by a subject while the display is providing directions to the subject with the received gaze direction transmitted by said system controller through said communications circuit and said communications module while monitoring for movement of a subject's head, when at least one of no correlation or movement of the subject's head occurs, said processor is configured to trigger an alert to be shown on said display; and
   wherein the directions are generated by said processor based on instructions performed by said processor.
26. The system according to Embodiment 25, wherein
   said device includes an accelerometer electrically connected to said processor such that said processor is configured to use an output of said accelerometer in conjunction with an output of said camera to determine if the subject's head is stable while said device is moved substantially in a straight line in front of the subject, and
   said processor is configured to correlate the accelerometer readings from said lens transmitted through said communications circuit and said communications module with the accelerometer signals from said accelerometer on said device, when a difference between the accelerometer signals after normalization for distance travelled by said device and said lens is greater than a threshold, then said processor is configured to trigger the alert to be shown on said display.
27. The system according to Embodiment 24, wherein
   said lens further includes an iris-facing pupil diameter sensor in electrical communication with said system controller, said iris-facing pupil diameter sensor configured to provide a signal representing pupil diameter;
   said device further includes a light source controllable by said processor, and
   said test protocol includes
   said processor activating said light source,
   said system controller measuring a before and after light source activation of said pupil diameter with said pupil diameter sensor, said system controller transmitting said measurements to said processor through said antennas,
   said processor comparing said measurements to determine pupil dilation, and
   said processor sending an alert to said display when at least one of the pupil dilation exceeds a dilation threshold and the pupil dilation is less than an undilated threshold.
28. The system according to Embodiment 27, wherein said contact lens further includes a photodetector in communication with said system controller; and
   wherein said system controller configured to use outputs of said photodetector to detect a light level of said light source.
29. The system according to Embodiment 24, wherein
   said lens further includes an iris-facing pupil diameter sensor in electrical communication with said system controller, said iris-facing pupil diameter sensor for measuring pupil diameter;
   said device further includes a light source controllable by said processor, and
   said test protocol includes
   said processor displaying instruction on said display directing the wearer to view a bright light,
   said system controller measuring a before and after light source activation of said pupil diameter with said pupil diameter sensor, said system controller transmitting said measurements to said processor through said antennas,
   said processor comparing said measurements to determine pupil dilation, and
   said processor sending an alert to said display when at least one of the pupil dilation exceeds a dilation threshold and the pupil dilation is less than an undilated threshold.
30. The system according to Embodiment 29, wherein said contact lens further includes a photodetector in communication with said system controller; and
   wherein said system controller configured to use outputs of said photodetector to detect a light level of said light source.
31. The system according to Embodiment 24, wherein
   said sensor includes at least one accelerometer; and
   said test protocol is prompted by detection of a possible concussion when said system controller determines an acceleration of a head of the wearer exceeds a concussion threshold based on a signal received from said accelerometer.
32. The system according to Embodiment 24, wherein said test protocol includes
   having a wearer of the lens focus on a place on a stationary object,
   turning the wearer's head right or left while having the wearer continue to look at the place,
   tracking the gaze of the wearer relative to the turning speed of the wearer's head to determine whether the differential is within a predetermined threshold,
   alerting at least one of the wearer through said alert mechanism and/or through transmitting an alert signal to said device to display an alert on said display.
33. The system according to Embodiment 32, wherein
   said eye movement sensor system includes at least one accelerometer; and
   the differential is determined based on a signal from said at least one accelerometer where the signal equaling zero is confirmation of tracking of the place on the wall by the wearer while when the signal is a non-zero value the wearer has a delay in tracking the place on the wall.
34. The system according to Embodiment 32, wherein said test protocol further includes storing on said device data from said test protocol for later use in a verification study.
35. A system for conducting a test protocol on a wearer of at least one contact lens, said system comprising:
   at least one powered ophthalmic contact lens having
   an iris-facing pupil diameter sensor configured to output a signal representing pupil diameter;
   at least one forward-facing photodetector;
   an alert mechanism;
   a system controller in communication with said iris-facing pupil diameter sensor and said at least one photodetector, the system controller configured to
   monitor outputs of said iris-facing pupil diameter sensor,
   monitor said at least one forward-facing photodetector for a detected light exceeding a brightness threshold,
   compare the output of the iris-facing pupil diameter sensor from before and after detection of the light exceeding the brightness threshold,
   when the difference between outputs of the iris-facing pupil diameter sensor exceeds a dilation threshold or is less than an undilated threshold, sending a signal to said alert mechanism.
36. The system according to Embodiment 35, wherein said alert mechanism alerts the user in response to the signal from the system controller.

## Claims

1. A powered ophthalmic lens, the powered ophthalmic lens comprising:
a contact lens; and
an eyelid position sensor system at least partially encapsulated in the contact lens, said eyelid position sensor system configured to detect vertical eyelid position and a signal conditioner configured to sample each individual sensor in said sensor system to detect eyelid position and provide an output lid signal;
an eye movement sensor system at least partially encapsulated in the contact lens, said eye movement sensor system including at least one movement sensor to track and determine eye position and a signal conditioner cooperatively associated with said movement sensor and configured to track and determine eye position in spatial coordinates based on information from the output of said movement sensor and provide an output movement signal;
a system controller in electrical communication with said eyelid position sensor system and said eye movement sensor system, said system controller having an associated memory containing a plurality of problem templates and at least two sets of registers for storing data received from said eyelid position sensor system and said eye movement sensor system, said system controller configured to compare the received output lid signal data and the output movement signal data to said plurality of problem templates and produce a control signal when at least one problem template is satisfied, and
at least one alert mechanism in electrical communication with said system controller, said alert mechanism configured to receive the output control signal and capable of at least one of providing an alert and storing data.

2. The powered ophthalmic lens according to Claim 1, wherein at least one of the plurality of problem templates is based on historical data for an intended wearer of said lens.

3. The powered ophthalmic lens according to Claim 1, further comprising:
a user input in electrical communication with said system controller; and
a storage memory in electrical communication with said system controller, and
wherein said system controller includes a buffer memory for storing a plurality of signals from said eyelid position sensor system and said eye movement sensor system such that upon receipt of a signal from said user input, the system controller copies the data in the buffer memory into said storage memory; wherein optionally said user input includes a receiver capable of receiving wireless input originating with an individual to store the data present in said buffer memory.

4. The powered ophthalmic lens according to Claim 1, further comprising:
a receiver in electrical communication with said system controller, said receiver configured to receive a data request from an external device; and
a transmitter in electrical communication with said system controller and said storage memory, and
wherein said system controller in response to a received data request, transmits the contents of said storage memory through said transmitter to the external device.

5. The powered ophthalmic lens according to Claim 1, wherein when said system controller determines an oscillating signal from said eye movement sensor system, said system controller copies the data in the buffer memory into a storage memory.

6. The powered ophthalmic lens according to Claim 1, wherein said eye movement sensor system includes at least one of at least one photodetector positioned to capture an image of the eye; at least one iris-facing camera configured to detect changes in images, patterns, or contrast to track eye movement; at least one accelerometer to track movement of at least one of the eye or the contact lens; and at least one neuromuscular sensor configured to detect neuromuscular activity associated with eye movement.

7. The powered ophthalmic lens according to Claim 1, wherein the eye movement sensor system further comprises a signal processor configured to receive signals from said movement sensor, perform digital signal processing, and output one or more to the system controller.

8. A lens pair comprising:
the powered ophthalmic lens according to Claim 1, wherein the eye movement sensor system further comprises a communication system for communication with at least a second contact lens,
said second contact lens having
an eye movement sensor system incorporated into the contact lens, the eye movement sensor system including at least one sensor to track and determine eye position and a signal conditioner cooperatively associated with the sensor and configured to track and determine eye position in spatial coordinates based on information from the sensor output and provide an output movement signal;
a system controller in electrical communication with said eye movement sensor system, and
a communication system for communicating the output of the eye movement sensor system to said first contact lens;
wherein optionally when said system controller in said first contact lens detects divergence of lines of vision of the wearer's eyes, said system controller sends the control signal to said alert mechanism; and/or
wherein optionally each lens further includes a rear-facing pupil diameter sensor in electrical communication with said system controller, said rear-facing pupil diameter sensor for measuring pupil diameter;
said system controller of said second lens is configured to transmit said pupil diameter measurement via said communication systems to said system controller of said first lens such that said first lens system controller is configured to determine whether the measured pupil dilations of the wearer's eye are substantially similar, when the pupil dilations are different, the first system controller configured to send the output control signal to said alert mechanism.

9. The powered ophthalmic lens according to Claim 1, wherein when said system controller detects a change in pupil size not in response to a change in environmental light condition as detected by said eyelid position sensor system and where the pupil size is based on at least one signal from said eye movement sensor system, said system controller sends the control signal to said alert mechanism.

10. The powered ophthalmic lens according to Claim 1, wherein when said system controller detects a stable accelerometer reading in a direction indicative that a wearer is in a prone position after a rapid acceleration in that direction where the readings are from said eye movement sensor system, said system controller sends the control signal to said alert mechanism.

11. The powered ophthalmic lens according to Claim 1, wherein the spatial coordinates are in three dimensions.

12. The powered ophthalmic lens according to Claim 1, wherein
said movement sensor includes at least one accelerometer; and
said system controller compares each signal from said at least one accelerometer against a threshold, when any signal exceeds the threshold, said system controller sends the control signal to said alert mechanism.

13. The powered ophthalmic lens according to Claim 1, further comprising:
an iris-facing light source is in electrical communication with said system controller; and
at least one iris-facing photosensor arranged to receive reflected light back from the eye where said light originates from said light source, said at least photosensor is in electrical communication with said system controller;
a transmitter in electrical communication with said system controller, and
wherein said system controller is configured to send an oximeter signal to said light source and receive a signal from said at least one photosensor, which received signal is transmitted to an external device for processing by said system controller through said transmitter.

14. The powered ophthalmic lens according to Claim 1, wherein said system controller is configured to use more than one system sensor to confirm any determination by said system controller of a need for the output control signal to be sent to said alert mechanism.

15. A powered ophthalmic lens, the powered ophthalmic lens comprising:
a contact lens; and
a first sensor in said contact lens;
at least one second sensor in said contact lens;
a system controller in electrical communication with said first sensor and said at least one second sensor, said system controller having an associated memory containing a plurality of problem templates and at least two sets of registers for storing data received from said sensors, said system controller configured to compare the received sensor data to said plurality of problem templates and produce a control signal when a match occurs, and
at least one alert mechanism in electrical communication with said system controller, said alert mechanism configured to receive the output control signal and capable of at least one of providing an alert and storing data.
